# EUROPEAN PATENT APPLICATION

(11) **EP 2 570 024 A1**
(43) Date of publication of application: **20.03.2013**
(21) Application number: 12180255.7
(22) Date of filing: 03.04.2008
(51) Int. Cl.: A01H 5/00, A01H 5/10, C12N 15/82, C12N 9/88

(54) **AHAS mutants**

(30) Priority: 04.04.2007 US 910028 P
(62) Divisional of application: 08733094.0
(71) Applicant: BASF Plant Science GmbH, 67056 Ludwigshafen (DE)
(72) Inventor: McElver, John A, Durham, NC 27712 (US); Singh, Bijay, Cary, NC 27519 (US)
(74) Representative: Reitstötter - Kinzebach

(57) **Abstract**

The invention provides nucleic acids encoding mutants of the acetohydroxyacid synthase (AHAS) large subunit comprising at least two mutations, for example double and triple mutants, which are useful for producing transgenic or non-transgenic plants with improved levels of tolerance to AHAS-inhibiting herbicides. The invention also provides expression vectors, cells, plants comprising the polynucleotides encoding the AHAS large subunit double and triple mutants, plants comprising two or more AHAS large subunit single mutant polypeptides, and methods for making and using the same.

SEQUENCE LISTING

<110>  BASF Plant Science GmbH

<120>  AHAS MUTANTS

<130>  M/50209-EP-DIV

<140>  EP 08733094.0
<141>  2008-04-03

<150>  US 60/910,028
<151>  2007-04-04

<160>  34

<170>  PatentIn version 3.4

<210>  1
<211>  670
<212>  PRT
<213>  Arabidopsis thaliana

<220>
<221>  MISC_FEATURE
<223>  Arabidopsis thaliana acetohydroxyacid synthase large subunit
       (AtAHASL)

<400>  1

Met Ala Ala Ala Thr Thr Thr Thr Thr Thr Ser Ser Ser Ile Ser Phe
1               5                   10                  15

Ser Thr Lys Pro Ser Pro Ser Ser Ser Lys Ser Pro Leu Pro Ile Ser
            20                  25                  30

Arg Phe Ser Leu Pro Phe Ser Leu Asn Pro Asn Lys Ser Ser Ser Ser
        35                  40                  45

Ser Arg Arg Arg Gly Ile Lys Ser Ser Ser Pro Ser Ser Ile Ser Ala
    50                  55                  60

Val Leu Asn Thr Thr Thr Asn Val Thr Thr Thr Pro Ser Pro Thr Lys
65                  70                  75                  80

Pro Thr Lys Pro Glu Thr Phe Ile Ser Arg Phe Ala Pro Asp Gln Pro
                85                  90                  95

Arg Lys Gly Ala Asp Ile Leu Val Glu Ala Leu Glu Arg Gln Gly Val
            100                 105                 110

Glu Thr Val Phe Ala Tyr Pro Gly Gly Ala Ser Met Glu Ile His Gln
        115                 120                 125

Ala Leu Thr Arg Ser Ser Ser Ile Arg Asn Val Leu Pro Arg His Glu
    130                 135                 140

Gln Gly Gly Val Phe Ala Ala Glu Gly Tyr Ala Arg Ser Ser Gly Lys
145                 150                 155                 160

Pro Gly Ile Cys Ile Ala Thr Ser Gly Pro Gly Ala Thr Asn Leu Val
                165                 170                 175

Ser Gly Leu Ala Asp Ala Leu Leu Asp Ser Val Pro Leu Val Ala Ile
            180                 185                 190

Thr Gly Gln Val Pro Arg Arg Met Ile Gly Thr Asp Ala Phe Gln Glu
        195                 200                 205

Thr Pro Ile Val Glu Val Thr Arg Ser Ile Thr Lys His Asn Tyr Leu
    210                 215                 220

Val Met Asp Val Glu Asp Ile Pro Arg Ile Ile Glu Glu Ala Phe Phe
225                 230                 235                 240

Leu Ala Thr Ser Gly Arg Pro Gly Pro Val Leu Val Asp Val Pro Lys
                245                 250                 255

Asp Ile Gln Gln Gln Leu Ala Ile Pro Asn Trp Glu Gln Ala Met Arg
            260                 265                 270

Leu Pro Gly Tyr Met Ser Arg Met Pro Lys Pro Pro Glu Asp Ser His
        275                 280                 285

Leu Glu Gln Ile Val Arg Leu Ile Ser Glu Ser Lys Lys Pro Val Leu
    290                 295                 300

Tyr Val Gly Gly Gly Cys Leu Asn Ser Ser Asp Glu Leu Gly Arg Phe
305                 310                 315                 320

Val Glu Leu Thr Gly Ile Pro Val Ala Ser Thr Leu Met Gly Leu Gly
                325                 330                 335

Ser Tyr Pro Cys Asp Asp Glu Leu Ser Leu His Met Leu Gly Met His
            340                 345                 350

Gly Thr Val Tyr Ala Asn Tyr Ala Val Glu His Ser Asp Leu Leu Leu
        355                 360                 365

Ala Phe Gly Val Arg Phe Asp Asp Arg Val Thr Gly Lys Leu Glu Ala
    370                 375                 380

Phe Ala Ser Arg Ala Lys Ile Val His Ile Asp Ile Asp Ser Ala Glu
385                 390                 395                 400

Ile Gly Lys Asn Lys Thr Pro His Val Ser Val Cys Gly Asp Val Lys
                405                 410                 415

Leu Ala Leu Gln Gly Met Asn Lys Val Leu Glu Asn Arg Ala Glu Glu
            420                 425                 430

Leu Lys Leu Asp Phe Gly Val Trp Arg Asn Glu Leu Asn Val Gln Lys
        435                 440                 445

Gln Lys Phe Pro Leu Ser Phe Lys Thr Phe Gly Glu Ala Ile Pro Pro
    450                 455                 460

Gln Tyr Ala Ile Lys Val Leu Asp Glu Leu Thr Asp Gly Lys Ala Ile
465                 470                 475                 480

Ile Ser Thr Gly Val Gly Gln His Gln Met Trp Ala Ala Gln Phe Tyr
                485                 490                 495

Asn Tyr Lys Lys Pro Arg Gln Trp Leu Ser Ser Gly Gly Leu Gly Ala
            500                 505                 510

Met Gly Phe Gly Leu Pro Ala Ala Ile Gly Ala Ser Val Ala Asn Pro
        515                 520                 525

Asp Ala Ile Val Val Asp Ile Asp Gly Asp Gly Ser Phe Ile Met Asn
    530                 535                 540

Val Gln Glu Leu Ala Thr Ile Arg Val Glu Asn Leu Pro Val Lys Val
545                 550                 555                 560

Leu Leu Leu Asn Asn Gln His Leu Gly Met Val Met Gln Trp Glu Asp
                565                 570                 575

Arg Phe Tyr Lys Ala Asn Arg Ala His Thr Phe Leu Gly Asp Pro Ala
            580                 585                 590

Gln Glu Asp Glu Ile Phe Pro Asn Met Leu Leu Phe Ala Ala Ala Cys
        595                 600                 605

Gly Ile Pro Ala Ala Arg Val Thr Lys Lys Ala Asp Leu Arg Glu Ala
    610                 615                 620

Ile Gln Thr Met Leu Asp Thr Pro Gly Pro Tyr Leu Leu Asp Val Ile
625                 630                 635                 640

Cys Pro His Gln Glu His Val Leu Pro Met Ile Pro Ser Gly Gly Thr
                645                 650                 655

Phe Asn Asp Val Ile Thr Glu Gly Asp Gly Arg Ile Lys Tyr
            660                 665                 670

<210>  2
<211>  638
<212>  PRT
<213>  Zea mays

<220>
<221>  MISC_FEATURE
<223>  Zea mays acetohydroxyacid synthase large subunit number two
       (ZmAHASL2)

<400>  2

Met Ala Thr Ala Ala Ala Ala Ser Thr Ala Leu Thr Gly Ala Thr Thr
1               5                   10                  15

Ala Ala Pro Lys Ala Arg Arg Arg Ala His Leu Leu Ala Thr Arg Arg
            20                  25                  30

Ala Leu Ala Ala Pro Ile Arg Cys Ser Ala Ala Ser Pro Ala Met Pro
        35                  40                  45

Met Ala Pro Pro Ala Thr Pro Leu Arg Pro Trp Gly Pro Thr Asp Pro
    50                  55                  60

Arg Lys Gly Ala Asp Ile Leu Val Glu Ser Leu Glu Arg Cys Gly Val
65                  70                  75                  80

Arg Asp Val Phe Ala Tyr Pro Gly Gly Ala Ser Met Glu Ile His Gln
                85                  90                  95

Ala Leu Thr Arg Ser Pro Val Ile Ala Asn His Leu Phe Arg His Glu
            100                 105                 110

Gln Gly Glu Ala Phe Ala Ala Ser Gly Tyr Ala Arg Ser Ser Gly Arg
        115                 120                 125

Val Gly Val Cys Ile Ala Thr Ser Gly Pro Gly Ala Thr Asn Leu Val
    130                 135                 140

Ser Ala Leu Ala Asp Ala Leu Leu Asp Ser Val Pro Met Val Ala Ile
145                 150                 155                 160

Thr Gly Gln Val Pro Arg Arg Met Ile Gly Thr Asp Ala Phe Gln Glu
                165                 170                 175

Thr Pro Ile Val Glu Val Thr Arg Ser Ile Thr Lys His Asn Tyr Leu
            180                 185                 190

Val Leu Asp Val Asp Asp Ile Pro Arg Val Val Gln Glu Ala Phe Phe
        195                 200                 205

Leu Ala Ser Ser Gly Arg Pro Gly Pro Val Leu Val Asp Ile Pro Lys
    210                 215                 220

Asp Ile Gln Gln Gln Met Ala Val Pro Val Trp Asp Lys Pro Met Ser
225                 230                 235                 240

Leu Pro Gly Tyr Ile Ala Arg Leu Pro Lys Pro Pro Ala Thr Glu Leu
                245                 250                 255

Leu Glu Gln Val Leu Arg Leu Val Gly Glu Ser Arg Arg Pro Val Leu
            260                 265                 270

Tyr Val Gly Gly Gly Cys Ala Ala Ser Gly Glu Glu Leu Arg Arg Phe
        275                 280                 285

Val Glu Leu Thr Gly Ile Pro Val Thr Thr Thr Leu Met Gly Leu Gly
    290                 295                 300

Asn Phe Pro Ser Asp Asp Pro Leu Ser Leu Arg Met Leu Gly Met His
305                 310                 315                 320

Gly Thr Val Tyr Ala Asn Tyr Ala Val Asp Lys Ala Asp Leu Leu Leu
                325                 330                 335

Ala Leu Gly Val Arg Phe Asp Asp Arg Val Thr Gly Lys Ile Glu Ala
            340                 345                 350

Phe Ala Ser Arg Ala Lys Ile Val His Val Asp Ile Asp Pro Ala Glu
        355                 360                 365

Ile Gly Lys Asn Lys Gln Pro His Val Ser Ile Cys Ala Asp Val Lys
    370                 375                 380

Leu Ala Leu Gln Gly Met Asn Ala Leu Leu Glu Gly Ser Thr Ser Lys
385                 390                 395                 400

Lys Ser Phe Asp Phe Gly Ser Trp Asn Asp Glu Leu Asp Gln Gln Lys
                405                 410                 415

Arg Glu Phe Pro Leu Gly Tyr Lys Thr Ser Asn Glu Glu Ile Gln Pro
            420                 425                 430

Gln Tyr Ala Ile Gln Val Leu Asp Glu Leu Thr Lys Gly Glu Ala Ile
        435                 440                 445

Ile Gly Thr Gly Val Gly Gln His Gln Met Trp Ala Ala Gln Tyr Tyr

450                 455                 460

Thr Tyr Lys Arg Pro Arg Gln Trp Leu Ser Ser Ala Gly Leu Gly Ala
465                 470                 475                 480

Met Gly Phe Gly Leu Pro Ala Ala Ala Gly Ala Ser Val Ala Asn Pro
                485                 490                 495

Gly Val Thr Val Val Asp Ile Asp Gly Asp Gly Ser Phe Leu Met Asn
            500                 505                 510

Val Gln Glu Leu Ala Met Ile Arg Ile Glu Asn Leu Pro Val Lys Val
        515                 520                 525

Phe Val Leu Asn Asn Gln His Leu Gly Met Val Val Gln Trp Glu Asp
    530                 535                 540

Arg Phe Tyr Lys Ala Asn Arg Ala His Thr Tyr Leu Gly Asn Pro Glu
545                 550                 555                 560

Asn Glu Ser Glu Ile Tyr Pro Asp Phe Val Thr Ile Ala Lys Gly Phe
                565                 570                 575

Asn Ile Pro Ala Val Arg Val Thr Lys Lys Asn Glu Val Arg Ala Ala
            580                 585                 590

Ile Lys Lys Met Leu Glu Thr Pro Gly Pro Tyr Leu Leu Asp Ile Ile
        595                 600                 605

Val Pro His Gln Glu His Val Leu Pro Met Ile Pro Ser Gly Gly Ala
    610                 615                 620

Phe Lys Asp Met Ile Leu Asp Gly Asp Gly Arg Thr Val Tyr
625                 630                 635

<210>  3
<211>  652
<212>  PRT
<213>  Brassica napus

<220>
<221>  MISC_FEATURE
<223>  Brassica napus A genome acetohydroxyacid synthase large subunit
       number one (BnAHASL1A)

<400>  3

Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser

            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Glu Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210>  4
<211>  644
<212>  PRT
<213>  Oryza sativa

<220>
<221>  MISC_FEATURE
<223>  Rice acetohydroxyacid synthase large subunit (OsAHASL)

<400>  4

Met Ala Thr Thr Ala Ala Ala Ala Ala Ala Ala Leu Ser Ala Ala Ala
1               5                   10                  15

Thr Ala Lys Thr Gly Arg Lys Asn His Gln Arg His His Val Leu Pro
            20                  25                  30

Ala Arg Gly Arg Val Gly Ala Ala Ala Val Arg Cys Ser Ala Val Ser
        35                  40                  45

Pro Val Thr Pro Pro Ser Pro Ala Pro Pro Ala Thr Pro Leu Arg Pro
    50                  55                  60

Trp Gly Pro Ala Glu Pro Arg Lys Gly Ala Asp Ile Leu Val Glu Ala
65                  70                  75                  80

Leu Glu Arg Cys Gly Val Ser Asp Val Phe Ala Tyr Pro Gly Gly Ala
                85                  90                  95

Ser Met Glu Ile His Gln Ala Leu Thr Arg Ser Pro Val Ile Thr Asn
            100                 105                 110

His Leu Phe Arg His Glu Gln Gly Glu Ala Phe Ala Ala Ser Gly Tyr
        115                 120                 125

Ala Arg Ala Ser Gly Arg Val Gly Val Cys Val Ala Thr Ser Gly Pro
    130                 135                 140

Gly Ala Thr Asn Leu Val Ser Ala Leu Ala Asp Ala Leu Leu Asp Ser
145                 150                 155                 160

Val Pro Met Val Ala Ile Thr Gly Gln Val Pro Arg Arg Met Ile Gly
                165                 170                 175

Thr Asp Ala Phe Gln Glu Thr Pro Ile Val Glu Val Thr Arg Ser Ile
            180                 185                 190

Thr Lys His Asn Tyr Leu Val Leu Asp Val Glu Asp Ile Pro Arg Val
        195                 200                 205

Ile Gln Glu Ala Phe Phe Leu Ala Ser Ser Gly Arg Pro Gly Pro Val
    210                 215                 220

Leu Val Asp Ile Pro Lys Asp Ile Gln Gln Gln Met Ala Val Pro Val
225                 230                 235                 240

Trp Asp Thr Ser Met Asn Leu Pro Gly Tyr Ile Ala Arg Leu Pro Lys
                245                 250                 255

Pro Pro Ala Thr Glu Leu Leu Glu Gln Val Leu Arg Leu Val Gly Glu
            260                 265                 270

Ser Arg Arg Pro Ile Leu Tyr Val Gly Gly Gly Cys Ser Ala Ser Gly
        275                 280                 285

Asp Glu Leu Arg Trp Phe Val Glu Leu Thr Gly Ile Pro Val Thr Thr
    290                 295                 300

Thr Leu Met Gly Leu Gly Asn Phe Pro Ser Asp Asp Pro Leu Ser Leu
305                 310                 315                 320

Arg Met Leu Gly Met His Gly Thr Val Tyr Ala Asn Tyr Ala Val Asp
                325                 330                 335

Lys Ala Asp Leu Leu Leu Ala Phe Gly Val Arg Phe Asp Asp Arg Val
            340                 345                 350

Thr Gly Lys Ile Glu Ala Phe Ala Ser Arg Ala Lys Ile Val His Ile

355                 360                 365

Asp Ile Asp Pro Ala Glu Ile Gly Lys Asn Lys Gln Pro His Val Ser
    370                 375                 380

Ile Cys Ala Asp Val Lys Leu Ala Leu Gln Gly Leu Asn Ala Leu Leu
385                 390                 395                 400

Gln Gln Ser Thr Thr Lys Thr Ser Ser Asp Phe Ser Ala Trp His Asn
                405                 410                 415

Glu Leu Asp Gln Gln Lys Arg Glu Phe Pro Leu Gly Tyr Lys Thr Phe
            420                 425                 430

Gly Glu Glu Ile Pro Pro Gln Tyr Ala Ile Gln Val Leu Asp Glu Leu
        435                 440                 445

Thr Lys Gly Glu Ala Ile Ile Ala Thr Gly Val Gly Gln His Gln Met
    450                 455                 460

Trp Ala Ala Gln Tyr Tyr Thr Tyr Lys Arg Pro Arg Gln Trp Leu Ser
465                 470                 475                 480

Ser Ala Gly Leu Gly Ala Met Gly Phe Gly Leu Pro Ala Ala Ala Gly
                485                 490                 495

Ala Ser Val Ala Asn Pro Gly Val Thr Val Val Asp Ile Asp Gly Asp
            500                 505                 510

Gly Ser Phe Leu Met Asn Ile Gln Glu Leu Ala Leu Ile Arg Ile Glu
        515                 520                 525

Asn Leu Pro Val Lys Val Met Val Leu Asn Asn Gln His Leu Gly Met
    530                 535                 540

Val Val Gln Trp Glu Asp Arg Phe Tyr Lys Ala Asn Arg Ala His Thr
545                 550                 555                 560

Tyr Leu Gly Asn Pro Glu Cys Glu Ser Glu Ile Tyr Pro Asp Phe Val
                565                 570                 575

Thr Ile Ala Lys Gly Phe Asn Ile Pro Ala Val Arg Val Thr Lys Lys
            580                 585                 590

Ser Glu Val Arg Ala Ala Ile Lys Lys Met Leu Glu Thr Pro Gly Pro
        595                 600                 605

Tyr Leu Leu Asp Ile Ile Val Pro His Gln Glu His Val Leu Pro Met
    610                 615                 620

Ile Pro Ser Gly Gly Ala Phe Lys Asp Met Ile Leu Asp Gly Asp Gly
625                 630                 635                 640

Arg Thr Val Tyr

<210>  5
<211>  655
<212>  PRT
<213>  Helianthus annuus

<220>
<221>  MISC_FEATURE
<223>  Sunflower acetohydroxyacid synthase large subunit number
       1(HaAHASL1)

<400>  5

Met Ala Ala Pro Pro Asn Pro Ser Ile Ser Phe Lys Pro Pro Ser Pro
1               5                   10                  15

Ala Ala Ala Leu Pro Pro Arg Ser Ala Phe Leu Pro Arg Phe Ala Leu
            20                  25                  30

Pro Ile Thr Ser Thr Thr Gln Lys Arg His Arg Leu His Ile Ser Asn
        35                  40                  45

Val Leu Ser Asp Ser Lys Ser Thr Thr Thr Thr Thr Thr Thr Thr Gln
    50                  55                  60

Arg Pro Leu Pro Val Gln Pro Phe Val Ser Arg Tyr Ala Pro Asp Gln
65                  70                  75                  80

Pro Arg Lys Gly Ala Asp Val Leu Val Glu Ala Leu Glu Arg Glu Gly
                85                  90                  95

Val Thr Asp Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ala Ser Gly
    130                 135                 140

Leu Pro Gly Val Cys Ile Ala Thr Ser Gly Pro Gly Ala Thr Asn Leu
145                 150                 155                 160

Val Ser Gly Leu Ala Asp Ala Leu Leu Asp Ser Val Pro Met Val Ala
                165                 170                 175

Ile Thr Gly Gln Val Pro Arg Arg Met Ile Gly Thr Asp Ala Phe Gln
        180                 185                 190

Glu Thr Pro Ile Val Glu Val Thr Arg Ser Ile Thr Lys His Asn Tyr
    195                 200                 205

Leu Val Leu Asp Val Glu Asp Ile Pro Arg Ile Val Arg Glu Ala Phe
    210                 215                 220

Tyr Leu Ala Ser Ser Gly Arg Pro Gly Pro Val Leu Ile Asp Val Pro
225                 230                 235                 240

Lys Asp Ile Gln Gln Gln Leu Val Val Pro Lys Trp Asp Glu Pro Met
                245                 250                 255

Arg Leu Pro Gly Tyr Leu Ser Arg Met Pro Lys Pro Gln Tyr Asp Gly
            260                 265                 270

His Leu Glu Gln Ile Val Arg Leu Val Gly Glu Ala Lys Arg Pro Val
        275                 280                 285

Leu Tyr Val Gly Gly Gly Cys Leu Asn Ser Asp Asp Glu Leu Arg Arg
    290                 295                 300

Phe Val Glu Leu Thr Gly Ile Pro Val Ala Ser Thr Leu Met Gly Leu
305                 310                 315                 320

Gly Ala Tyr Pro Ala Ser Ser Asp Leu Ser Leu His Met Leu Gly Met
                325                 330                 335

His Gly Thr Val Tyr Ala Asn Tyr Ala Val Asp Lys Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Pro Ala
    370                 375                 380

Glu Ile Gly Lys Asn Lys Gln Pro His Val Ser Ile Cys Gly Asp Ile
385                 390                 395                 400

Lys Val Ala Leu Gln Gly Leu Asn Lys Ile Leu Glu Glu Lys Asn Ser
                405                 410                 415

Val Thr Asn Leu Asp Phe Ser Thr Trp Arg Lys Glu Leu Asp Glu Gln
            420                 425                 430

Lys Met Lys Phe Pro Leu Ser Phe Lys Thr Phe Gly Glu Ala Ile Pro
        435                 440                 445

Pro Gln Tyr Ala Ile Gln Val Leu Asp Glu Leu Thr Gly Gly Asn Ala
    450                 455                 460

Ile Ile Ser Thr Gly Val Gly Gln His Gln Met Trp Ala Ala Gln Phe
465                 470                 475                 480

Tyr Lys Tyr Asn Lys Pro Arg Gln Trp Leu Thr Ser Gly Gly Leu Gly
                485                 490                 495

Ala Met Gly Phe Gly Leu Pro Ala Ala Ile Gly Ala Ala Val Ala Arg
            500                 505                 510

Pro Asp Ala Val Val Val Asp Ile Asp Gly Asp Gly Ser Phe Met Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Val Gln Trp Glu
545                 550                 555                 560

Asp Arg Phe Tyr Lys Ala Asn Arg Ala His Thr Tyr Leu Gly Asn Pro
                565                 570                 575

Ser Lys Glu Ser Glu Ile Phe Pro Asn Met Val Lys Phe Ala Glu Ala
            580                 585                 590

Cys Asp Ile Pro Ala Ala Arg Val Thr Gln Lys Ala Asp Leu Arg Ala
        595                 600                 605

Ala Ile Gln Lys Met Leu Asp Thr Pro Gly Pro Tyr Leu Leu Asp Val
    610                 615                 620

Ile Val Pro His Gln Glu His Val Leu Pro Met Ile Pro Ala Gly Gly
625                 630                 635                 640

Gly Phe Ser Asp Val Ile Thr Glu Gly Asp Gly Arg Thr Lys Tyr
                645                 650                 655

<210>  6
<211>  646
<212>  PRT
<213>  Helianthus annuus

<220>
<221>  MISC_FEATURE

<223>  Sunflower acetohydroxyacid synthase large subunit number
      2(HaAHASL2)

<400>  6

Met Ala Ala Ile His Pro Pro His Pro Ser Ile Thr Ala Lys Pro Pro
1               5                   10                  15

Pro Ser Ser Ala Ala Ala Val Ala Leu Pro Pro His Phe Ala Phe Ser
            20                  25                  30

Ile Thr Ser Thr Ser His Lys Arg His Arg Leu His Ile Ser Asn Val
        35                  40                  45

Leu Ser Asp Ser Thr Thr Thr Thr Gly Ala Thr Thr Ile His Pro Pro
    50                  55                  60

Pro Phe Val Ser Arg Tyr Ala Pro Asp Gln Pro Arg Lys Gly Ala Asp
65                  70                  75                  80

Val Leu Val Glu Ala Leu Glu Arg Glu Gly Val Thr Asp Val Phe Ala
                85                  90                  95

Tyr Pro Gly Gly Ala Ser Met Glu Ile His Gln Ala Leu Thr Arg Ser
            100                 105                 110

Asn Thr Ile Arg Asn Val Leu Pro Arg His Glu Gln Gly Gly Val Phe
        115                 120                 125

Ala Ala Glu Gly Tyr Ala Arg Ala Ser Gly Val Pro Gly Val Cys Ile
    130                 135                 140

Ala Thr Ser Gly Pro Gly Ala Thr Asn Leu Val Ser Gly Leu Ala Asp
145                 150                 155                 160

Ala Leu Leu Asp Ser Val Pro Met Val Ala Ile Thr Gly Gln Val Pro
                165                 170                 175

Arg Arg Met Ile Gly Thr Asp Ala Phe Gln Glu Thr Pro Ile Val Glu
            180                 185                 190

Val Thr Arg Ser Ile Thr Lys His Asn Tyr Leu Val Leu Asp Val Glu
        195                 200                 205

Asp Ile Pro Arg Ile Val Arg Glu Ala Phe Tyr Leu Ala Ser Ser Gly
    210                 215                 220

Arg Pro Gly Pro Val Leu Ile Asp Val Pro Lys Asp Ile Gln Gln Gln
225                 230                 235                 240

Leu Val Val Pro Lys Trp Asp Glu Pro Met Arg Leu Pro Gly Tyr Leu
                245                 250                 255

Ser Arg Met Pro Lys Pro Gln Tyr Asp Gly His Leu Glu Gln Ile Val
            260                 265                 270

Arg Leu Val Gly Glu Ala Lys Arg Pro Val Leu Tyr Val Gly Gly Gly
        275                 280                 285

Cys Leu Asn Ser Asp Asp Glu Leu Arg Arg Phe Val Glu Leu Thr Gly
    290                 295                 300

Ile Pro Val Ala Ser Thr Leu Met Gly Leu Gly Ala Tyr Pro Ala Ser
305                 310                 315                 320

Ser Asp Leu Ser Leu His Met Leu Gly Met His Gly Thr Val Tyr Ala
                325                 330                 335

Asn Tyr Ala Val Asp Lys Ser Asp Leu Leu Leu Ala Phe Gly Val Arg
            340                 345                 350

Phe Asp Asp Arg Val Thr Gly Lys Leu Glu Ala Phe Ala Ser Arg Ala
        355                 360                 365

Lys Ile Val His Ile Asp Ile Asp Pro Ala Glu Ile Gly Lys Asn Lys
    370                 375                 380

Gln Pro His Val Ser Ile Cys Gly Asp Ile Lys Val Ala Leu Gln Gly
385                 390                 395                 400

Leu Asn Lys Ile Leu Glu Glu Lys Asn Ser Leu Asp Phe Ser Asn Trp
                405                 410                 415

Arg Lys Glu Leu Asp Glu Gln Lys Val Lys Phe Pro Leu Ser Phe Lys
            420                 425                 430

Thr Phe Gly Glu Ala Ile Pro Pro Gln Tyr Ala Ile His Val Leu Asp
        435                 440                 445

Glu Leu Thr Gly Gly Asn Ala Ile Ile Ser Thr Gly Val Gly Gln His
    450                 455                 460

Gln Met Trp Ala Ala Gln Phe Tyr Lys Tyr Asn Lys Pro Arg Gln Trp
465                 470                 475                 480

Leu Thr Ser Gly Gly Leu Gly Ala Met Gly Phe Gly Leu Pro Ala Ala
                485                 490                 495

Ile Gly Ala Ala Val Ala Arg Pro Asp Ala Val Val Val Asp Ile Asp

500                 505                 510

Gly Asp Gly Ser Phe Met Met Asn Val Gln Glu Leu Ala Thr Ile Arg
        515                 520                 525

Val Glu Asn Leu Pro Val Lys Ile Leu Leu Leu Asn Asn Gln His Leu
    530                 535                 540

Gly Met Val Val Gln Trp Glu Asp Arg Phe Tyr Lys Ala Asn Arg Ala
545                 550                 555                 560

His Thr Tyr Leu Gly Asn Pro Ser Lys Glu Ser Glu Ile Phe Pro Asn
                565                 570                 575

Met Leu Lys Phe Ala Glu Ala Cys Asp Ile Pro Ala Ala Arg Val Thr
            580                 585                 590

Arg Lys Gly Asp Leu Arg Ala Ala Ile Gln Lys Met Leu Asp Thr Pro
        595                 600                 605

Gly Pro Tyr Leu Leu Asp Val Ile Val Pro His Gln Glu His Val Leu
    610                 615                 620

Pro Met Ile Pro Ala Gly Gly Gly Phe Ser Asp Val Ile Thr Glu Gly
625                 630                 635                 640

Asp Gly Arg Met Lys Tyr
                645

<210>  7
<211>  646
<212>  PRT
<213>  Helianthus annuus

<220>
<221>  misc_feature
<223>  Sunflower acetohydroxyacid synthase large subunit number
       3(HaAHASL3)

<400>  7

Met Ala Val Pro Leu Thr Phe Ile Ser Gly Lys Pro Pro Phe Ser Ala
1               5                   10                  15

Thr Pro Ser Gln Leu Thr Thr Asn Leu Ser Leu Pro Leu Thr Leu Leu
            20                  25                  30

Pro Ile Ile Pro Ser Lys Thr His Val Ser Lys His Leu Ile Ile Thr
        35                  40                  45

Asn Ala Ile Ala Lys His Ser His Ser His Lys Ala Phe Val Val Ser

50                  55                  60

Arg Phe Gly Pro Asp Glu Pro Arg Lys Gly Ser Asp Val Leu Val Glu
65                  70                  75                  80

Ala Leu Glu Arg Glu Gly Val Thr Asn Val Phe Ala Tyr Pro Gly Gly
                85                  90                  95

Ala Thr Leu Glu Ile His Gln Ala Leu Thr Arg Ser Thr Ile Ile Arg
            100                 105                 110

Asn Ile Leu Pro Arg His Glu Gln Gly Gly Val Phe Ala Ala Glu Gly
        115                 120                 125

Tyr Ala Arg Ala Ser Gly Leu Thr Gly Val Cys Ile Ser Thr Ser Gly
    130                 135                 140

Pro Gly Ala Thr Asn Leu Val Ser Gly Leu Ala Asp Ala Leu Leu Asp
145                 150                 155                 160

Ser Val Pro Ile Val Ala Ile Thr Gly Gln Val Pro Arg Asn Met Ile
                165                 170                 175

Gly Thr Asp Ala Phe Gln Glu Thr Pro Ile Val Glu Val Ser Arg Ser
            180                 185                 190

Ile Thr Lys His Asn Tyr Leu Val Leu Asn Val Gln Asp Ile Pro Arg
        195                 200                 205

Ile Val His Glu Ala Phe Phe Leu Ala Ser Ser Gly Arg Pro Gly Pro
    210                 215                 220

Val Leu Ile Asp Ile Pro Lys Asp Ile Gln Gln Gln Leu Val Val Pro
225                 230                 235                 240

Asn Trp Asp Glu Gln Gln Gln Pro Met Arg Leu Asp Gly Tyr Ile Ser
                245                 250                 255

Arg Leu Pro Lys Pro Pro Asn Glu Thr His Leu Arg Gln Ile Val Arg
            260                 265                 270

Phe Ile Lys Glu Ser Lys Arg Pro Val Leu Tyr Val Gly Gly Gly Cys
        275                 280                 285

Met Asn Ser Ser Asp Glu Leu Gly Arg Phe Val Glu Leu Thr Gly Ile
    290                 295                 300

Pro Val Ala Asn Thr Leu Met Gly Leu Gly Thr Tyr Pro Gly Ser His
305                 310                 315                 320

Asp Leu Ser Leu His Met Leu Gly Met His Gly Thr Val Tyr Ala Asn
                325                 330                 335

Tyr Ala Ile Asp Lys Ser Asp Leu Leu Leu Ala Phe Gly Val Arg Phe
            340                 345                 350

Asp Asp Arg Val Thr Gly Lys Ile Glu Ala Phe Ala Ser Arg Ala Lys
        355                 360                 365

Ile Val His Ile Asp Ile Asp Pro Ala Glu Ile Gly Lys Asn Lys Gln
    370                 375                 380

Pro His Phe Ser Ile Cys Gly Asp Ile Lys Ala Ala Leu Gln Gly Leu
385                 390                 395                 400

Asn Lys Ile Leu Glu Arg Gly Glu Asp Leu Asp Phe Asp Phe Ser Pro
                405                 410                 415

Trp Lys Glu Glu Val Met Asn Gln Lys Ala Ser Asn Pro Leu Ser Tyr
            420                 425                 430

Lys Thr Phe Gly Asp Ala Ile Pro Pro Gln Tyr Ala Ile Gln Val Leu
        435                 440                 445

Asn Glu Val Thr Gly Gly Asn Ala Ile Ile Thr Thr Gly Val Gly Gln
    450                 455                 460

His Gln Met Trp Ser Ala Gln Phe Tyr Lys Tyr Asn Arg Pro Arg Gln
465                 470                 475                 480

Trp Leu Thr Ser Ala Gly Leu Gly Ala Met Gly Phe Gly Leu Pro Ala
                485                 490                 495

Ala Ile Gly Ala Ala Val Ala Arg Pro Asp Ala Val Val Val Asp Ile
            500                 505                 510

Asp Gly Asp Gly Ser Phe Met Met Asn Val Gln Glu Leu Ala Thr Ile
        515                 520                 525

Arg Val Glu Asn Leu Pro Val Lys Met Met Val Leu Asn Asn Gln His
    530                 535                 540

Leu Gly Met Val Val Gln Trp Glu Asp Arg Phe Tyr Lys Ala Asn Arg
545                 550                 555                 560

Ala His Thr Tyr Leu Gly Asn Pro Thr Asn Glu Ser Gly Ile Phe Pro
                565                 570                 575

Asn Met Leu Lys Phe Ala Glu Ala Cys Asp Ile Pro Ala Ala Arg Val
            580                 585                 590

Thr Lys Lys Gly Asp Val Arg Thr Ala Ile Gln Lys Met Leu Asp Thr
        595                 600                 605

Pro Gly Pro Tyr Leu Leu Asp Val Ile Val Pro His Gln Glu His Val
    610                 615                 620

Leu Pro Met Ile Pro Ala Gly Gly Gly Phe Asn Asp Ile Ile Thr Asp
625                 630                 635                 640

Gly Asp Gly Arg Thr Gln
                645

<210>  8
<211>  638
<212>  PRT
<213>  Zea mays

<220>
<221>  MISC_FEATURE
<223>  Zea mays acetohydroxyacid synthase large subunit number
       1(ZmAHASL1)

<400>  8

Met Ala Thr Ala Ala Thr Ala Ala Ala Ala Leu Thr Gly Ala Thr Thr
1               5                   10                  15

Ala Thr Pro Lys Ser Arg Arg Arg Ala His His Leu Ala Thr Arg Arg
            20                  25                  30

Ala Leu Ala Ala Pro Ile Arg Cys Ser Ala Leu Ser Arg Ala Thr Pro
        35                  40                  45

Thr Ala Pro Pro Ala Thr Pro Leu Arg Pro Trp Gly Pro Asn Glu Pro
    50                  55                  60

Arg Lys Gly Ser Asp Ile Leu Val Glu Ala Leu Glu Arg Cys Gly Val
65                  70                  75                  80

Arg Asp Val Phe Ala Tyr Pro Gly Gly Ala Ser Met Glu Ile His Gln
                85                  90                  95

Ala Leu Thr Arg Ser Pro Val Ile Ala Asn His Leu Phe Arg His Glu
            100                 105                 110

Gln Gly Glu Ala Phe Ala Ala Ser Ala Tyr Ala Arg Ser Ser Gly Arg
        115                 120                 125

Val Gly Val Cys Ile Ala Thr Ser Gly Pro Gly Ala Thr Asn Leu Val
    130                 135                 140

Ser Ala Leu Ala Asp Ala Leu Leu Asp Ser Val Pro Met Val Ala Ile
145                 150                 155                 160

Thr Gly Gln Val Pro Arg Arg Met Ile Gly Thr Asp Ala Phe Gln Glu
                165                 170                 175

Thr Pro Ile Val Glu Val Thr Arg Ser Ile Thr Lys His Asn Tyr Leu
            180                 185                 190

Val Leu Asp Val Asp Asp Ile Pro Arg Val Val Gln Glu Ala Phe Phe
        195                 200                 205

Leu Ala Ser Ser Gly Arg Pro Gly Pro Val Leu Val Asp Ile Pro Lys
    210                 215                 220

Asp Ile Gln Gln Gln Met Ala Val Pro Ala Trp Asp Thr Pro Met Ser
225                 230                 235                 240

Leu Pro Gly Tyr Ile Ala Arg Leu Pro Lys Pro Pro Ala Thr Glu Phe
                245                 250                 255

Leu Glu Gln Val Leu Arg Leu Val Gly Glu Ser Arg Arg Pro Val Leu
            260                 265                 270

Tyr Val Gly Gly Gly Cys Ala Ala Ser Gly Glu Glu Leu Cys Arg Phe
        275                 280                 285

Val Glu Leu Thr Gly Ile Pro Val Thr Thr Thr Leu Met Gly Leu Gly
    290                 295                 300

Asn Phe Pro Ser Asp Asp Pro Leu Ser Leu Arg Met Leu Gly Met His
305                 310                 315                 320

Gly Thr Val Tyr Ala Asn Tyr Ala Val Asp Lys Ala Asp Leu Leu Leu
                325                 330                 335

Ala Phe Gly Val Arg Phe Asp Asp Arg Val Thr Gly Lys Ile Glu Ala
            340                 345                 350

Phe Ala Gly Arg Ala Lys Ile Val His Ile Asp Ile Asp Pro Ala Glu
        355                 360                 365

Ile Gly Lys Asn Lys Gln Pro His Val Ser Ile Cys Ala Asp Val Lys
    370                 375                 380

Leu Ala Leu Gln Gly Met Asn Thr Leu Leu Glu Gly Ser Thr Ser Lys
385                 390                 395                 400

Lys Ser Phe Asp Phe Gly Ser Trp His Asp Glu Leu Asp Gln Gln Lys
                405                 410                 415

Arg Glu Phe Pro Leu Gly Tyr Lys Ile Phe Asn Glu Glu Ile Gln Pro
            420                 425                 430

Gln Tyr Ala Ile Gln Val Leu Asp Glu Leu Thr Lys Gly Glu Ala Ile
        435                 440                 445

Ile Ala Thr Gly Val Gly Gln His Gln Met Trp Ala Ala Gln Tyr Tyr
    450                 455                 460

Thr Tyr Lys Arg Pro Arg Gln Trp Leu Ser Ser Ala Gly Leu Gly Ala
465                 470                 475                 480

Met Gly Phe Gly Leu Pro Ala Ala Ala Gly Ala Ala Val Ala Asn Pro
                485                 490                 495

Gly Val Thr Val Val Asp Ile Asp Gly Asp Gly Ser Phe Leu Met Asn
            500                 505                 510

Ile Gln Glu Leu Ala Met Ile Arg Ile Glu Asn Leu Pro Val Lys Val
        515                 520                 525

Phe Val Leu Asn Asn Gln His Leu Gly Met Val Val Gln Trp Glu Asp
    530                 535                 540

Arg Phe Tyr Lys Ala Asn Arg Ala His Thr Phe Leu Gly Asn Pro Glu
545                 550                 555                 560

Asn Glu Ser Glu Ile Tyr Pro Asp Phe Val Ala Ile Ala Lys Gly Phe
                565                 570                 575

Asn Ile Pro Ala Val Arg Val Thr Lys Lys Ser Glu Val His Ala Ala
            580                 585                 590

Ile Lys Lys Met Leu Glu Ala Pro Gly Pro Tyr Leu Leu Asp Ile Ile
        595                 600                 605

Val Pro His Gln Glu His Val Leu Pro Met Ile Pro Ser Gly Gly Ala
    610                 615                 620

Phe Lys Asp Met Ile Leu Asp Gly Asp Gly Arg Thr Val Tyr
625                 630                 635

<210>  9

<211>  665
<212>  PRT
<213>  Amaranthus sp.

<220>
<221>  MISC_FEATURE
<223>  Amaranthus acetohydroxyacid synthase large subunit (AsAHASL)

<400>  9

Met Val Thr Leu Asn His Ile Ser Ser Phe Thr Lys Pro Asn Lys Thr
1               5                   10                  15

Tyr Leu Gln Ser Ser Ile Tyr Ala Ile Pro Phe Ser Asn Ser Leu Lys
            20                  25                  30

Pro Thr Ser Ser Ser Ser Ile Leu Ala Ala Leu Phe Lys Ser His His
        35                  40                  45

Leu Leu Leu Gln Ser Pro Lys Pro Lys Pro Pro Ser Ala Thr Ile Thr
    50                  55                  60

Gln Ser Pro Ser Ser Leu Thr Asp Asp Lys Pro Ser Ser Phe Val Ser
65                  70                  75                  80

Arg Phe Ser Pro Glu Glu Pro Arg Lys Gly Cys Asp Val Leu Val Glu
                85                  90                  95

Ala Leu Glu Arg Glu Gly Val Thr Asp Val Phe Ala Tyr Pro Gly Gly
            100                 105                 110

Ala Ser Met Glu Ile His Gln Ala Leu Thr Arg Ser Asn Ile Ile Arg
        115                 120                 125

Asn Val Leu Pro Arg His Glu Gln Gly Gly Val Phe Ala Ala Gln Gly
    130                 135                 140

Tyr Ala Arg Ala Thr Gly Arg Val Gly Val Cys Ile Ala Thr Ser Gly
145                 150                 155                 160

Pro Gly Ala Thr Asn Leu Val Ser Gly Phe Ala Asp Ala Leu Leu Asp
                165                 170                 175

Ser Val Pro Leu Val Ala Ile Thr Gly Gln Val Pro Arg Arg Met Ile
            180                 185                 190

Gly Thr Asp Ala Phe Gln Glu Thr Pro Ile Val Glu Val Thr Arg Ser
        195                 200                 205

Ile Thr Lys His Asn Tyr Leu Val Leu Asp Val Glu Asp Ile Pro Arg
    210                 215                 220

Ile Val Lys Glu Ala Phe Phe Leu Ala Asn Ser Gly Arg Pro Gly Pro
225                 230                 235                 240

Val Leu Ile Asp Ile Pro Lys Asp Ile Gln Gln Gln Leu Val Val Pro
                245                 250                 255

Asn Trp Glu Gln Pro Ile Lys Leu Gly Gly Tyr Leu Ser Arg Leu Pro
            260                 265                 270

Lys Pro Thr Phe Ser Ala Asn Glu Glu Gly Leu Leu Asp Gln Ile Val
        275                 280                 285

Arg Leu Val Gly Glu Ser Lys Arg Pro Val Leu Tyr Thr Gly Gly Gly
    290                 295                 300

Cys Leu Asn Ser Ser Glu Glu Leu Arg Lys Phe Val Glu Leu Thr Gly
305                 310                 315                 320

Ile Pro Val Ala Ser Thr Leu Met Gly Leu Gly Ala Phe Pro Cys Thr
                325                 330                 335

Asp Asp Leu Ser Leu Gln Met Leu Gly Met His Gly Thr Val Tyr Ala
            340                 345                 350

Asn Tyr Ala Val Asp Lys Ala Asp Leu Leu Leu Ala Phe Gly Val Arg
        355                 360                 365

Phe Asp Asp Arg Val Thr Gly Lys Leu Glu Ala Phe Ala Ser Arg Ala
    370                 375                 380

Lys Ile Val His Ile Asp Ile Asp Ser Ala Glu Ile Gly Lys Asn Lys
385                 390                 395                 400

Gln Pro His Val Ser Ile Cys Gly Glu Ile Lys Val Ala Leu Gln Gly
                405                 410                 415

Leu Asn Lys Ile Leu Glu Ser Arg Lys Gly Lys Leu Lys Leu Asp Phe
            420                 425                 430

Ser Asn Trp Arg Glu Glu Leu Asn Glu Gln Lys Lys Lys Phe Pro Leu
        435                 440                 445

Ser Phe Lys Ser Phe Gly Asp Ala Ile Pro Pro Gln Tyr Ala Ile Gln
    450                 455                 460

Val Leu Asp Glu Leu Thr Leu Gly Asp Ala Ile Val Ser Thr Gly Val
465                 470                 475                 480

Gly Gln His Gln Met Trp Ala Ala Gln Phe Tyr Lys Tyr Arg Asn Pro
                485                 490                 495

Arg Gln Trp Leu Thr Ser Gly Gly Leu Gly Ala Met Gly Phe Gly Leu
            500                 505                 510

Pro Ala Cys Tyr Gly Ala Ala Val Ala Arg Pro Asp Ala Val Val Val
        515                 520                 525

Asp Ile Asp Gly Asp Gly Ser Phe Ile Met Asn Val Gln Glu Leu Ala
    530                 535                 540

Thr Ile Arg Val Glu Asn Leu Pro Val Lys Ile Met Leu Leu Asn Asn
545                 550                 555                 560

Gln His Leu Gly Met Val Val Gln Trp Glu Asp Arg Phe Tyr Lys Ala
                565                 570                 575

Asn Arg Ala His Thr Tyr Leu Gly Asn Pro Ser Asn Ser Ser Glu Ile
            580                 585                 590

Phe Pro Asp Met Leu Lys Phe Ala Glu Ala Cys Asp Ile Pro Ala Ala
        595                 600                 605

Arg Val Thr Lys Val Ser Asp Leu Arg Ala Ala Ile Gln Thr Met Leu
    610                 615                 620

Asp Thr Pro Gly Pro Tyr Leu Leu Asp Val Ile Val Pro His Gln Glu
625                 630                 635                 640

His Val Leu Pro Met Ile Pro Ser Gly Ala Ala Phe Lys Asp Thr Ile
                645                 650                 655

Thr Glu Gly Asp Gly Arg Arg Ala Tyr
            660                 665

<210>  10
<211>  655
<212>  PRT
<213>  Brassica napus

<220>
<221>  MISC_FEATURE
<223>  Brassica napus acetohydroxyacid synthase C genome large subunit
       number 1 (BnAHASL1C)

<400>  10

Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30
Leu Thr Pro Gln Lys Asp Ser Ser Arg Leu His Arg Pro Leu Ala Ile
        35                  40                  45
Ser Ala Val Leu Asn Ser Pro Val Asn Val Ala Pro Pro Ser Pro Glu
    50                  55                  60
Lys Thr Asp Lys Asn Lys Thr Phe Val Ser Arg Tyr Ala Pro Asp Glu
65                  70                  75                  80
Pro Arg Lys Gly Ala Asp Ile Leu Val Glu Ala Leu Glu Arg Gln Gly
                85                  90                  95
Val Glu Thr Val Phe Ala Tyr Pro Gly Gly Ala Ser Met Glu Ile His
            100                 105                 110
Gln Ala Leu Thr Arg Ser Ser Thr Ile Arg Asn Val Leu Pro Arg His
        115                 120                 125
Glu Gln Gly Gly Val Phe Ala Ala Glu Gly Tyr Ala Arg Ser Ser Gly
    130                 135                 140
Lys Pro Gly Ile Cys Ile Ala Thr Ser Gly Pro Gly Ala Thr Asn Leu
145                 150                 155                 160
Val Ser Gly Leu Ala Asp Ala Met Leu Asp Ser Val Pro Leu Val Ala
                165                 170                 175
Ile Thr Gly Gln Val Pro Arg Arg Met Ile Gly Thr Asp Ala Phe Gln
            180                 185                 190
Glu Thr Pro Ile Val Glu Val Thr Arg Ser Ile Thr Lys His Asn Tyr
        195                 200                 205
Leu Val Met Asp Val Asp Asp Ile Pro Arg Ile Val Gln Glu Ala Phe
    210                 215                 220
Phe Leu Ala Thr Ser Gly Arg Pro Gly Pro Val Leu Val Asp Val Pro
225                 230                 235                 240
Lys Asp Ile Gln Gln Gln Leu Ala Ile Pro Asn Trp Asp Gln Pro Met
                245                 250                 255
Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys

530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Ala Gly Ala
            580                 585                 590

Cys Gly Ile Pro Ala Ala Arg Val Thr Lys Lys Glu Glu Leu Arg Glu
        595                 600                 605

Ala Ile Gln Thr Met Leu Asp Thr Pro Gly Pro Tyr Leu Leu Asp Val
    610                 615                 620

Ile Cys Pro His Gln Glu His Val Leu Pro Met Ile Pro Ser Gly Gly
625                 630                 635                 640

Thr Phe Lys Asp Val Ile Thr Glu Gly Asp Gly Arg Thr Lys Tyr
                645                 650                 655

<210>  11
<211>  637
<212>  PRT
<213>  Brassica napus

<220>
<221>  MISC_FEATURE
<223>  Brassica napus acetohydroxyacid synthase A genome large subunit
       number 2 (BnAHASL2A)

<400>  11

Met Ala Ser Phe Ser Phe Phe Gly Thr Ile Pro Ser Ser Pro Thr Lys
1               5                   10                  15

Ala Ser Val Phe Ser Leu Pro Val Ser Val Thr Thr Leu Pro Ser Phe
            20                  25                  30

Pro Arg Arg Arg Ala Thr Arg Val Ser Val Ser Ala Asn Ser Lys Lys
        35                  40                  45

Asp Gln Asp Arg Thr Ala Ser Arg Arg Glu Asn Pro Ser Thr Phe Ser
    50                  55                  60

Ser Lys Tyr Ala Pro Asn Val Pro Arg Ser Gly Ala Asp Ile Leu Val
65                  70                  75                  80

Glu Ala Leu Glu Arg Gln Gly Val Asp Val Val Phe Ala Tyr Pro Gly

                85                  90                  95

Gly Ala Ser Met Glu Ile His Gln Ala Leu Thr Arg Ser Asn Thr Ile
            100                 105                 110

Arg Asn Val Leu Pro Arg His Glu Gln Gly Gly Ile Phe Ala Ala Glu
        115                 120                 125

Gly Tyr Ala Arg Ser Ser Gly Lys Pro Gly Ile Cys Ile Ala Thr Ser
    130                 135                 140

Gly Pro Gly Ala Met Asn Leu Val Ser Gly Leu Ala Asp Ala Leu Phe
145                 150                 155                 160

Asp Ser Val Pro Leu Ile Ala Ile Thr Gly Gln Val Pro Arg Arg Met
                165                 170                 175

Ile Gly Thr Met Ala Phe Gln Glu Thr Pro Val Val Glu Val Thr Arg
            180                 185                 190

Thr Ile Thr Lys His Asn Tyr Leu Val Met Glu Val Asp Asp Ile Pro
        195                 200                 205

Arg Ile Val Arg Glu Ala Phe Phe Leu Ala Thr Ser Val Arg Pro Gly
    210                 215                 220

Pro Val Leu Ile Asp Val Pro Lys Asp Val Gln Gln Gln Phe Ala Ile
225                 230                 235                 240

Pro Asn Trp Glu Gln Pro Met Arg Leu Pro Leu Tyr Met Ser Thr Met
                245                 250                 255

Pro Lys Pro Pro Lys Val Ser His Leu Glu Gln Ile Leu Arg Leu Val
            260                 265                 270

Ser Glu Ser Lys Arg Pro Val Leu Tyr Val Gly Gly Gly Cys Leu Asn
        275                 280                 285

Ser Ser Glu Glu Leu Arg Arg Phe Val Glu Leu Thr Gly Ile Pro Val
    290                 295                 300

Ala Ser Thr Phe Met Gly Leu Gly Ser Tyr Pro Cys Asp Asp Glu Glu
305                 310                 315                 320

Phe Ser Leu Gln Met Leu Gly Met His Gly Thr Val Tyr Ala Asn Tyr
                325                 330                 335

Ala Val Glu Tyr Ser Asp Leu Leu Leu Ala Phe Gly Val Arg Phe Asp
            340                 345                 350

Asp Arg Val Thr Gly Lys Leu Glu Ala Phe Ala Ser Arg Ala Lys Ile
        355                 360                 365

Val His Ile Asp Ile Asp Ser Thr Glu Ile Gly Lys Asn Lys Thr Pro
    370                 375                 380

His Val Ser Val Cys Cys Asp Val Gln Leu Ala Leu Gln Gly Met Asn
385                 390                 395                 400

Glu Val Leu Glu Asn Arg Arg Asp Val Leu Asp Phe Gly Glu Trp Arg
                405                 410                 415

Cys Glu Leu Asn Glu Gln Arg Leu Lys Phe Pro Leu Arg Tyr Lys Thr
            420                 425                 430

Phe Gly Glu Glu Ile Pro Pro Gln Tyr Ala Ile Gln Leu Leu Asp Glu
        435                 440                 445

Leu Thr Asp Gly Lys Ala Ile Ile Thr Thr Gly Val Gly Gln His Gln
    450                 455                 460

Met Trp Ala Ala Gln Phe Tyr Arg Phe Lys Lys Pro Arg Gln Trp Leu
465                 470                 475                 480

Ser Ser Gly Gly Leu Gly Ala Met Gly Phe Gly Leu Pro Ala Ala Met
                485                 490                 495

Gly Ala Ala Ile Ala Asn Pro Gly Ala Val Val Val Asp Ile Asp Gly
            500                 505                 510

Asp Gly Ser Phe Ile Met Asn Ile Gln Glu Leu Ala Thr Ile Arg Val
        515                 520                 525

Glu Asn Leu Pro Val Lys Val Leu Leu Ile Asn Asn Gln His Leu Gly
    530                 535                 540

Met Val Leu Gln Trp Glu Asp His Phe Tyr Ala Ala Asn Arg Ala Asp
545                 550                 555                 560

Ser Phe Leu Gly Asp Pro Ala Asn Pro Glu Ala Val Phe Pro Asp Met
                565                 570                 575

Leu Leu Phe Ala Ala Ser Cys Gly Ile Pro Ala Ala Arg Val Thr Arg
            580                 585                 590

Arg Glu Asp Leu Arg Glu Ala Ile Gln Thr Met Leu Asp Thr Pro Gly
        595                 600                 605

Pro Phe Leu Leu Asp Val Val Cys Pro His Gln Asp His Val Leu Pro
    610                 615                 620

Leu Ile Pro Ser Gly Gly Thr Phe Lys Asp Ile Ile Val
625                 630                 635

<210>  12
<211>  668
<212>  PRT
<213>  Camelina microcarpa

<220>
<221>  MISC_FEATURE
<223>  Camelina microcarpa acetohydroxyacid synthase large subunit
       number 1 (CmAHASL1)

<400>  12

Met Ala Ala Ala Thr Thr Thr Ser Ser Ser Ser Ile Pro Phe Ser Thr
1               5                   10                  15

Lys His Ser Ser Ser Lys Ser Pro Leu Pro Ile Ser Arg Phe Thr Leu
            20                  25                  30

Pro Phe Ser Leu Asn Pro Asn Lys Ser Ser Ser Ser Ser Ser Arg Arg
        35                  40                  45

Arg Gly Ile Lys Ser Thr Ala Leu Ser Ile Ser Ala Val Leu Asn Thr
    50                  55                  60

Thr Thr Asn Val Ser Thr Thr Thr Pro Gln Ser Lys Pro Thr Lys Pro
65                  70                  75                  80

Glu Lys Lys Lys Phe Val Ser Arg Phe Ala Pro Asp Gln Pro Arg Lys
                85                  90                  95

Gly Ala Asp Ile Leu Val Glu Ala Leu Glu Arg Gln Gly Val Glu Thr
            100                 105                 110

Ala Phe Ala Tyr Pro Gly Gly Ala Ser Met Glu Ile His Gln Ala Leu
        115                 120                 125

Thr Arg Ser Ser Ser Ile Arg Asn Val Leu Pro Arg His Glu Gln Gly
    130                 135                 140

Gly Val Phe Ala Ala Glu Gly Tyr Ala Arg Ser Thr Gly Lys Pro Gly
145                 150                 155                 160

Ile Cys Ile Ala Thr Ser Gly Pro Gly Ala Thr Asn Leu Val Ser Gly
                165                 170                 175

Leu Ala Asp Ala Leu Leu Asp Ser Val Pro Leu Val Ala Ile Thr Gly
            180                 185                 190

Gln Val Pro Arg Arg Met Ile Gly Thr Asp Ala Phe Gln Glu Thr Pro
        195                 200                 205

Ile Val Glu Val Thr Arg Ser Ile Thr Lys His Asn Tyr Leu Val Met
    210                 215                 220

Asp Val Glu Asp Ile Pro Arg Ile Val Glu Glu Ala Phe Phe Leu Ala
225                 230                 235                 240

Thr Ser Gly Arg Pro Gly Pro Val Leu Val Asp Val Pro Lys Asp Ile
                245                 250                 255

Gln Gln Gln Leu Ala Ile Pro Asn Trp Glu Gln Ser Met Arg Leu Pro
            260                 265                 270

Gly Tyr Met Ser Arg Met Pro Lys Pro Pro Glu Asp Ser His Leu Glu
        275                 280                 285

Gln Ile Val Arg Leu Ile Ser Glu Ser Lys Lys Pro Val Leu Tyr Val
    290                 295                 300

Gly Gly Gly Cys Leu Asn Ser Ser Glu Glu Leu Gly Arg Phe Val Glu
305                 310                 315                 320

Leu Thr Gly Ile Pro Val Ala Ser Thr Leu Met Gly Leu Gly Ala Tyr
                325                 330                 335

Pro Cys Asp Asp Glu Leu Ser Leu His Met Leu Gly Met His Gly Thr
            340                 345                 350

Val Tyr Ala Asn Tyr Ser Val Glu His Ser Asp Leu Leu Leu Ala Phe
        355                 360                 365

Gly Val Arg Phe Asp Asp Arg Val Thr Gly Lys Leu Glu Ala Phe Ala
    370                 375                 380

Ser Arg Ala Lys Ile Val His Ile Asp Ile Asp Ser Ala Glu Ile Gly
385                 390                 395                 400

Lys Asn Lys Thr Pro His Val Ser Val Cys Gly Asp Val Lys Leu Ala
                405                 410                 415

Leu Gln Gly Met Asn Lys Val Leu Glu Asn Arg Ala Glu Glu Leu Lys
            420                 425                 430

Leu Asp Phe Gly Val Trp Arg Ser Glu Leu Asn Glu Gln Lys Gln Lys
        435                 440                 445

Phe Pro Leu Ser Phe Lys Thr Phe Gly Glu Ala Ile Pro Pro Gln Tyr
    450                 455                 460

Ala Ile Gln Val Leu Asp Glu Leu Thr Asp Gly Arg Ala Ile Ile Ser
465                 470                 475                 480

Thr Gly Val Gly Gln His Gln Met Trp Ala Ala Gln Phe Tyr Lys Tyr
                485                 490                 495

Lys Lys Pro Arg Gln Trp Leu Ser Ser Ala Gly Leu Gly Ala Met Gly
            500                 505                 510

Phe Gly Leu Pro Ala Ala Ile Gly Ala Ser Val Ala Asn Pro Asp Ser
        515                 520                 525

Ile Val Val Asp Ile Asp Gly Asp Gly Ser Phe Ile Met Asn Val Gln
    530                 535                 540

Glu Leu Ala Thr Ile Arg Val Glu Asn Leu Pro Val Lys Ile Leu Ile
545                 550                 555                 560

Leu Asn Asn Gln His Leu Gly Met Val Met Gln Trp Glu Asp Arg Phe
                565                 570                 575

Tyr Lys Ala Asn Arg Ala His Thr Tyr Leu Gly Asn Pro Ala Glu Glu
            580                 585                 590

Asp Glu Ile Phe Pro Asn Met Leu Gln Phe Ala Ser Ala Cys Gly Ile
        595                 600                 605

Pro Ala Ala Arg Val Thr Lys Ile Ala Glu Leu Arg Glu Ala Ile Gln
    610                 615                 620

Lys Met Leu Asp Thr Pro Gly Pro Tyr Leu Leu Asp Val Ile Cys Pro
625                 630                 635                 640

His Gln Glu His Val Leu Pro Met Ile Pro Ser Gly Gly Thr Phe Asn
                645                 650                 655

Asp Val Ile Thr Glu Gly Asp Gly Arg Thr Lys Tyr
            660                 665

<210>  13
<211>  665
<212>  PRT
<213>  Camelina microcarpa

<220>
<221>  MISC_FEATURE
<223>  Camelina microcarpa acetohydroxyacid synthase large subunit
       number 2 (CmAHASL2)

<400>  13

Met Ala Ala Ala Thr Thr Thr Ser Ser Ser Ser Ile Pro Phe Ser Thr
1               5                   10                  15

Lys Pro Ser Ser Ser Lys Ser Pro Leu Pro Ile Ser Arg Phe Thr Leu
            20                  25                  30

Pro Phe Ser Leu Asn Pro Asn Lys Ser Ser Ser Arg Arg Arg Gly Ile
        35                  40                  45

Lys Ser Thr Ser Leu Ser Ile Ser Ala Val Leu Asn Thr Thr Thr Asn
    50                  55                  60

Val Ser Thr Thr Thr Pro Pro Ser Lys Pro Thr Lys Pro Glu Lys Lys
65                  70                  75                  80

Lys Phe Val Ser Arg Phe Ala Pro Asp Gln Pro Arg Lys Gly Ala Asp
                85                  90                  95

Ile Leu Val Glu Ala Leu Glu Arg Gln Gly Val Glu Thr Val Phe Ala
            100                 105                 110

Tyr Pro Gly Gly Ala Ser Met Glu Ile His Gln Ala Leu Thr Arg Ser
        115                 120                 125

Ser Ser Ile Arg Asn Val Leu Pro Arg His Glu Gln Gly Gly Val Phe
    130                 135                 140

Ala Ala Glu Gly Tyr Ala Arg Ser Thr Gly Lys Pro Gly Ile Cys Ile
145                 150                 155                 160

Ala Thr Ser Gly Pro Gly Ala Thr Asn Leu Val Ser Gly Leu Ala Asp
                165                 170                 175

Ala Leu Leu Asp Ser Val Pro Leu Val Ala Ile Thr Gly Gln Val Pro
            180                 185                 190

Arg Arg Met Ile Gly Thr Asp Ala Phe Gln Glu Thr Pro Ile Val Glu
        195                 200                 205

Val Thr Arg Ser Ile Thr Lys His Asn Tyr Leu Val Met Asp Val Glu
    210                 215                 220

Asp Ile Pro Arg Ile Val Glu Glu Ala Phe Phe Leu Ala Thr Ser Gly

225                 230                 235                 240

Arg Pro Gly Pro Val Leu Val Asp Val Pro Lys Asp Ile Gln Gln Gln
                245                 250                 255

Leu Ala Ile Pro Asn Trp Glu Gln Ser Met Arg Leu Pro Gly Tyr Met
            260                 265                 270

Ser Arg Met Pro Lys Pro Pro Glu Asp Ser His Leu Glu Gln Ile Val
        275                 280                 285

Arg Leu Ile Ser Glu Ser Lys Lys Pro Val Leu Tyr Val Gly Gly Gly
    290                 295                 300

Cys Leu Asn Ser Ser Glu Glu Leu Gly Arg Phe Val Glu Leu Thr Gly
305                 310                 315                 320

Ile Pro Val Ala Ser Thr Leu Met Gly Leu Gly Ala Tyr Pro Cys Asp
                325                 330                 335

Asp Glu Leu Ser Leu His Met Leu Gly Met His Gly Thr Val Tyr Ala
            340                 345                 350

Asn Tyr Ser Val Glu His Ser Asp Leu Leu Leu Ala Phe Gly Val Arg
        355                 360                 365

Phe Asp Asp Arg Val Thr Gly Lys Leu Glu Ala Phe Ala Ser Arg Ala
    370                 375                 380

Lys Ile Val His Ile Asp Ile Asp Ser Ala Glu Ile Gly Lys Asn Lys
385                 390                 395                 400

Thr Pro His Val Ser Val Cys Gly Asp Val Lys Leu Ala Leu Gln Gly
                405                 410                 415

Met Asn Lys Val Leu Glu Asn Arg Gly Glu Glu Leu Lys Leu Asp Phe
            420                 425                 430

Gly Val Trp Arg Ser Glu Leu Asn Glu Gln Lys Gln Lys Phe Pro Leu
        435                 440                 445

Ser Phe Lys Thr Phe Gly Glu Ala Ile Pro Pro Gln Tyr Ala Ile Gln
    450                 455                 460

Val Leu Asp Glu Leu Thr Asp Gly Lys Ala Ile Ile Ser Thr Gly Val
465                 470                 475                 480

Gly Gln His Gln Met Trp Ala Ala Gln Phe Tyr Lys Tyr Lys Lys Pro
                485                 490                 495

Arg Gln Trp Leu Ser Ser Ala Gly Leu Gly Ala Met Gly Phe Gly Leu
                500                 505                 510

Pro Ala Ala Ile Gly Ala Ser Val Ala Asn Pro Asp Ala Val Val Val
        515                 520                 525

Asp Ile Asp Gly Asp Gly Ser Phe Ile Met Asn Val Gln Glu Leu Ala
    530                 535                 540

Thr Ile Arg Val Glu Asn Leu Pro Val Lys Ile Leu Ile Leu Asn Asn
545                 550                 555                 560

Gln His Leu Gly Met Val Met Gln Trp Glu Asp Arg Phe Tyr Lys Ala
                565                 570                 575

Asn Arg Ala His Thr Tyr Leu Gly Asn Pro Ala Thr Glu Asp Glu Ile
                580                 585                 590

Phe Pro Asn Met Leu Gln Phe Ala Ser Ala Cys Gly Ile Pro Ser Ala
        595                 600                 605

Arg Val Thr Lys Lys Ala Glu Leu Arg Glu Ala Ile Gln Lys Met Leu
    610                 615                 620

Asp Thr Pro Gly Pro Tyr Leu Leu Asp Val Ile Cys Pro His Gln Glu
625                 630                 635                 640

His Val Leu Pro Met Ile Pro Ser Gly Gly Thr Phe Asn Asp Val Ile
                645                 650                 655

Thr Glu Gly Asp Gly Arg Thr Lys Tyr
                660                 665

<210>  14
<211>  567
<212>  PRT
<213>  Solanum ptychanthum

<220>
<221>  MISC_FEATURE
<223>  Partial Solanum ptychanthum acetohydroxyacid synthase large
       subunit (SpAHASL)

<400>  14

Pro Arg Lys Gly Cys Asp Val Leu Val Glu Ala Leu Glu Arg Glu Gly
1               5                   10                  15

Val Thr Asp Val Phe Ala Tyr Pro Gly Gly Thr Ser Met Glu Ile His
                20                  25                  30

Gln Ala Leu Thr Arg Ser Asn Ile Ile Arg Asn Val Leu Pro Arg His
        35                  40                  45

Glu Gln Gly Gly Val Phe Ala Ala Glu Gly Tyr Ala Arg Ala Thr Gly
    50                  55                  60

Phe Pro Gly Val Cys Ile Ala Thr Ser Gly Pro Gly Ala Thr Asn Leu
65                  70                  75                  80

Val Ser Gly Leu Ala Asp Ala Leu Leu Asp Ser Ile Pro Ile Val Ala
                85                  90                  95

Ile Thr Gly Gln Val Pro Arg Arg Met Ile Gly Thr Asp Ala Phe Gln
            100                 105                 110

Glu Thr Pro Ile Val Glu Val Thr Arg Ser Ile Thr Lys His Asn Tyr
        115                 120                 125

Leu Val Met Asp Val Glu Asp Ile Pro Arg Ile Val Arg Glu Ala Phe
    130                 135                 140

Phe Leu Ala Lys Ser Gly Arg Pro Gly Pro Val Leu Ile Asp Val Pro
145                 150                 155                 160

Lys Asp Ile Gln Gln Gln Leu Val Ile Pro Asn Trp Asp Gln Pro Met
                165                 170                 175

Arg Leu Pro Gly Tyr Met Ser Arg Leu Pro Lys Leu Pro Asn Glu Met
            180                 185                 190

Leu Leu Glu Gln Ile Val Arg Leu Ile Ser Glu Ser Lys Lys Pro Val
        195                 200                 205

Leu Tyr Val Gly Gly Gly Cys Ser Gln Ser Ser Glu Glu Leu Arg Arg
    210                 215                 220

Phe Val Glu Leu Thr Gly Ile Pro Val Ala Ser Thr Leu Met Gly Leu
225                 230                 235                 240

Gly Ala Phe Pro Thr Gly Asp Glu Leu Ser Leu Gln Met Leu Gly Met
                245                 250                 255

His Gly Thr Val Tyr Ala Asn Tyr Ala Val Asp Ser Ser Asp Leu Leu
            260                 265                 270

Leu Ala Phe Gly Val Arg Phe Asp Asp Arg Val Thr Gly Lys Leu Glu
        275                 280                 285

Ala Phe Ala Ser Arg Ala Lys Ile Val His Ile Asp Ile Asp Ser Ala
    290                 295                 300

Glu Ile Gly Lys Asn Lys Leu Pro His Ala Ser Ile Cys Ala Asp Ile
305                 310                 315                 320

Lys Leu Ala Leu Gln Gly Leu Asn Ser Ile Leu Glu Gly Lys Glu Gly
                325                 330                 335

Lys Leu Lys Leu Asp Phe Ser Ala Trp Arg Gln Glu Leu Asn Glu Gln
            340                 345                 350

Lys Val Lys Tyr Pro Leu Asn Phe Lys Thr Phe Gly Glu Ala Ile Pro
        355                 360                 365

Pro Gln Tyr Ala Ile Gln Val Leu Asp Glu Leu Thr Asn Gly Asn Ala
    370                 375                 380

Ile Ile Ser Thr Gly Val Gly Gln His Gln Met Trp Ala Ala Gln Phe
385                 390                 395                 400

Tyr Lys Tyr Lys Lys Pro Arg Gln Trp Leu Thr Ser Gly Gly Leu Gly
                405                 410                 415

Ala Met Gly Phe Gly Leu Pro Ala Ala Met Gly Ala Ala Val Gly Arg
            420                 425                 430

Pro Gly Glu Ile Val Val Asp Ile Asp Gly Asp Gly Ser Phe Met Met
        435                 440                 445

Asn Val Gln Glu Leu Ala Thr Ile Lys Val Glu Asn Leu Pro Val Lys
    450                 455                 460

Ile Met Leu Leu Asn Asn Gln His Leu Gly Met Val Val Gln Trp Glu
465                 470                 475                 480

Asp Arg Phe Tyr Lys Ala Asn Arg Ala His Thr Tyr Leu Gly Asp Pro
                485                 490                 495

Ala Asn Glu Glu Glu Ile Phe Pro Asn Met Leu Lys Phe Ala Glu Ala
            500                 505                 510

Cys Gly Val Pro Ala Ala Arg Val Thr His Arg Asp Glu Leu Arg Ala
        515                 520                 525

Ala Ile Gln Lys Met Leu Asp Thr Pro Gly Pro Tyr Leu Leu Asp Val
    530                 535                 540

Ile Val Pro His Gln Glu His Val Leu Pro Met Ile Pro Ser Gly Gly
545                 550                 555                 560

Ala Phe Lys Asp Val Ile Thr
                565

<210>  15
<211>  641
<212>  PRT
<213>  Sorghum bicolor

<220>
<221>  MISC_FEATURE
<223>  Sorghum bicolor acetohydroxyacid synthase large subunit (SbAHASL)

<400>  15

Met Ala Thr Thr Ala Ala Ala Ala Ala Ala Ala Leu Ala Gly Ala Thr
1               5                   10                  15

Thr Ala Ala Pro Lys Ala Arg Arg Arg Ala His Leu Leu Ala Ala Arg
            20                  25                  30

Arg Ala Leu Ala Ala Pro Ile Arg Cys Ser Ala Ala Pro Pro Ala Thr
        35                  40                  45

Leu Thr Val Thr Ala Pro Pro Ala Thr Pro Leu Arg Pro Trp Gly Pro
    50                  55                  60

Thr Asp Pro Arg Lys Gly Ala Asp Ile Leu Val Glu Ala Leu Glu Arg
65                  70                  75                  80

Cys Gly Val Arg Asp Val Phe Ala Tyr Pro Gly Gly Ala Ser Met Glu
                85                  90                  95

Ile His Gln Ala Leu Thr Arg Ser Pro Val Ile Ala Asn His Leu Phe
            100                 105                 110

Arg His Glu Gln Gly Glu Ala Phe Ala Ala Ser Gly Phe Ala Arg Ser
        115                 120                 125

Ser Gly Arg Val Gly Val Cys Val Ala Thr Ser Gly Pro Gly Ala Thr
    130                 135                 140

Asn Leu Val Ser Ala Leu Ala Asp Ala Leu Leu Asp Ser Val Pro Met
145                 150                 155                 160

Val Ala Ile Thr Gly Gln Val Pro Arg Arg Met Ile Gly Thr Asp Ala
                165                 170                 175

Phe Gln Glu Thr Pro Ile Val Glu Val Thr Arg Ser Ile Thr Lys His

180                 185                 190

Asn Tyr Leu Val Leu Asp Val Asp Asp Ile Pro Arg Val Val Gln Glu
    195                 200                 205

Ala Phe Phe Leu Ala Ser Ser Gly Arg Pro Gly Pro Val Leu Val Asp
    210                 215                 220

Ile Pro Lys Asp Ile Gln Gln Gln Met Ala Val Pro Val Trp Asp Thr
225                 230                 235                 240

Pro Met Ser Leu Pro Gly Tyr Ile Ala Arg Leu Pro Lys Pro Pro Ala
                245                 250                 255

Thr Glu Leu Leu Glu Gln Val Leu Arg Leu Val Gly Glu Ser Arg Arg
            260                 265                 270

Pro Val Leu Tyr Val Gly Gly Gly Cys Ala Ala Ser Gly Glu Glu Leu
    275                 280                 285

Arg Arg Phe Val Glu Met Thr Gly Ile Pro Val Thr Thr Thr Leu Met
    290                 295                 300

Gly Leu Gly Asn Phe Pro Gly Asp Asp Pro Leu Ser Leu Arg Met Leu
305                 310                 315                 320

Gly Met His Gly Thr Val Tyr Ala Asn Tyr Ala Val Asp Lys Ala Asp
                325                 330                 335

Leu Leu Leu Ala Phe Gly Val Arg Phe Asp Asp Arg Val Thr Gly Lys
            340                 345                 350

Ile Glu Ala Phe Ala Ser Arg Ala Lys Ile Val His Ile Asp Ile Asp
    355                 360                 365

Pro Ala Glu Ile Gly Lys Asn Lys Gln Pro His Val Ser Ile Cys Ala
    370                 375                 380

Asp Val Lys Leu Ala Leu Gln Gly Met Asn Ala Leu Leu Glu Gly Ser
385                 390                 395                 400

Thr Ser Lys Lys Ser Phe Asp Phe Gly Ser Trp Gln Ala Glu Leu Asp
                405                 410                 415

Gln Gln Lys Arg Glu Phe Pro Leu Gly Tyr Lys Thr Phe Asp Asp Glu
            420                 425                 430

Ile Gln Pro Gln Tyr Ala Ile Gln Val Leu Asp Glu Leu Thr Lys Gly
    435                 440                 445

Glu Ala Ile Ile Ala Thr Gly Val Gly Gln His Gln Met Trp Ala Ala
    450                 455                 460

Gln Tyr Tyr Thr Tyr Lys Arg Pro Arg Gln Trp Leu Ser Ser Ala Gly
465                 470                 475                 480

Leu Gly Ala Met Gly Phe Gly Leu Pro Ala Ala Ala Gly Ala Ala Val
                485                 490                 495

Ala Asn Pro Gly Ile Thr Val Val Asp Ile Asp Gly Asp Gly Ser Phe
            500                 505                 510

Leu Met Asn Ile Gln Glu Leu Ala Met Ile Arg Ile Glu Asn Leu Pro
        515                 520                 525

Val Lys Val Phe Val Leu Asn Asn Gln His Leu Gly Met Val Val Gln
    530                 535                 540

Trp Glu Asp Arg Phe Tyr Lys Ala Asn Arg Ala His Thr Tyr Leu Gly
545                 550                 555                 560

Asn Pro Glu Asn Glu Ser Glu Ile Tyr Pro Asp Phe Val Thr Ile Ala
                565                 570                 575

Lys Gly Phe Asn Ile Pro Ala Val Arg Val Thr Lys Lys Ser Glu Val
            580                 585                 590

His Ala Ala Ile Lys Lys Met Leu Glu Thr Pro Gly Pro Tyr Leu Leu
        595                 600                 605

Asp Ile Ile Val Pro His Gln Glu His Val Leu Pro Met Ile Pro Ser
    610                 615                 620

Gly Gly Ala Phe Lys Asp Met Ile Leu Asp Gly Asp Gly Arg Thr Val
625                 630                 635                 640

Tyr

<210>  16
<211>  657
<212>  PRT
<213>  Solanum tuberosum

<220>
<221>  MISC_FEATURE
<223>  Potato acetohydroxyacid synthase large subunit number 1
       (StAHASL1)

<400>  16

Met Ala Ala Ala Ser Pro Ser Pro Cys Phe Ser Lys Asn Leu Pro Pro
1               5                   10                  15

Ser Ser Ser Lys Ser Ser Ile Leu Leu Pro Lys Ser Thr Phe Thr Phe
            20                  25                  30

His Asn His Pro Lys Lys Thr Ser Pro Leu His Leu Thr His Thr Gln
        35                  40                  45

His His Ser Arg Phe Thr Val Ser Asn Val Ile Leu Ser Thr Thr Thr
    50                  55                  60

His Asn Asp Val Ser Glu Pro Glu Ile Phe Val Ser Arg Phe Ala Pro
65                  70                  75                  80

Asp Glu Pro Arg Lys Gly Cys Asp Val Leu Val Glu Ala Leu Glu Arg
                85                  90                  95

Glu Gly Val Lys Asp Val Phe Ala Tyr Pro Gly Gly Ala Ser Met Glu
            100                 105                 110

Ile His Gln Ala Leu Thr Arg Ser Asn Ile Ile Arg Asn Val Leu Pro
        115                 120                 125

Arg His Glu Gln Gly Gly Val Phe Ala Ala Glu Gly Tyr Ala Arg Ala
    130                 135                 140

Thr Gly Phe Pro Gly Val Cys Ile Ala Thr Ser Gly Pro Gly Ala Thr
145                 150                 155                 160

Asn Leu Val Ser Gly Leu Ala Asp Ala Leu Leu Asp Ser Ile Pro Ile
                165                 170                 175

Val Ala Ile Thr Gly Gln Val Pro Arg Arg Met Ile Gly Thr Asp Ala
            180                 185                 190

Phe Gln Glu Thr Pro Ile Val Glu Val Thr Arg Ser Ile Thr Lys His
        195                 200                 205

Asn Tyr Leu Val Met Asp Val Glu Asp Ile Pro Arg Val Val Arg Glu
    210                 215                 220

Ala Phe Phe Leu Ala Lys Ser Gly Arg Pro Gly Pro Val Leu Ile Asp
225                 230                 235                 240

Val Pro Lys Asp Ile Gln Gln Pro Leu Val Ile Pro Asn Trp Asp Gln
                245                 250                 255

Pro Met Arg Leu Pro Gly Tyr Met Ser Arg Leu Pro Lys Leu Pro Asn
        260                 265                 270

Glu Met Leu Leu Glu Gln Ile Val Arg Leu Ile Ser Glu Ser Lys Lys
    275                 280                 285

Pro Val Leu Tyr Val Gly Gly Gly Cys Thr Gln Ser Ser Glu Glu Leu
    290                 295                 300

Arg Arg Phe Val Glu Leu Thr Gly Ile Pro Val Ala Ser Thr Leu Met
305                 310                 315                 320

Gly Leu Gly Ala Phe Pro Val Gly Asp Glu Leu Ser Leu Gln Met Leu
                325                 330                 335

Gly Met His Gly Thr Val Tyr Ala Asn Tyr Ala Val Asp Ser Ser Asp
            340                 345                 350

Leu Leu Leu Ala Phe Gly Val Arg Phe Asp Asp Arg Val Thr Gly Lys
        355                 360                 365

Leu Glu Ala Phe Ala Ser Arg Ala Lys Ile Val His Ile Asp Ile Asp
    370                 375                 380

Ser Ala Glu Ile Gly Lys Asn Lys Gln Pro His Val Ser Ile Cys Ala
385                 390                 395                 400

Asp Ile Lys Leu Ala Leu Gln Gly Leu Asn Ser Ile Leu Glu Gly Lys
                405                 410                 415

Glu Gly Lys Leu Lys Leu Asp Phe Ser Ala Trp Arg Gln Glu Leu Thr
            420                 425                 430

Glu Gln Lys Val Lys Tyr Pro Leu Asn Tyr Lys Thr Phe Gly Glu Ala
        435                 440                 445

Ile Pro Pro Gln Tyr Ala Ile Gln Val Leu Asp Glu Leu Thr Asn Gly
    450                 455                 460

Asn Ala Ile Ile Ser Thr Gly Val Gly Gln His Gln Met Trp Ala Ala
465                 470                 475                 480

Gln Tyr Tyr Lys Tyr Lys Lys Pro Arg Gln Trp Leu Thr Ser Gly Gly
                485                 490                 495

Leu Gly Ala Met Gly Phe Gly Leu Pro Ala Ala Ile Gly Ala Ala Val
            500                 505                 510

Gly Arg Pro Gly Glu Ile Val Val Asp Ile Asp Gly Asp Gly Ser Phe
        515                 520                 525

Ile Met Asn Val Gln Glu Leu Ala Thr Ile Lys Val Glu Asn Leu Pro
    530                 535                 540

Val Lys Ile Met Leu Leu Asn Asn Gln His Leu Gly Met Val Val Gln
545                 550                 555                 560

Trp Glu Asp Arg Phe Tyr Lys Ala Asn Arg Ala His Thr Tyr Leu Gly
                565                 570                 575

Asp Pro Ala Asn Glu Glu Glu Ile Phe Pro Asn Met Leu Lys Phe Ala
            580                 585                 590

Glu Ala Cys Gly Val Pro Ala Ala Arg Val Ser His Arg Asp Asp Leu
        595                 600                 605

Arg Ala Ala Ile Gln Lys Met Leu Asp Thr Pro Gly Pro Tyr Leu Leu
    610                 615                 620

Asp Val Ile Val Pro His Gln Glu His Val Leu Pro Met Ile Pro Ser
625                 630                 635                 640

Gly Gly Ala Phe Lys Asp Val Ile Thr Glu Gly Asp Gly Arg Arg Ser
                645                 650                 655

Tyr

<210>  17
<211>  659
<212>  PRT
<213>  Solanum tuberosum

<220>
<221>  MISC_FEATURE
<223>  Potato acetohydroxyacid synthase large subunit number 2
       (StAHASL2)

<220>
<221>  misc_feature
<222>  (406)..(406)
<223>  Xaa can be any naturally occurring amino acid

<400>  17

Met Ala Ala Ala Ala Ser Pro Ser Pro Cys Phe Ser Lys Thr Leu Pro
1               5                   10                  15

Pro Ser Ser Ser Lys Ser Ser Thr Ile Leu Pro Arg Ser Thr Phe Pro
            20                  25                  30

Phe His Asn His Pro Gln Lys Ala Ser Pro Leu His Leu Thr His Thr
        35                  40                  45

His His His Arg Arg Gly Phe Ala Val Ser Asn Val Val Ile Ser Thr
    50                  55                  60

Thr Thr His Asn Asp Val Ser Glu Pro Glu Thr Phe Val Ser Arg Phe
65                  70                  75                  80

Ala Pro Asp Glu Pro Arg Lys Gly Cys Asp Val Leu Val Glu Ala Leu
                85                  90                  95

Glu Arg Glu Gly Val Thr Asp Val Phe Ala Tyr Pro Gly Gly Ala Ser
            100                 105                 110

Met Glu Ile His Gln Ala Leu Thr Arg Ser Asn Ile Ile Arg Asn Val
        115                 120                 125

Leu Pro Arg His Glu Gln Gly Gly Val Phe Ala Ala Glu Gly Tyr Ala
    130                 135                 140

Arg Ala Thr Gly Phe Pro Gly Val Cys Ile Ala Thr Ser Gly Pro Gly
145                 150                 155                 160

Ala Thr Asn Leu Val Ser Gly Leu Ala Asp Ala Leu Leu Asp Ser Ile
                165                 170                 175

Pro Ile Val Ala Ile Thr Gly Gln Val Pro Arg Arg Met Ile Gly Thr
            180                 185                 190

Asp Ala Phe Gln Glu Thr Pro Ile Val Glu Val Thr Arg Ser Ile Thr
        195                 200                 205

Lys His Asn Tyr Leu Val Met Asp Val Glu Asp Ile Pro Arg Val Val
    210                 215                 220

Arg Glu Ala Phe Phe Leu Ala Lys Ser Gly Arg Pro Gly Pro Val Leu
225                 230                 235                 240

Ile Asp Val Pro Lys Asp Ile Gln Gln Gln Leu Val Ile Pro Asn Trp
                245                 250                 255

Asp Gln Pro Met Arg Leu Pro Gly Tyr Met Ser Arg Leu Pro Lys Leu
            260                 265                 270

Pro Asn Glu Met Leu Leu Glu Gln Ile Ile Arg Leu Ile Ser Glu Ser
        275                 280                 285

Lys Lys Pro Val Leu Tyr Val Gly Gly Gly Cys Leu Gln Ser Ser Glu
    290                 295                 300

Glu Leu Arg Arg Phe Val Glu Leu Thr Gly Ile Ser Val Ala Ser Thr
305                 310                 315                 320

Leu Met Gly Leu Gly Ala Phe Pro Thr Gly Asp Glu Leu Ser Leu Gln
                325                 330                 335

Met Leu Gly Met His Gly Thr Val Tyr Ala Asn Tyr Ala Val Asp Gly
            340                 345                 350

Ser Asp Leu Leu Leu Ala Phe Gly Val Arg Phe Asp Asp Arg Val Thr
        355                 360                 365

Gly Lys Leu Glu Ala Phe Ala Ser Arg Ala Lys Ile Val His Ile Asp
    370                 375                 380

Ile Asp Ser Ala Glu Ile Gly Lys Asn Lys Gln Pro His Val Ser Ile
385                 390                 395                 400

Cys Ala Asp Ile Lys Xaa Ala Leu Gln Gly Leu Asn Ser Ile Leu Glu
                405                 410                 415

Gly Lys Glu Gly Lys Leu Lys Leu Asp Phe Ser Ala Trp Arg Gln Glu
            420                 425                 430

Leu Thr Glu Gln Lys Val Lys Tyr Pro Leu Ser Phe Lys Thr Phe Gly
        435                 440                 445

Glu Ala Ile Pro Pro Gln Tyr Ala Ile Gln Val Leu Asp Glu Leu Thr
    450                 455                 460

Asn Gly Asn Ala Ile Ile Ser Thr Gly Val Gly Gln His Gln Met Trp
465                 470                 475                 480

Ala Ala Gln Tyr Tyr Lys Tyr Lys Lys Pro Arg Gln Trp Leu Thr Ser
                485                 490                 495

Gly Gly Leu Gly Ala Met Gly Phe Gly Leu Pro Ala Ala Ile Gly Ala
            500                 505                 510

Ala Val Gly Arg Pro Gly Glu Ile Val Val Asp Ile Asp Gly Asp Gly
        515                 520                 525

Ser Phe Ile Met Asn Val Gln Glu Leu Ala Thr Ile Lys Val Glu Asn
    530                 535                 540

Leu Pro Val Lys Ile Met Leu Leu Asn Asn Gln His Leu Gly Met Val

545                 550                 555                 560

Val Gln Trp Glu Asp Arg Phe Tyr Lys Ala Asn Arg Ala His Thr Tyr
                565                 570                 575

Leu Gly Asp Pro Ala Asn Glu Glu Glu Ile Phe Pro Asn Met Leu Lys
            580                 585                 590

Phe Ala Glu Ala Cys Gly Val Pro Ala Ala Arg Val Ser His Arg Asp
        595                 600                 605

Asp Leu Arg Ala Ala Ile Gln Lys Met Leu Asp Thr Pro Gly Pro Tyr
    610                 615                 620

Leu Leu Asp Val Ile Val Pro His Gln Glu His Val Leu Pro Met Ile
625                 630                 635                 640

Pro Ser Gly Gly Ala Phe Lys Asp Val Ile Thr Glu Gly Asp Gly Arg
                645                 650                 655

Arg Ser Tyr

<210>  18
<211>  651
<212>  PRT
<213>  Glycine max

<220>
<221>  MISC_FEATURE
<223>  Soybean acetohydroxyacid synthase large subunit (GmAHASL)

<400>  18

Met Ala Ala Thr Ala Ser Arg Thr Thr Arg Phe Ser Ser Ser Ser Ser
1               5                   10                  15

His Pro Thr Phe Pro Lys Arg Ile Thr Arg Ser Thr Leu Pro Leu Ser
            20                  25                  30

His Gln Thr Leu Thr Lys Pro Asn His Ala Leu Lys Ile Lys Cys Ser
        35                  40                  45

Ile Ser Lys Pro Pro Thr Ala Ala Pro Phe Thr Lys Glu Ala Pro Thr
    50                  55                  60

Thr Glu Pro Phe Val Ser Arg Phe Ala Ser Gly Glu Pro Arg Lys Gly
65                  70                  75                  80

Ala Asp Ile Leu Val Glu Ala Leu Glu Arg Gln Gly Val Thr Thr Val
                85                  90                  95

Phe Ala Tyr Pro Gly Gly Ala Ser Met Glu Ile His Gln Ala Leu Thr
        100                 105                 110

Arg Ser Ala Ala Ile Arg Asn Val Leu Pro Arg His Glu Gln Gly Gly
    115                 120                 125

Val Phe Ala Ala Glu Gly Tyr Ala Arg Ser Ser Gly Leu Pro Gly Val
    130                 135                 140

Cys Ile Ala Thr Ser Gly Pro Gly Ala Thr Asn Leu Val Ser Gly Leu
145                 150                 155                 160

Ala Asp Ala Leu Met Asp Ser Val Pro Val Val Ala Ile Thr Gly Gln
                165                 170                 175

Val Pro Arg Arg Met Ile Gly Thr Asp Ala Phe Gln Glu Thr Pro Ile
        180                 185                 190

Val Glu Val Ser Arg Ser Ile Thr Lys His Asn Tyr Leu Ile Leu Asp
    195                 200                 205

Val Asp Asp Ile Pro Arg Val Val Ala Glu Ala Phe Phe Val Ala Thr
    210                 215                 220

Ser Gly Arg Pro Gly Pro Val Leu Ile Asp Ile Pro Lys Asp Val Gln
225                 230                 235                 240

Gln Gln Leu Ala Val Pro Asn Trp Asp Glu Pro Val Asn Leu Pro Gly
                245                 250                 255

Tyr Leu Ala Arg Leu Pro Arg Pro Pro Ala Glu Ala Gln Leu Glu His
        260                 265                 270

Ile Val Arg Leu Ile Met Glu Ala Gln Lys Pro Val Leu Tyr Val Gly
    275                 280                 285

Gly Gly Ser Leu Asn Ser Ser Ala Glu Leu Arg Arg Phe Val Glu Leu
    290                 295                 300

Thr Gly Ile Pro Val Ala Ser Thr Leu Met Gly Leu Gly Thr Phe Pro
305                 310                 315                 320

Ile Gly Asp Glu Tyr Ser Leu Gln Met Leu Gly Met His Gly Thr Val
                325                 330                 335

Tyr Ala Asn Tyr Ala Val Asp Asn Ser Asp Leu Leu Leu Ala Phe Gly
        340                 345                 350

Val Arg Phe Asp Asp Arg Val Thr Gly Lys Leu Glu Ala Phe Ala Ser
        355                 360                 365

Arg Ala Lys Ile Val His Ile Asp Ile Asp Ser Ala Glu Ile Gly Lys
    370                 375                 380

Asn Lys Gln Ala His Val Ser Val Cys Ala Asp Leu Lys Leu Ala Leu
385                 390                 395                 400

Lys Gly Ile Asn Met Ile Leu Glu Glu Lys Gly Val Glu Gly Lys Phe
                405                 410                 415

Asp Leu Gly Gly Trp Arg Glu Glu Ile Asn Val Gln Lys His Lys Phe
            420                 425                 430

Pro Leu Gly Tyr Lys Thr Phe Gln Asp Ala Ile Ser Pro Gln His Ala
        435                 440                 445

Ile Glu Val Leu Asp Glu Leu Thr Asn Gly Asp Ala Ile Val Ser Thr
    450                 455                 460

Gly Val Gly Gln His Gln Met Trp Ala Ala Gln Phe Tyr Lys Tyr Lys
465                 470                 475                 480

Arg Pro Arg Gln Trp Leu Thr Ser Gly Gly Leu Gly Ala Met Gly Phe
                485                 490                 495

Gly Leu Pro Ala Ala Ile Gly Ala Ala Val Ala Asn Pro Gly Ala Val
            500                 505                 510

Val Val Asp Ile Asp Gly Asp Gly Ser Phe Ile Met Asn Val Gln Glu
        515                 520                 525

Leu Ala Thr Ile Arg Val Glu Asn Leu Pro Val Lys Ile Leu Leu Leu
    530                 535                 540

Asn Asn Gln His Leu Gly Met Val Val Gln Trp Glu Asp Arg Phe Tyr
545                 550                 555                 560

Lys Ser Asn Arg Ala His Thr Tyr Leu Gly Asp Pro Ser Ser Glu Ser
                565                 570                 575

Glu Ile Phe Pro Asn Met Leu Lys Phe Ala Asp Ala Cys Gly Ile Pro
            580                 585                 590

Ala Ala Arg Val Thr Lys Lys Glu Glu Leu Arg Ala Ala Ile Gln Arg
        595                 600                 605

Met Leu Asp Thr Pro Gly Pro Tyr Leu Leu Asp Val Ile Val Pro His
    610                 615                 620

Gln Glu His Val Leu Pro Met Ile Pro Ser Asn Gly Ser Phe Lys Asp
625                 630                 635                 640

Val Ile Thr Glu Gly Asp Gly Arg Thr Arg Tyr
                645                 650

<210>  19
<211>  648
<212>  PRT
<213>  Xanthium sp.

<220>
<221>  MISC_FEATURE
<223>  Cocklebur acetohydroxyacid synthase large subunit (XsAHASL)

<400>  19

Met Ala Ala Ile Pro His Thr Asn Pro Ser Ile Thr Thr Lys Pro Pro
1               5                   10                  15

Ser Ser Pro Pro Arg Pro Thr Phe Leu Ala Arg Phe Thr Phe Pro Ile
            20                  25                  30

Thr Ser Thr Ser His Lys Arg His Arg Leu His Ile Ser Asn Val Leu
        35                  40                  45

Ser Asp Ser Lys Pro Thr Ile Thr His Ser Pro Leu Pro Thr Glu Ser
    50                  55                  60

Phe Ile Ser Arg Tyr Ala Pro Asp Gln Pro Arg Lys Gly Ala Asp Val
65                  70                  75                  80

Leu Val Glu Ala Leu Glu Arg Glu Gly Val Thr Asp Val Phe Ala Tyr
                85                  90                  95

Pro Gly Gly Ala Ser Met Glu Ile His Gln Ala Leu Thr Arg Ser Thr
            100                 105                 110

Thr Ile Arg Asn Val Leu Pro Arg His Glu Gln Gly Gly Val Phe Ala
        115                 120                 125

Ala Glu Gly Tyr Ala Arg Ala Ser Gly Leu Pro Gly Val Cys Ile Ala
    130                 135                 140

Thr Ser Gly Pro Gly Ala Thr Asn Leu Val Ser Gly Leu Ala Asp Ala
145                 150                 155                 160

Leu Leu Asp Ser Val Pro Met Val Ala Ile Thr Gly Gln Val Pro Arg

165                 170                 175

Arg Met Ile Gly Thr Asp Ala Phe Gln Glu Thr Pro Ile Val Glu Val
            180                 185                 190

Thr Arg Ser Ile Thr Lys His Asn Tyr Leu Val Leu Asp Val Glu Asp
        195                 200                 205

Ile Pro Arg Ile Val Arg Glu Ala Phe Tyr Leu Ala Ser Ser Gly Arg
    210                 215                 220

Pro Gly Pro Val Leu Ile Asp Val Pro Lys Asp Ile Gln Gln Gln Leu
225                 230                 235                 240

Val Val Pro Lys Trp Asp Glu Pro Ile Arg Leu Pro Gly Tyr Leu Ser
                245                 250                 255

Arg Leu Pro Lys Thr Glu Asn Asn Gly Gln Leu Glu His Ile Val Arg
            260                 265                 270

Leu Val Ser Glu Ala Lys Arg Pro Val Leu Tyr Val Gly Gly Gly Cys
        275                 280                 285

Leu Asn Ser Gly Asp Glu Leu Arg Arg Phe Val Glu Leu Thr Gly Ile
    290                 295                 300

Pro Val Ala Ser Thr Leu Met Gly Leu Gly Ala Tyr Pro Ala Ser Ser
305                 310                 315                 320

Asp Leu Ser Leu His Met Leu Gly Met His Gly Thr Val Tyr Ala Asn
                325                 330                 335

Tyr Ala Val Asp Lys Ser Asp Leu Leu Leu Ala Phe Gly Val Arg Phe
            340                 345                 350

Asp Asp Arg Val Thr Gly Lys Leu Glu Ala Phe Ala Ser Arg Ala Lys
        355                 360                 365

Ile Val His Ile Asp Ile Asp Ser Ala Glu Ile Gly Lys Asn Lys Gln
    370                 375                 380

Pro His Val Ser Ile Cys Gly Asp Ile Lys Val Ala Leu Gln Gly Leu
385                 390                 395                 400

Asn Lys Ile Leu Glu Val Lys Asn Ser Val Thr Asn Leu Asp Phe Ser
                405                 410                 415

Asn Trp Arg Lys Glu Leu Asp Glu Gln Lys Val Lys Tyr Pro Leu Ser
            420                 425                 430

Phe Lys Thr Phe Gly Glu Ala Ile Pro Pro Gln Tyr Ala Ile Gln Val
        435                 440                 445

Leu Asp Glu Leu Thr Gly Gly Asn Ala Ile Ile Ser Thr Gly Val Gly
    450                 455                 460

Gln His Gln Met Trp Ala Ala Gln Phe Tyr Lys Tyr Asn Lys Pro Arg
465                 470                 475                 480

Gln Trp Leu Thr Ser Gly Gly Leu Gly Ala Met Gly Phe Gly Leu Pro
                485                 490                 495

Ala Ala Ile Gly Ala Ala Val Ala Arg Pro Asp Ala Val Val Val Asp
            500                 505                 510

Ile Asp Gly Asp Gly Ser Phe Ile Met Ser Val Gln Glu Leu Ala Thr
        515                 520                 525

Ile Arg Val Glu Asn Leu Pro Val Lys Ile Leu Leu Leu Asn Asn Gln
    530                 535                 540

His Leu Gly Met Val Val Gln Trp Glu Asp Arg Phe Tyr Lys Ala Asn
545                 550                 555                 560

Arg Ala His Thr Tyr Leu Gly Asn Pro Ser Lys Glu Ser Glu Ile Phe
                565                 570                 575

Pro Asn Met Leu Lys Phe Ala Glu Ala Cys Asp Ile Pro Ala Ala Arg
            580                 585                 590

Val Thr Arg Lys Ala Asp Leu Arg Ala Ala Ile Gln Lys Met Leu Asp
        595                 600                 605

Thr Pro Gly Pro Tyr Leu Leu Asp Val Ile Val Pro His Gln Glu His
    610                 615                 620

Val Leu Pro Met Ile Pro Ala Gly Gly Gly Phe Met Asp Val Ile Thr
625                 630                 635                 640

Glu Gly Asp Gly Arg Met Lys Tyr
                645

<210>  20
<211>  640
<212>  PRT
<213>  Lolium multiflorum

<220>

<221>  MISC_FEATURE
<223>  Ryegrass acetohydroxyacid synthase large subunit (LmAHASL)

<400>  20

Met Ala Thr Ala Thr Ser Thr Ala Val Ala Phe Ser Gly Ala Thr Ala
1               5                   10                  15

Thr Leu Pro Lys Pro Arg Thr Leu Pro Arg His Leu Leu Pro Ser Ser
            20                  25                  30

Arg Arg Ala Leu Ala Ala Pro Ile Arg Cys Ser Ala Val Ser Pro Ser
        35                  40                  45

Pro Ser Pro Ala Pro Pro Ala Thr Ala Leu Arg Pro Trp Gly Pro Ser
    50                  55                  60

Glu Pro Arg Lys Gly Ala Asp Ile Leu Val Glu Ala Leu Glu Arg Cys
65                  70                  75                  80

Gly Ile Ser Asp Val Phe Ala Tyr Pro Gly Gly Ala Ser Met Glu Ile
                85                  90                  95

His Gln Ala Leu Thr Ser Ser Pro Leu Ile Thr Asn His Leu Phe Arg
            100                 105                 110

His Glu Gln Gly Glu Ala Phe Ala Ala Ser Gly Tyr Ala Arg Ala Ser
        115                 120                 125

Gly Arg Val Gly Val Cys Val Ala Thr Ser Gly Pro Gly Ala Thr Asn
    130                 135                 140

Leu Val Ser Ala Leu Ala Asp Ala Leu Leu Asp Ser Ile Pro Met Val
145                 150                 155                 160

Ala Ile Thr Gly Gln Val Pro Arg Arg Met Ile Gly Thr Asp Ala Phe
                165                 170                 175

Gln Glu Thr Pro Ile Val Glu Val Thr Arg Ser Ile Thr Lys His Asn
            180                 185                 190

Tyr Leu Val Leu Asp Val Glu Asp Ile Pro Arg Val Ile Gln Glu Ala
        195                 200                 205

Phe Phe Leu Ala Ser Ser Gly Arg Pro Gly Pro Val Leu Val Asp Ile
    210                 215                 220

Pro Lys Asp Ile Gln Gln Gln Met Ala Val Pro Val Trp Asp Ala Pro
225                 230                 235                 240

Met Ser Leu Pro Gly Tyr Ile Ala Arg Leu Pro Lys Pro Pro Ala Thr
                245                 250                 255
Glu Leu Leu Glu Gln Val Leu Arg Leu Val Gly Glu Glu Arg Arg Pro
            260                 265                 270
Ile Leu Tyr Val Gly Gly Gly Cys Ser Ala Ser Gly Glu Asp Val Arg
        275                 280                 285
Arg Phe Val Glu Leu Thr Gly Ile Pro Val Thr Thr Thr Leu Met Gly
    290                 295                 300
Leu Gly Asn Phe Pro Ser Asp Asp Pro Leu Ser Leu Arg Met Leu Gly
305                 310                 315                 320
Met His Gly Thr Val Tyr Ala Asn Tyr Ala Val Asp Lys Ala Asp Leu
                325                 330                 335
Leu Leu Ala Phe Gly Val Arg Phe Asp Asp Arg Val Thr Gly Lys Ile
            340                 345                 350
Glu Ala Phe Ala Ser Arg Ser Lys Ile Val His Ile Asp Ile Asp Pro
        355                 360                 365
Ala Glu Ile Gly Lys Asn Lys Gln Pro His Val Ser Ile Cys Ala Asp
    370                 375                 380
Val Lys Leu Ala Leu Gln Gly Leu Asn Ala Val Leu Thr Gly Ser Lys
385                 390                 395                 400
Cys Asp Lys Ser Phe Asp Phe Ala Ser Trp His Asp Glu Leu Glu Gln
                405                 410                 415
Gln Lys Arg Glu Phe Pro Leu Gly Tyr Lys Thr Phe Gly Glu Ala Ile
            420                 425                 430
Pro Pro Gln Tyr Ala Ile Gln Val Leu Asp Glu Leu Thr Lys Gly Glu
        435                 440                 445
Ala Ile Ile Ala Thr Gly Val Gly Gln His Gln Met Trp Ala Ala Gln
    450                 455                 460
Tyr Tyr Thr Tyr Lys Arg Pro Arg Gln Val Leu Ser Ser Ala Gly Leu
465                 470                 475                 480
Gly Ala Met Gly Phe Gly Leu Pro Ala Ala Ala Gly Thr Ala Val Ala
                485                 490                 495
Asn Pro Gly Val Thr Val Val Asp Ile Asp Gly Asp Gly Ser Phe Leu

500                 505                 510

Met Asn Ile Gln Glu Leu Ala Leu Ile Arg Ile Glu Asn Leu Pro Val
        515                 520                 525

Lys Val Met Ile Leu Asn Asn Gln His Leu Gly Met Val Val Gln Trp
    530                 535                 540

Glu Asp Arg Phe Tyr Lys Ala Asn Arg Ala His Thr Tyr Leu Gly Asn
545                 550                 555                 560

Pro Glu Asn Glu Ser Glu Ile Tyr Pro Asp Phe Val Thr Ile Ala Lys
                565                 570                 575

Gly Phe Asn Val Pro Ala Val Arg Val Thr Lys Arg Ser Glu Val Arg
            580                 585                 590

Ala Ala Ile Lys Lys Met Leu Glu Thr Pro Gly Pro Tyr Leu Leu Asp
        595                 600                 605

Ile Ile Val Pro His Gln Glu His Val Leu Pro Met Ile Pro Ser Gly
    610                 615                 620

Gly Ala Phe Lys Asp Ile Ile Met Glu Gly Asp Gly Arg Ile Ser Tyr
625                 630                 635                 640

<210>  21
<211>  607
<212>  PRT
<213>  Triticum aestivum

<220>
<221>  MISC_FEATURE
<223>  Wheat A genome acetohydroxyacid synthase large subunit
       (TaAHASL1A), partial

<400>  21

Leu Pro Ala Arg Val Val Arg Cys Cys Ala Ala Ser Pro Ala Ala Thr
1               5                   10                  15

Ser Ala Ala Pro Pro Ala Thr Ala Leu Arg Pro Trp Gly Pro Ser Glu
            20                  25                  30

Pro Arg Lys Gly Ala Asp Ile Leu Val Glu Ala Leu Glu Arg Cys Gly
        35                  40                  45

Ile Val Asp Val Phe Ala Tyr Pro Gly Gly Ala Ser Met Glu Ile His
    50                  55                  60

Gln Ala Leu Thr Arg Ser Pro Val Ile Thr Asn His Leu Phe Arg His

65                  70                  75                  80

Glu Gln Gly Glu Ala Phe Ala Ala Ser Gly Tyr Ala Arg Ala Ser Gly
                85                  90                  95

Arg Val Gly Val Cys Val Ala Thr Ser Gly Pro Gly Ala Thr Asn Leu
            100                 105                 110

Val Ser Ala Leu Ala Asp Ala Leu Leu Asp Ser Ile Pro Met Val Ala
        115                 120                 125

Ile Thr Gly Gln Val Pro Arg Arg Met Ile Gly Thr Asp Ala Phe Gln
    130                 135                 140

Glu Thr Pro Ile Val Glu Val Thr Arg Ser Ile Thr Lys His Asn Tyr
145                 150                 155                 160

Leu Val Leu Asp Val Glu Asp Ile Pro Arg Val Ile Gln Glu Ala Phe
                165                 170                 175

Phe Leu Ala Ser Ser Gly Arg Pro Gly Pro Val Leu Val Asp Ile Pro
            180                 185                 190

Lys Asp Ile Gln Gln Gln Met Ala Val Pro Ile Trp Asp Thr Pro Met
        195                 200                 205

Ser Leu Pro Gly Tyr Ile Ala Arg Leu Pro Lys Pro Pro Ser Thr Glu
    210                 215                 220

Ser Leu Glu Gln Val Leu Arg Leu Val Gly Glu Ser Arg Arg Pro Ile
225                 230                 235                 240

Leu Tyr Val Gly Gly Gly Cys Ala Ala Ser Gly Glu Glu Leu Arg Arg
                245                 250                 255

Phe Val Glu Leu Thr Gly Ile Pro Val Thr Thr Thr Leu Met Gly Leu
            260                 265                 270

Gly Asn Phe Pro Ser Asp Asp Pro Leu Ser Leu Arg Met Leu Gly Met
        275                 280                 285

His Gly Thr Val Tyr Ala Asn Tyr Ala Val Asp Lys Ala Asp Leu Leu
    290                 295                 300

Leu Ala Phe Gly Val Arg Phe Asp Asp Arg Val Thr Gly Lys Ile Glu
305                 310                 315                 320

Ala Phe Ala Ser Arg Ser Lys Ile Val His Ile Asp Ile Asp Pro Ala
                325                 330                 335

Glu Ile Gly Lys Asn Lys Gln Pro His Val Ser Ile Cys Ala Asp Val
            340                 345                 350

Lys Leu Ala Leu Gln Gly Leu Asn Ala Leu Leu Asn Gly Ser Lys Ala
        355                 360                 365

Gln Gln Gly Leu Asp Phe Gly Pro Trp His Lys Glu Leu Asp Gln Gln
    370                 375                 380

Lys Arg Glu Phe Pro Leu Gly Phe Lys Thr Phe Gly Glu Ala Ile Pro
385                 390                 395                 400

Pro Gln Tyr Ala Ile Gln Val Leu Asp Glu Leu Thr Lys Gly Glu Ala
                405                 410                 415

Ile Ile Ala Thr Gly Val Gly Gln His Gln Met Trp Ala Ala Gln Tyr
            420                 425                 430

Tyr Thr Tyr Lys Arg Pro Arg Gln Trp Leu Ser Ser Ser Gly Leu Gly
        435                 440                 445

Ala Met Gly Phe Gly Leu Pro Ala Ala Ala Gly Ala Ala Val Ala Asn
    450                 455                 460

Pro Gly Val Thr Val Val Asp Ile Asp Gly Asp Gly Ser Phe Leu Met
465                 470                 475                 480

Asn Ile Gln Glu Leu Ala Leu Ile Arg Ile Glu Asn Leu Pro Val Lys
                485                 490                 495

Val Met Ile Leu Asn Asn Gln His Leu Gly Met Val Val Gln Trp Glu
            500                 505                 510

Asp Arg Phe Tyr Lys Ala Asn Arg Ala His Thr Tyr Leu Gly Asn Pro
        515                 520                 525

Glu Asn Glu Ser Glu Ile Tyr Pro Asp Phe Val Thr Ile Ala Lys Gly
    530                 535                 540

Phe Asn Val Pro Ala Val Arg Val Thr Lys Lys Ser Glu Val Thr Ala
545                 550                 555                 560

Ala Ile Lys Lys Met Leu Glu Thr Pro Gly Pro Tyr Leu Leu Asp Ile
                565                 570                 575

Ile Val Pro His Gln Glu His Val Leu Pro Met Ile Pro Ser Gly Gly
            580                 585                 590

Ala Phe Lys Asp Met Ile Met Glu Gly Asp Gly Arg Thr Ser Tyr
        595                 600                 605

<210>  22
<211>  607
<212>  PRT
<213>  Triticum aestivum

<220>
<221>  MISC_FEATURE
<223>  Wheat B genome acetohydroxyacid synthase large subunit
       (TaAHASL1B), partial

<400>  22

Leu Pro Ala Arg Ile Val Arg Cys Cys Ala Ala Ser Pro Ala Ala Thr
1               5                   10                  15

Ser Ala Ala Pro Pro Ala Thr Ala Leu Arg Pro Trp Gly Pro Ser Glu
            20                  25                  30

Pro Arg Lys Gly Ala Asp Ile Leu Val Glu Ala Leu Glu Arg Cys Gly
        35                  40                  45

Ile Val Asp Val Phe Ala Tyr Pro Gly Gly Ala Ser Met Glu Ile His
    50                  55                  60

Gln Ala Leu Thr Arg Ser Pro Val Ile Thr Asn His Leu Phe Arg His
65                  70                  75                  80

Glu Gln Gly Glu Ala Phe Ala Ala Ser Gly Tyr Ala Arg Ala Ser Gly
                85                  90                  95

Arg Val Gly Val Cys Val Ala Thr Ser Gly Pro Gly Ala Thr Asn Leu
            100                 105                 110

Val Ser Ala Leu Ala Asp Ala Leu Leu Asp Ser Ile Pro Met Val Ala
        115                 120                 125

Ile Thr Gly Gln Val Pro Arg Arg Met Ile Gly Thr Asp Ala Phe Gln
    130                 135                 140

Glu Thr Pro Ile Val Glu Val Thr Arg Ser Ile Thr Lys His Asn Tyr
145                 150                 155                 160

Leu Val Leu Asp Val Glu Asp Ile Pro Arg Val Ile Gln Glu Ala Phe
                165                 170                 175

Phe Leu Ala Ser Ser Gly Arg Pro Gly Pro Val Leu Val Asp Ile Pro
            180                 185                 190

Lys Asp Ile Gln Gln Gln Met Ala Val Pro Val Trp Asp Thr Pro Met
        195                 200                 205

Ser Leu Pro Gly Tyr Ile Ala Arg Leu Pro Lys Pro Pro Ser Thr Glu
    210                 215                 220

Ser Leu Glu Gln Val Leu Arg Leu Val Gly Glu Ser Arg Arg Pro Ile
225                 230                 235                 240

Leu Tyr Val Gly Gly Gly Cys Ala Ala Ser Gly Glu Glu Leu Arg Arg
                245                 250                 255

Phe Val Glu Leu Thr Gly Ile Pro Val Thr Thr Thr Leu Met Gly Leu
            260                 265                 270

Gly Asn Phe Pro Ser Asp Asp Pro Leu Ser Leu Arg Met Leu Gly Met
        275                 280                 285

His Gly Thr Val Tyr Ala Asn Tyr Ala Val Asp Lys Ala Asp Leu Leu
    290                 295                 300

Leu Ala Phe Gly Val Arg Phe Asp Asp Arg Val Thr Gly Lys Ile Glu
305                 310                 315                 320

Ala Phe Ala Ser Arg Ser Lys Ile Val His Ile Asp Ile Asp Pro Ala
                325                 330                 335

Glu Ile Gly Lys Asn Lys Gln Pro His Val Ser Ile Cys Ala Asp Val
            340                 345                 350

Lys Leu Ala Leu Gln Gly Leu Asn Ala Leu Leu Asn Gly Ser Lys Ala
        355                 360                 365

Gln Gln Gly Leu Asp Phe Gly Pro Trp His Lys Glu Leu Asp Gln Gln
    370                 375                 380

Lys Arg Glu Phe Pro Leu Gly Phe Lys Thr Phe Gly Glu Ala Ile Pro
385                 390                 395                 400

Pro Gln Tyr Ala Ile Gln Val Leu Asp Glu Leu Thr Lys Gly Glu Ala
                405                 410                 415

Ile Ile Ala Thr Gly Val Gly Gln His Gln Met Trp Ala Ala Gln Tyr
            420                 425                 430

Tyr Thr Tyr Lys Arg Pro Arg Gln Trp Leu Ser Ser Ser Gly Leu Gly
        435                 440                 445

Ala Met Gly Phe Gly Leu Pro Ala Ala Ala Gly Ala Ala Val Ala Asn
    450                 455                 460

Pro Gly Val Thr Val Val Asp Ile Asp Gly Asp Gly Ser Phe Leu Met
465                 470                 475                 480

Asn Ile Gln Glu Leu Ala Leu Ile Arg Ile Glu Asn Leu Pro Val Lys
                485                 490                 495

Val Met Ile Leu Asn Asn Gln His Leu Gly Met Val Val Gln Trp Glu
            500                 505                 510

Asp Arg Phe Tyr Lys Ala Asn Arg Ala His Thr Tyr Leu Gly Asn Pro
        515                 520                 525

Glu Asn Glu Ser Glu Ile Tyr Pro Asp Phe Val Thr Ile Ala Lys Gly
    530                 535                 540

Phe Asn Val Pro Ala Val Arg Val Thr Lys Lys Ser Glu Val Thr Ala
545                 550                 555                 560

Ala Ile Lys Lys Met Leu Glu Thr Pro Gly Pro Tyr Leu Leu Asp Ile
                565                 570                 575

Ile Val Pro His Gln Glu His Val Leu Pro Met Ile Pro Ser Gly Gly
            580                 585                 590

Ala Phe Lys Asp Met Ile Met Glu Gly Asp Gly Arg Thr Ser Tyr
        595                 600                 605

<210>  23
<211>  607
<212>  PRT
<213>  Triticum aestivum

<220>
<221>  MISC_FEATURE
<223>  Wheat D genome acetohydroxyacid synthase large subunit
       (TaAHASL1D), partial

<400>  23

Leu Pro Ala Arg Ile Val Arg Cys Cys Ala Ala Ser Pro Ala Ala Thr
1               5                   10                  15

Ser Ala Ala Pro Pro Ala Thr Ala Leu Arg Pro Trp Gly Pro Ser Glu
            20                  25                  30

Pro Arg Lys Gly Ala Asp Ile Leu Val Glu Ala Leu Glu Arg Cys Gly
        35                  40                  45

Ile Val Asp Val Phe Ala Tyr Pro Gly Gly Ala Ser Met Glu Ile His
    50                  55                  60

Gln Ala Leu Thr Arg Ser Pro Val Ile Thr Asn His Leu Phe Arg His
65                  70                  75                  80

Glu Gln Gly Glu Ala Phe Ala Ala Ser Gly Tyr Ala Arg Ala Ser Gly
                85                  90                  95

Arg Val Gly Val Cys Val Ala Thr Ser Gly Pro Gly Ala Thr Asn Leu
            100                 105                 110

Val Ser Ala Leu Ala Asp Ala Leu Leu Asp Ser Ile Pro Met Val Ala
        115                 120                 125

Ile Thr Gly Gln Val Pro Arg Arg Met Ile Gly Thr Asp Ala Phe Gln
    130                 135                 140

Glu Thr Pro Ile Val Glu Val Thr Arg Ser Ile Thr Lys His Asn Tyr
145                 150                 155                 160

Leu Val Leu Asp Val Glu Asp Ile Pro Arg Val Ile Gln Glu Ala Phe
                165                 170                 175

Phe Leu Ala Ser Ser Gly Arg Pro Gly Pro Val Leu Val Asp Ile Pro
            180                 185                 190

Lys Asp Ile Gln Gln Gln Met Ala Val Pro Val Trp Asp Thr Pro Met
        195                 200                 205

Ser Leu Pro Gly Tyr Ile Ala Arg Leu Pro Lys Pro Pro Ser Thr Glu
    210                 215                 220

Ser Leu Glu Gln Val Leu Arg Leu Val Gly Glu Ser Arg Arg Pro Ile
225                 230                 235                 240

Leu Tyr Val Gly Gly Gly Cys Ala Ala Ser Gly Glu Glu Leu Arg Arg
                245                 250                 255

Phe Val Glu Leu Thr Gly Ile Pro Val Thr Thr Thr Leu Met Gly Leu
            260                 265                 270

Gly Asn Phe Pro Ser Asp Asp Pro Leu Ser Leu Arg Met Leu Gly Met
        275                 280                 285

His Gly Thr Val Tyr Ala Asn Tyr Ala Val Asp Lys Ala Asp Leu Leu
    290                 295                 300

Leu Ala Phe Gly Val Arg Phe Asp Asp Arg Val Thr Gly Lys Ile Glu

305                 310                 315                 320

Ala Phe Ala Ser Arg Ser Lys Ile Val His Ile Asp Ile Asp Pro Ala
                325                 330                 335

Glu Ile Gly Lys Asn Lys Gln Pro His Val Ser Ile Cys Ala Asp Val
            340                 345                 350

Lys Leu Ala Leu Gln Gly Leu Asn Asp Leu Leu Asn Gly Ser Lys Ala
        355                 360                 365

Gln Gln Gly Leu Asp Phe Gly Pro Trp His Lys Glu Leu Asp Gln Gln
    370                 375                 380

Lys Arg Glu Phe Pro Leu Gly Phe Lys Thr Phe Gly Glu Ala Ile Pro
385                 390                 395                 400

Pro Gln Tyr Ala Ile Gln Val Leu Asp Glu Leu Thr Lys Gly Glu Ala
                405                 410                 415

Ile Ile Ala Thr Gly Val Gly Gln His Gln Met Trp Ala Ala Gln Tyr
            420                 425                 430

Tyr Thr Tyr Lys Arg Pro Arg Gln Trp Leu Ser Ser Ser Gly Leu Gly
        435                 440                 445

Ala Met Gly Phe Gly Leu Pro Ala Ala Ala Gly Ala Ala Val Ala Asn
    450                 455                 460

Pro Gly Val Thr Val Val Asp Ile Asp Gly Asp Gly Ser Phe Leu Met
465                 470                 475                 480

Asn Ile Gln Glu Leu Ala Leu Ile Arg Ile Glu Asn Leu Pro Val Lys
                485                 490                 495

Val Met Ile Leu Asn Asn Gln His Leu Gly Met Val Val Gln Trp Glu
            500                 505                 510

Asp Arg Phe Tyr Lys Ala Asn Arg Ala His Thr Tyr Leu Gly Asn Pro
        515                 520                 525

Glu Asn Glu Ser Glu Ile Tyr Pro Asp Phe Val Thr Ile Ala Lys Gly
    530                 535                 540

Phe Asn Val Pro Ala Val Arg Val Thr Lys Lys Ser Glu Val Thr Ala
545                 550                 555                 560

Ala Ile Lys Lys Met Leu Glu Thr Pro Gly Pro Tyr Leu Leu Asp Ile
                565                 570                 575

Ile Val Pro His Gln Glu His Val Leu Pro Met Ile Pro Ser Gly Gly
            580                 585                 590

Ala Phe Lys Asp Met Ile Met Glu Gly Asp Gly Arg Thr Ser Tyr
        595                 600                 605

<210>  24
<211>  659
<212>  PRT
<213>  Gossypium hirsutum

<220>
<221>  MISC_FEATURE
<223>  Cotton acetohydroxyacid synthase large subunit (GHAHASA5)

<400>  24

Met Ala Ala Ala Thr Ala Asn Ser Ala Leu Pro Lys Leu Ser Thr Leu
1               5                   10                  15

Thr Ser Ser Phe Lys Ser Ser Ile Pro Ile Ser Lys Ser Ser Leu Pro
            20                  25                  30

Phe Ser Thr Thr Pro Gln Lys Pro Thr Pro Tyr Arg Ser Phe Asp Val
        35                  40                  45

Ser Cys Ser Leu Ser His Ala Ser Ser Asn Pro Arg Ser Ala Ala Thr
    50                  55                  60

Ser Val Thr Pro Lys Asn Ala Pro Pro His Asp Phe Ile Ser Arg Tyr
65                  70                  75                  80

Ala Asp Asp Glu Pro Arg Lys Gly Ala Asp Ile Leu Val Glu Ala Leu
                85                  90                  95

Val Arg Glu Gly Val Lys Asp Val Phe Ala Tyr Pro Gly Gly Ala Ser
            100                 105                 110

Met Glu Ile His Gln Ala Leu Thr Arg Ser Lys Ile Ile Arg Asn Val
        115                 120                 125

Leu Pro Arg His Glu Gln Gly Gly Val Phe Ala Ala Glu Gly Tyr Ala
    130                 135                 140

Arg Ser Ser Gly Ile Pro Gly Val Cys Ile Ala Thr Ser Gly Pro Gly
145                 150                 155                 160

Ala Thr Asn Leu Val Ser Gly Leu Ala Asp Ala Met Leu Asp Ser Ile
                165                 170                 175

Pro Leu Val Ala Ile Thr Gly Gln Val Pro Arg Arg Met Ile Gly Thr
        180                 185                 190

Asp Ala Phe Gln Glu Thr Pro Ile Val Glu Val Thr Arg Ser Ile Thr
    195                 200                 205

Lys His Asn Tyr Leu Val Leu Asp Val Asp Asp Ile Pro Arg Ile Val
    210                 215                 220

Ser Glu Ala Phe Phe Leu Ala Ser Ser Gly Arg Pro Gly Pro Val Leu
225                 230                 235                 240

Ile Asp Val Pro Lys Asp Ile Gln Gln Gln Leu Ala Val Pro Lys Trp
                245                 250                 255

Asn His Ser Leu Arg Leu Pro Gly Tyr Leu Ser Arg Leu Pro Lys Ala
            260                 265                 270

Pro Gly Glu Ala His Leu Glu Gln Ile Val Arg Leu Val Ser Glu Ser
        275                 280                 285

Lys Lys Pro Val Leu Tyr Val Gly Gly Gly Cys Leu Asn Ser Ser Glu
    290                 295                 300

Glu Leu Lys Arg Phe Val Glu Leu Thr Gly Ile Pro Val Ala Ser Thr
305                 310                 315                 320

Leu Met Gly Leu Gly Ala Phe Pro Ile Ser Asp Asp Leu Ser Leu Gln
                325                 330                 335

Met Leu Gly Met His Gly Thr Val Tyr Ala Asn Tyr Ala Val Asp Lys
            340                 345                 350

Ser Asp Leu Leu Leu Ala Phe Gly Val Arg Phe Asp Asp Arg Val Thr
        355                 360                 365

Gly Lys Leu Glu Ala Phe Ala Ser Arg Ala Lys Ile Val His Ile Asp
    370                 375                 380

Ile Asp Ser Ala Glu Ile Gly Lys Asn Lys Gln Pro His Val Ser Val
385                 390                 395                 400

Cys Ser Asp Val Lys Leu Ala Leu Gln Gly Ile Asn Lys Ile Leu Glu
                405                 410                 415

Thr Lys Val Ala Lys Leu Asn Leu Asp Tyr Ser Glu Trp Arg Gln Glu
            420                 425                 430

Leu Asn Glu Gln Lys Leu Lys Phe Pro Leu Ser Tyr Lys Thr Phe Gly
        435                 440                 445

Glu Ala Ile Pro Pro Gln Tyr Ala Ile Gln Val Leu Asp Glu Leu Thr
    450                 455                 460

Gly Gly Asn Ala Ile Ile Ser Thr Gly Val Gly Gln His Gln Met Trp
465                 470                 475                 480

Ala Ala Gln Phe Tyr Lys Tyr Lys Lys Pro Arg Gln Trp Leu Thr Ser
                485                 490                 495

Gly Gly Leu Gly Ala Met Gly Phe Gly Leu Pro Ala Ala Ile Gly Ala
            500                 505                 510

Ala Val Ala Asn Pro Glu Ala Val Val Val Asp Ile Asp Gly Asp Gly
        515                 520                 525

Ser Phe Ile Met Asn Val Gln Glu Leu Ala Thr Ile Arg Val Glu Asn
    530                 535                 540

Leu Pro Val Lys Ile Leu Leu Leu Asn Asn Gln His Leu Gly Met Val
545                 550                 555                 560

Val Gln Trp Glu Asp Arg Phe Tyr Lys Ala Asn Arg Ala His Thr Tyr
                565                 570                 575

Leu Gly Asp Pro Ser Asn Glu Ser Glu Ile Phe Pro Asn Met Leu Lys
            580                 585                 590

Phe Ala Glu Ala Cys Gly Ile Pro Ala Ala Arg Val Thr Lys Lys Glu
        595                 600                 605

Asp Leu Lys Ala Ala Met Gln Lys Met Leu Asp Thr Pro Gly Pro Tyr
    610                 615                 620

Leu Leu Asp Val Ile Val Pro His Gln Glu His Val Leu Pro Met Ile
625                 630                 635                 640

Pro Ser Gly Gly Ala Phe Lys Asp Val Ile Thr Glu Gly Asp Gly Arg
                645                 650                 655

Thr Gln Tyr

<210>  25
<211>  659
<212>  PRT
<213>  Gossypium hirsutum

<220>
<221>  MISC_FEATURE
<223>  Cotton acetohydroxyacid synthase large subunit (GHAHASA19)

<400>  25

Met Ala Ala Ala Thr Ser Asn Ser Ala Leu Pro Lys Leu Ser Thr Leu
1               5                   10                  15

Thr Ser Ser Phe Lys Ser Ser Ile Pro Ile Ser Lys Ser Ser Leu Pro
            20                  25                  30

Phe Ser Thr Thr Pro Gln Lys Pro Thr Pro Tyr Arg Ser Phe Asp Val
        35                  40                  45

Ser Cys Ser Leu Ser His Ala Ser Ser Asn Pro Arg Asn Pro Ala Ala
    50                  55                  60

Ser Val Thr Gln Lys Thr Ala Pro Pro His Tyr Phe Ile Ser Arg Tyr
65                  70                  75                  80

Ala Asp Asp Glu Pro Arg Lys Gly Ala Asp Ile Leu Val Glu Ala Leu
                85                  90                  95

Glu Arg Glu Gly Val Lys Asp Val Phe Ala Tyr Pro Gly Gly Ala Ser
            100                 105                 110

Met Glu Ile His Gln Ala Leu Thr Arg Ser Lys Ile Ile Arg Asn Val
        115                 120                 125

Leu Pro Arg His Glu Gln Gly Gly Val Phe Ala Ala Glu Gly Tyr Ala
    130                 135                 140

Arg Ser Ser Gly Ile Ser Gly Val Cys Ile Ala Thr Ser Gly Pro Gly
145                 150                 155                 160

Arg Thr Asn Leu Val Ser Gly Leu Ala Asp Ala Met Leu Asp Ser Ile
                165                 170                 175

Pro Leu Val Ala Ile Thr Gly Gln Val Pro Arg Arg Met Ile Gly Thr
            180                 185                 190

Asp Ala Phe Gln Glu Thr Pro Ile Val Glu Val Thr Arg Ser Ile Thr
        195                 200                 205

Lys His Asn Tyr Leu Val Leu Asp Val Asp Asp Ile Pro Arg Ile Val
    210                 215                 220

Ser Glu Ala Phe Phe Leu Ala Ser Ser Gly Arg Pro Gly Pro Val Leu
225                 230                 235                 240

Ile Asp Val Pro Lys Asp Ile Gln Gln Gln Leu Ala Val Pro Lys Trp
                245                 250                 255

Asn His Ser Leu Arg Leu Pro Gly Tyr Leu Ser Arg Leu Pro Lys Ala
            260                 265                 270

Pro Ala Glu Ala His Leu Glu Gln Ile Val Arg Leu Val Ser Glu Ser
        275                 280                 285

Lys Lys Pro Val Leu Tyr Val Gly Gly Gly Cys Leu Asn Ser Ser Glu
    290                 295                 300

Glu Leu Lys Arg Phe Val Glu Leu Thr Gly Ile Pro Val Ala Ser Thr
305                 310                 315                 320

Leu Met Gly Leu Gly Ala Phe Pro Ile Ser Asp Glu Leu Ser Leu Gln
                325                 330                 335

Met Leu Gly Met His Gly Thr Val Tyr Ala Asn Tyr Ala Val Asp Lys
            340                 345                 350

Ser Asp Leu Leu Leu Ala Phe Gly Val Arg Phe Asp Asp Arg Val Thr
        355                 360                 365

Gly Lys Leu Glu Ala Phe Ala Ser Arg Ala Lys Ile Val His Ile Asp
    370                 375                 380

Ile Asp Ser Ala Glu Ile Gly Lys Asn Lys Gln Pro His Met Ser Val
385                 390                 395                 400

Cys Ser Asp Val Lys Leu Ala Leu Gln Gly Ile Asn Lys Ile Leu Glu
                405                 410                 415

Thr Thr Gly Ala Lys Leu Asn Leu Asp Tyr Ser Glu Trp Arg Gln Glu
            420                 425                 430

Leu Asn Glu Gln Lys Leu Lys Phe Pro Leu Ser Tyr Lys Thr Phe Gly
        435                 440                 445

Glu Ala Ile Pro Pro Gln Tyr Ala Ile Gln Val Leu Asp Glu Leu Thr
    450                 455                 460

Gly Gly Asn Ala Ile Ile Ser Thr Gly Val Gly Gln His Gln Met Trp
465                 470                 475                 480

Ala Ala Gln Phe Tyr Lys Tyr Lys Lys Pro Arg Gln Trp Leu Thr Ser
                485                 490                 495

Gly Gly Leu Gly Ala Met Gly Phe Gly Leu Pro Ala Ala Ile Gly Ala
                500                 505                 510

Ala Val Ala Asn Pro Glu Ala Val Val Val Asp Ile Asp Gly Asp Gly
        515                 520                 525

Ser Phe Ile Met Asn Val Gln Glu Leu Ala Thr Ile Arg Val Glu Asn
    530                 535                 540

Leu Pro Val Lys Ile Leu Leu Leu Asn Asn Gln His Leu Gly Met Val
545                 550                 555                 560

Val Gln Trp Glu Asp Arg Phe Tyr Lys Ala Asn Arg Ala His Thr Tyr
                565                 570                 575

Leu Gly Asp Pro Ser Asn Glu Ser Glu Ile Phe Pro Asn Met Leu Lys
                580                 585                 590

Phe Ala Glu Ala Cys Gly Ile Pro Ala Ala Arg Val Thr Lys Lys Glu
        595                 600                 605

Asp Leu Lys Ala Ala Ile Gln Lys Met Leu Asp Thr Pro Gly Pro Tyr
    610                 615                 620

Leu Leu Asp Val Ile Val Pro His Gln Glu His Val Leu Pro Met Ile
625                 630                 635                 640

Pro Ser Gly Gly Ala Phe Lys Asp Val Ile Thr Glu Gly Asp Gly Arg
                645                 650                 655

Thr Gln Tyr

<210>  26
<211>  562
<212>  PRT
<213>  Escherichia coli

<220>
<221>  MISC_FEATURE
<223>  E. coli acetohydroxyacid synthase large subunit I (IlvB)

<400>  26

Met Ala Ser Ser Gly Thr Thr Ser Thr Arg Lys Arg Phe Thr Gly Ala
1               5                   10                  15

Glu Phe Ile Val His Phe Leu Glu Gln Gln Gly Ile Lys Ile Val Thr
            20                  25                  30

Gly Ile Pro Gly Gly Ser Ile Leu Pro Val Tyr Asp Ala Leu Ser Gln
        35                  40                  45

Ser Thr Gln Ile Arg His Ile Leu Ala Arg His Glu Gln Gly Ala Gly
    50                  55                  60

Phe Ile Ala Gln Gly Met Ala Arg Thr Asp Gly Lys Pro Ala Val Cys
65                  70                  75                  80

Met Ala Cys Ser Gly Pro Gly Ala Thr Asn Leu Val Thr Ala Ile Ala
                85                  90                  95

Asp Ala Arg Leu Asp Ser Ile Pro Leu Ile Cys Ile Thr Gly Gln Val
            100                 105                 110

Pro Ala Ser Met Ile Gly Thr Asp Ala Phe Gln Glu Val Asp Thr Tyr
        115                 120                 125

Gly Ile Ser Ile Pro Ile Thr Lys His Asn Tyr Leu Val Arg His Ile
    130                 135                 140

Glu Glu Leu Pro Gln Val Met Ser Asp Ala Phe Arg Ile Ala Gln Ser
145                 150                 155                 160

Gly Arg Pro Gly Pro Val Trp Ile Asp Ile Pro Lys Asp Val Gln Thr
                165                 170                 175

Ala Val Phe Glu Ile Glu Thr Gln Pro Ala Met Ala Glu Lys Ala Ala
            180                 185                 190

Ala Pro Ala Phe Ser Glu Glu Ser Ile Arg Asp Ala Ala Ala Met Ile
        195                 200                 205

Asn Ala Ala Lys Arg Pro Val Leu Tyr Leu Gly Gly Gly Val Ile Asn
    210                 215                 220

Ala Pro Ala Arg Val Arg Glu Leu Ala Glu Lys Ala Gln Leu Pro Thr
225                 230                 235                 240

Thr Met Thr Leu Met Ala Leu Gly Met Leu Pro Lys Ala His Pro Leu
                245                 250                 255

Ser Leu Gly Met Leu Gly Met His Gly Val Arg Ser Thr Asn Tyr Ile
            260                 265                 270

Leu Gln Glu Ala Asp Leu Leu Ile Val Leu Gly Ala Arg Phe Asp Asp
        275                 280                 285

Arg Ala Ile Gly Lys Thr Glu Gln Phe Cys Pro Asn Ala Lys Ile Ile

290                 295                 300

His Val Asp Ile Asp Arg Ala Glu Leu Gly Lys Ile Lys Gln Pro His
305                 310                 315                 320

Val Ala Ile Gln Ala Asp Val Asp Asp Val Leu Ala Gln Leu Ile Pro
                325                 330                 335

Leu Val Glu Ala Gln Pro Arg Ala Glu Trp His Gln Leu Val Ala Asp
            340                 345                 350

Leu Gln Arg Glu Phe Pro Cys Pro Ile Pro Lys Ala Cys Asp Pro Leu
        355                 360                 365

Ser His Tyr Gly Leu Ile Asn Ala Val Ala Ala Cys Val Asp Asp Asn
    370                 375                 380

Ala Ile Ile Thr Thr Asp Val Gly Gln His Gln Met Trp Thr Ala Gln
385                 390                 395                 400

Ala Tyr Pro Leu Asn Arg Pro Arg Gln Trp Leu Thr Ser Gly Gly Leu
                405                 410                 415

Gly Thr Met Gly Phe Gly Leu Pro Ala Ala Ile Gly Ala Ala Leu Ala
            420                 425                 430

Asn Pro Asp Arg Lys Val Leu Cys Phe Ser Gly Asp Gly Ser Leu Met
        435                 440                 445

Met Asn Ile Gln Glu Met Ala Thr Ala Ser Glu Asn Gln Leu Asp Val
    450                 455                 460

Lys Ile Ile Leu Met Asn Asn Glu Ala Leu Gly Leu Val His Gln Gln
465                 470                 475                 480

Gln Ser Leu Phe Tyr Glu Gln Gly Val Phe Ala Ala Thr Tyr Pro Gly
                485                 490                 495

Lys Ile Asn Phe Met Gln Ile Ala Ala Gly Phe Gly Leu Glu Thr Cys
            500                 505                 510

Asp Leu Asn Asn Glu Ala Asp Pro Gln Ala Ser Leu Gln Glu Ile Ile
        515                 520                 525

Asn Arg Pro Gly Pro Ala Leu Ile His Val Arg Ile Asp Ala Glu Glu
    530                 535                 540

Lys Val Tyr Pro Met Val Pro Pro Gly Ala Ala Asn Thr Glu Met Val
545                 550                 555                 560

Gly Glu

<210> 27
<211> 548
<212> PRT
<213> Escherichia coli

<220>
<221> MISC_FEATURE
<223> E. coli acetohydroxyacid synthase large subunit II (IlvG)

<400> 27

Met Asn Gly Ala Gln Trp Val Val His Ala Leu Arg Ala Gln Gly Val
1               5                   10                  15

Asn Thr Val Phe Gly Tyr Pro Gly Gly Ala Ile Met Pro Val Tyr Asp
            20                  25                  30

Ala Leu Tyr Asp Gly Gly Val Glu His Leu Leu Cys Arg His Glu Gln
        35                  40                  45

Gly Ala Ala Met Ala Ala Ile Gly Tyr Ala Arg Ala Thr Gly Lys Thr
    50                  55                  60

Gly Val Cys Ile Ala Thr Ser Gly Pro Gly Ala Thr Asn Leu Ile Thr
65                  70                  75                  80

Gly Leu Ala Asp Ala Leu Leu Asp Ser Ile Pro Val Val Ala Ile Thr
                85                  90                  95

Gly Gln Val Ser Ala Pro Phe Ile Gly Thr Asp Ala Phe Gln Glu Val
            100                 105                 110

Asp Val Leu Gly Leu Ser Leu Ala Cys Thr Lys His Ser Phe Leu Val
        115                 120                 125

Gln Ser Leu Glu Glu Leu Pro Arg Ile Met Ala Glu Ala Phe Asp Val
    130                 135                 140

Ala Cys Ser Gly Arg Pro Gly Pro Val Leu Val Asp Ile Pro Lys Asp
145                 150                 155                 160

Ile Gln Leu Ala Ser Gly Asp Leu Glu Pro Trp Phe Thr Thr Val Glu
                165                 170                 175

Asn Glu Val Thr Phe Pro His Ala Glu Val Glu Gln Ala Arg Gln Met
            180                 185                 190

Leu Ala Lys Ala Gln Lys Pro Met Leu Tyr Val Gly Gly Gly Val Gly
        195                 200                 205

Met Ala Gln Ala Val Pro Ala Leu Arg Glu Phe Leu Ala Ala Thr Lys
    210                 215                 220

Met Pro Ala Thr Cys Thr Leu Lys Gly Leu Gly Ala Val Glu Ala Asp
225                 230                 235                 240

Tyr Pro Tyr Tyr Leu Gly Met Leu Gly Met His Gly Thr Lys Ala Ala
                245                 250                 255

Asn Phe Ala Val Gln Glu Cys Asp Leu Leu Ile Ala Val Gly Ala Arg
            260                 265                 270

Phe Asp Asp Arg Val Thr Gly Lys Leu Asn Thr Phe Ala Pro His Ala
        275                 280                 285

Ser Val Ile His Met Asp Ile Asp Pro Ala Glu Met Asn Lys Leu Arg
    290                 295                 300

Gln Ala His Val Ala Leu Gln Gly Asp Leu Asn Ala Leu Leu Pro Ala
305                 310                 315                 320

Leu Gln Gln Pro Leu Asn Gln Tyr Asp Trp Gln Gln His Cys Ala Gln
                325                 330                 335

Leu Arg Asp Glu His Ser Trp Arg Tyr Asp His Pro Gly Asp Ala Ile
            340                 345                 350

Tyr Ala Pro Leu Leu Leu Lys Gln Leu Ser Asp Arg Lys Pro Ala Asp
        355                 360                 365

Cys Val Val Thr Thr Asp Val Gly Gln His Gln Met Trp Ala Ala Gln
    370                 375                 380

His Ile Ala His Thr Arg Pro Glu Asn Phe Ile Thr Ser Ser Gly Leu
385                 390                 395                 400

Gly Thr Met Gly Phe Gly Leu Pro Ala Ala Val Gly Ala Gln Val Ala
                405                 410                 415

Arg Pro Asn Asp Thr Val Val Cys Ile Ser Gly Asp Gly Ser Phe Met
            420                 425                 430

Met Asn Val Gln Glu Leu Gly Thr Val Lys Arg Lys Gln Leu Pro Leu
        435                 440                 445

Lys Ile Val Leu Leu Asp Asn Gln Arg Leu Gly Met Val Arg Gln Trp
    450                 455                 460

Gln Gln Leu Phe Phe Gln Glu Arg Tyr Ser Glu Thr Thr Leu Thr Asp
465                 470                 475                 480

Asn Pro Asp Phe Leu Met Leu Ala Ser Ala Phe Gly Ile His Gly Gln
                485                 490                 495

His Ile Thr Arg Lys Asp Gln Val Glu Ala Ala Leu Asp Thr Met Leu
            500                 505                 510

Asn Ser Asp Gly Pro Tyr Leu Leu His Val Ser Ile Asp Glu Leu Glu
        515                 520                 525

Asn Val Trp Pro Leu Val Pro Pro Gly Ala Ser Asn Ser Glu Met Leu
    530                 535                 540

Glu Lys Leu Ser
545

<210>  28
<211>  574
<212>  PRT
<213>  Escherichia coli

<220>
<221>  MISC_FEATURE
<223>  E. coli acetohydroxyacid synthase large subunit III (IlvI)

<400>  28

Met Glu Met Leu Ser Gly Ala Glu Met Val Val Arg Ser Leu Ile Asp
1               5                   10                  15

Gln Gly Val Lys Gln Val Phe Gly Tyr Pro Gly Gly Ala Val Leu Asp
            20                  25                  30

Ile Tyr Asp Ala Leu His Thr Val Gly Gly Ile Asp His Val Leu Val
        35                  40                  45

Arg His Glu Gln Ala Ala Val His Met Ala Asp Gly Leu Ala Arg Ala
    50                  55                  60

Thr Gly Glu Val Gly Val Val Leu Val Thr Ser Gly Pro Gly Ala Thr
65                  70                  75                  80

Asn Ala Ile Thr Gly Ile Ala Thr Ala Tyr Met Asp Ser Ile Pro Leu
                85                  90                  95

Val Val Leu Ser Gly Gln Val Ala Thr Ser Leu Ile Gly Tyr Asp Ala

            100                 105                 110

Phe Gln Glu Cys Asp Met Val Gly Ile Ser Arg Pro Val Val Lys His
        115                 120                 125

Ser Phe Leu Val Lys Gln Thr Glu Asp Ile Pro Gln Val Leu Lys Lys
    130                 135                 140

Ala Phe Trp Leu Ala Ala Ser Gly Arg Pro Gly Pro Val Val Val Asp
145                 150                 155                 160

Leu Pro Lys Asp Ile Leu Asn Pro Ala Asn Lys Leu Pro Tyr Val Trp
                165                 170                 175

Pro Glu Ser Val Ser Met Arg Ser Tyr Asn Pro Thr Thr Thr Gly His
            180                 185                 190

Lys Gly Gln Ile Lys Arg Ala Leu Gln Thr Leu Val Ala Ala Lys Lys
        195                 200                 205

Pro Val Val Tyr Val Gly Gly Gly Ala Ile Thr Ala Gly Cys His Gln
    210                 215                 220

Gln Leu Lys Glu Thr Val Glu Ala Leu Asn Leu Pro Val Val Cys Ser
225                 230                 235                 240

Leu Met Gly Leu Gly Ala Phe Pro Ala Thr His Arg Gln Ala Leu Gly
                245                 250                 255

Met Leu Gly Met His Gly Thr Tyr Glu Ala Asn Met Thr Met His Asn
            260                 265                 270

Ala Asp Val Ile Phe Ala Val Gly Val Arg Phe Asp Asp Arg Thr Thr
        275                 280                 285

Asn Asn Leu Ala Lys Tyr Cys Pro Asn Ala Thr Val Leu His Ile Asp
    290                 295                 300

Ile Asp Pro Thr Ser Ile Ser Lys Thr Val Thr Ala Asp Ile Pro Ile
305                 310                 315                 320

Val Gly Asp Ala Arg Gln Val Leu Glu Gln Met Leu Glu Leu Leu Ser
                325                 330                 335

Gln Glu Ser Ala His Gln Pro Leu Asp Glu Ile Arg Asp Trp Trp Gln
            340                 345                 350

Gln Ile Glu Gln Trp Arg Ala Arg Gln Cys Leu Lys Tyr Asp Thr His
        355                 360                 365

Ser Glu Lys Ile Lys Pro Gln Ala Val Ile Glu Thr Leu Trp Arg Leu
    370                 375                 380

Thr Lys Gly Asp Ala Tyr Val Thr Ser Asp Val Gly Gln His Gln Met
385                 390                 395                 400

Phe Ala Ala Leu Tyr Tyr Pro Phe Asp Lys Pro Arg Arg Trp Ile Asn
                405                 410                 415

Ser Gly Gly Leu Gly Thr Met Gly Phe Gly Leu Pro Ala Ala Leu Gly
            420                 425                 430

Val Lys Met Ala Leu Pro Glu Glu Thr Val Val Cys Val Thr Gly Asp
        435                 440                 445

Gly Ser Ile Gln Met Asn Ile Gln Glu Leu Ser Thr Ala Leu Gln Tyr
    450                 455                 460

Glu Leu Pro Val Leu Val Val Asn Leu Asn Asn Arg Tyr Leu Gly Met
465                 470                 475                 480

Val Lys Gln Trp Gln Asp Met Ile Tyr Ser Gly Arg His Ser Gln Ser
                485                 490                 495

Tyr Met Gln Ser Leu Pro Asp Phe Val Arg Leu Ala Glu Ala Tyr Gly
            500                 505                 510

His Val Gly Ile Gln Ile Ser His Pro His Glu Leu Glu Ser Lys Leu
        515                 520                 525

Ser Glu Ala Leu Glu Gln Val Arg Asn Asn Arg Leu Val Phe Val Asp
    530                 535                 540

Val Thr Val Asp Gly Ser Glu His Val Tyr Pro Met Gln Ile Arg Gly
545                 550                 555                 560

Gly Gly Met Asp Glu Met Trp Leu Ser Lys Thr Glu Arg Thr
                565                 570

<210>  29
<211>  1917
<212>  DNA
<213>  Zea mays

<220>
<221>  misc_feature
<223>  Maize acetohydroxyacid synthase large subunit 2 (ZmAHASL2) coding
        sequence, S621N allele.

<400> 29
atggccaccg ccgccgccgc gtctaccgcg ctcactggcg ccactaccgc tgcgcccaag 60

gcgaggcgcc gggcgcacct cctggccacc cgccgcgccc tcgccgcgcc catcaggtgc 120

tcagcggcgt cacccgccat gccgatggct cccccggcca ccccgctccg gccgtggggc 180

cccaccgatc cccgcaaggg cgccgacatc ctcgtcgagt ccctcgagcg ctgcggcgtc 240

cgcgacgtct tcgcctaccc cggcggcgcg tccatggaga tccaccaggc actcacccgc 300

tcccccgtca tcgccaacca cctcttccgc cacgagcaag gggaggcctt tgcggcctcc 360

ggctacgcgc gctcctcggg ccgcgtcggc gtctgcatcg ccacctccgg ccccggcgcc 420

accaaccttg tctccgcgct cgccgacgcg ctgctcgatt ccgtccccat ggtcgccatc 480

acgggacagg tgccgcgacg catgattggc accgacgcct tccaggagac gcccatcgtc 540

gaggtcaccc gctccatcac caagcacaac tacctggtcc tcgacgtcga cgacatcccc 600

cgcgtcgtgc aggaggcttt cttcctcgcc tcctctggtc gaccggggcc ggtgcttgtc 660

gacatcccca aggacatcca gcagcagatg gcggtgcctg tctgggacaa gcccatgagt 720

ctgcctgggt acattgcgcg ccttcccaag ccccctgcga ctgagttgct tgagcaggtg 780

ctgcgtcttg ttggtgaatc ccggcgccct gttctttatg ttggcggtgg ctgcgcagca 840

tctggtgagg agttgcgacg ctttgtggag ctgactggaa tcccggtcac aactactctt 900

atgggcctcg gcaacttccc cagcgacgac ccactgtctc tgcgcatgct aggtatgcat 960

ggcacggtgt atgcaaatta tgcagtggat aaggccgatc tgttgcttgc acttggtgtg 1020

cggtttgatg atcgtgtgac agggaagatt gaggcttttg caagcagggc taagattgtg 1080

cacgttgata ttgatccggc tgagattggc aagaacaagc agccacatgt gtccatctgt 1140

gcagatgtta agcttgcttt gcagggcatg aatgctcttc ttgaaggaag cacatcaaag 1200

aagagctttg actttggctc atggaacgat gagttggatc agcagaagag ggaattcccc 1260

cttgggtata aaacatctaa tgaggagatc cagccacaat atgctattca ggttcttgat 1320

gagctgacga aaggcgaggc catcatcggc acaggtgttg ggcagcacca gatgtgggcg 1380

gcacagtact acacttacaa gcggccaagg cagtggttgt cttcagctgg tcttggggct 1440

atgggatttg gtttgccggc tgctgctggt gcttctgtgg ccaacccagg tgttactgtt 1500

gttgacatcg atggagatgg tagctttctc atgaacgttc aggagctagc tatgatccga 1560

attgagaacc tcccggtgaa ggtctttgtg ctaaacaacc agcacctggg gatggtggtg 1620

cagtgggagg acaggttcta taaggccaac agagcgcaca catacttggg aaacccagag 1680

aatgaaagtg agatatatcc agatttcgtg acgatcgcca aagggttcaa cattccagcg 1740

gtccgtgtga caaagaagaa cgaagtccgc gcagcgataa agaagatgct cgagactcca 1800

gggccgtacc tcttggatat aatcgtccca caccaggagc atgtgttgcc tatgatccct 1860

aatggtgggg ctttcaagga tatgatcctg gatggtgatg gcaggactgt gtactga 1917

<210> 30
<211> 2013
<212> DNA
<213> Arabidopsis thaliana

<220>
<221> misc_feature
<223> Arabidopsis acetohydroxyacid synthase large subunit (AtAHASL)
      coding sequence, S653N mutant allele.

<400> 30
atggcggcgg caacaacaac aacaacaaca tcttcttcga tctccttctc caccaaacca 60

tctccttcct cctccaaatc accattacca atctccagat tctccctccc attctcccta 120

aaccccaaca aatcatcctc ctcctcccgc cgccgcggta tcaaatccag ctctccctcc 180

tccatctccg ccgtgctcaa cacaaccacc aatgtcacaa ccactccctc tccaaccaaa 240

cctaccaaac ccgaaacatt catctcccga ttcgctccag atcaaccccg caaaggcgct 300

gatatcctcg tcgaagcttt agaacgtcaa ggcgtagaaa ccgtattcgc ttaccctgga 360

ggtgcatcaa tggagattca ccaagcctta acccgctctt cctcaatccg taacgtcctt 420

cctcgtcacg aacaaggagg tgtattcgca gcagaaggat acgctcgatc ctcaggtaaa 480

ccaggtatct gtatagccac ttcaggtccc ggagctacaa atctcgttag cggattagcc 540

gatgcgttgt tagatagtgt tcctcttgta gcaatcacag gacaagtccc tcgtcgtatg 600

attggtacag atgcgtttca agagactccg attgttgagg taacgcgttc gattacgaag 660

cataactatc ttgtgatgga tgttgaagat atccctagga ttattgagga agctttcttt 720

ttagctactt ctggtagacc tggacctgtt ttggttgatg ttcctaaaga tattcaacaa 780

cagcttgcga ttcctaattg ggaacaggct atgagattac ctggttatat gtctaggatg 840

cctaaacctc cggaagattc tcatttggag cagattgtta ggttgatttc tgagtctaag 900

aagcctgtgt tgtatgttgg tggtggttgt ttgaattcta gcgatgaatt gggtaggttt 960

gttgagctta cggggatccc tgttgcgagt acgttgatgg ggctgggatc ttatccttgt 1020

gatgatgagt tgtcgttaca tatgcttgga atgcatggga ctgtgtatgc aaattacgct 1080

gtggagcata gtgatttgtt gttggcgttt ggggtaaggt ttgatgatcg tgtcacgggt 1140

aagcttgagg cttttgctag tagggctaag attgttcata ttgatattga ctcggctgag 1200

attgggaaga ataagactcc tcatgtgtct gtgtgtggtg atgttaagct ggctttgcaa 1260

gggatgaata aggttcttga gaaccgagcg gaggagctta agcttgattt tggagtttgg 1320

aggaatgagt tgaacgtaca gaaacagaag tttccgttga gctttaagac gtttggggaa 1380

gctattcctc cacagtatgc gattaaggtc cttgatgagt tgactgatgg aaaagccata 1440

ataagtactg gtgtcgggca acatcaaatg tgggcggcgc agttctacaa ttacaagaaa 1500

ccaaggcagt ggctatcatc aggaggcctt ggagctatgg gatttggact tcctgctgcg 1560

attggagcgt ctgttgctaa ccctgatgcg atagttgtgg atattgacgg agatggaagc 1620

tttataatga atgtgcaaga gctagccact attcgtgtag agaatcttcc agtgaaggta 1680

cttttattaa acaaccagca tcttggcatg gttatgcaat gggaagatcg gttctacaaa 1740

gctaaccgag ctcacacatt tctcggggat ccggctcagg aggacgagat attcccgaac 1800

atgttgctgt ttgcagcagc ttgcgggatt ccagcggcga gggtgacaaa gaaagcagat 1860

ctccgagaag ctattcagac aatgctggat acaccaggac cttacctgtt ggatgtgatt 1920

tgtccgcacc aagaacatgt gttgccgatg atcccgaatg gtggcacttt caacgatgtc 1980

ataacggaag gagatggccg gattaaatac tga 2013

<210> 31
<211> 2013
<212> DNA
<213> Arabidopsis thaliana

<220>
<221> misc_feature
<223> Arabidopsis acetohydroxyacid synthase large subunit (AtAHASL)
      coding sequence, wild type allele.

<400> 31
atggcggcgg caacaacaac aacaacaaca tcttcttcga tctccttctc caccaaacca 60

tctccttcct cctccaaatc accattacca atctccagat tctccctccc attctcccta 120

aaccccaaca aatcatcctc ctcctcccgc cgccgcggta tcaaatccag ctctccctcc 180

tccatctccg ccgtgctcaa cacaaccacc aatgtcacaa ccactccctc tccaaccaaa 240

cctaccaaac ccgaaacatt catctcccga ttcgctccag atcaaccccg caaaggcgct 300

gatatcctcg tcgaagcttt agaacgtcaa ggcgtagaaa ccgtattcgc ttaccctgga 360

ggtgcatcaa tggagattca ccaagcctta acccgctctt cctcaatccg taacgtcctt 420

cctcgtcacg aacaaggagg tgtattcgca gcagaaggat acgctcgatc ctcaggtaaa 480

ccaggtatct gtatagccac ttcaggtccc ggagctacaa atctcgttag cggattagcc 540

gatgcgttgt tagatagtgt tcctcttgta gcaatcacag gacaagtccc tcgtcgtatg 600

attggtacag atgcgtttca agagactccg attgttgagg taacgcgttc gattacgaag 660

cataactatc ttgtgatgga tgttgaagat atccctagga ttattgagga agctttcttt 720

ttagctactt ctggtagacc tggacctgtt ttggttgatg ttcctaaaga tattcaacaa 780

cagcttgcga ttcctaattg ggaacaggct atgagattac ctggttatat gtctaggatg 840

cctaaacctc cggaagattc tcatttggag cagattgtta ggttgatttc tgagtctaag 900

aagcctgtgt tgtatgttgg tggtggttgt ttgaattcta gcgatgaatt gggtaggttt 960

gttgagctta cggggatccc tgttgcgagt acgttgatgg ggctgggatc ttatccttgt 1020

gatgatgagt tgtcgttaca tatgcttgga atgcatggga ctgtgtatgc aaattacgct 1080

gtggagcata gtgatttgtt gttggcgttt ggggtaaggt ttgatgatcg tgtcacgggt 1140

aagcttgagg cttttgctag tagggctaag attgttcata ttgatattga ctcggctgag 1200

attgggaaga ataagactcc tcatgtgtct gtgtgtggtg atgttaagct ggctttgcaa 1260

gggatgaata aggttcttga gaaccgagcg gaggagctta agcttgattt tggagtttgg 1320

aggaatgagt tgaacgtaca gaaacagaag tttccgttga gctttaagac gtttggggaa 1380

gctattcctc cacagtatgc gattaaggtc cttgatgagt tgactgatgg aaaagccata 1440

ataagtactg gtgtcgggca acatcaaatg tgggcggcgc agttctacaa ttacaagaaa 1500

ccaaggcagt ggctatcatc aggaggcctt ggagctatgg gatttggact tcctgctgcg 1560

attggagcgt ctgttgctaa ccctgatgcg atagttgtgg atattgacgg agatggaagc 1620

tttataatga atgtgcaaga gctagccact attcgtgtag agaatcttcc agtgaaggta 1680

cttttattaa acaaccagca tcttggcatg gttatgcaat gggaagatcg gttctacaaa 1740

gctaaccgag ctcacacatt tctcggggat ccggctcagg aggacgagat attcccgaac 1800

atgttgctgt ttgcagcagc ttgcgggatt ccagcggcga gggtgacaaa gaaagcagat 1860

ctccgagaag ctattcagac aatgctggat acaccaggac cttacctgtt ggatgtgatt 1920

tgtccgcacc aagaacatgt gttgccgatg atcccgagtg gtggcacttt caacgatgtc 1980

ataacggaag gagatggccg gattaaatac tga 2013

<210> 32
<211> 1917
<212> DNA
<213> Zea mays

<220>
<221> misc_feature
<223> Maize acetohydroxyacid synthase large subunit 2 (ZmAHASL2) coding
sequence, wild type allele.

<400> 32
atggccaccg ccgccgccgc gtctaccgcg ctcactggcg ccactaccgc tgcgcccaag 60

gcgaggcgcc gggcgcacct cctggccacc cgccgcgccc tcgccgcgcc catcaggtgc 120

tcagcggcgt cacccgccat gccgatggct cccccggcca ccccgctccg gccgtggggc 180

cccaccgatc cccgcaaggg cgccgacatc ctcgtcgagt ccctcgagcg ctgcggcgtc 240

cgcgacgtct tcgcctaccc cggcggcgcg tccatggaga tccaccaggc actcacccgc 300

tcccccgtca tcgccaacca cctcttccgc cacgagcaag gggaggcctt tgcggcctcc 360

ggctacgcgc gctcctcggg ccgcgtcggc gtctgcatcg ccacctccgg ccccggcgcc 420

accaaccttg tctccgcgct cgccgacgcg ctgctcgatt ccgtccccat ggtcgccatc 480

acgggacagg tgccgcgacg catgattggc accgacgcct tccaggagac gcccatcgtc 540

gaggtcaccc gctccatcac caagcacaac tacctggtcc tcgacgtcga cgacatcccc 600

cgcgtcgtgc aggaggcttt cttcctcgcc tcctctggtc gaccggggcc ggtgcttgtc 660

gacatcccca aggacatcca gcagcagatg gcggtgcctg tctgggacaa gcccatgagt 720

ctgcctgggt acattgcgcg ccttcccaag ccccctgcga ctgagttgct tgagcaggtg 780

ctgcgtcttg ttggtgaatc ccggcgccct gttctttatg ttggcggtgg ctgcgcagca 840

tctggtgagg agttgcgacg ctttgtggag ctgactggaa tcccggtcac aactactctt 900

atgggcctcg gcaacttccc cagcgacgac ccactgtctc tgcgcatgct aggtatgcat 960

ggcacggtgt atgcaaatta tgcagtggat aaggccgatc tgttgcttgc acttggtgtg 1020

cggtttgatg atcgtgtgac agggaagatt gaggcttttg caagcagggc taagattgtg 1080

cacgttgata ttgatccggc tgagattggc aagaacaagc agccacatgt gtccatctgt 1140

gcagatgtta agcttgcttt gcagggcatg aatgctcttc ttgaaggaag cacatcaaag 1200

aagagctttg actttggctc atggaacgat gagttggatc agcagaagag ggaattcccc 1260

cttgggtata aaacatctaa tgaggagatc cagccacaat atgctattca ggttcttgat 1320

gagctgacga aaggcgaggc catcatcggc acaggtgttg ggcagcacca gatgtgggcg 1380

gcacagtact acacttacaa gcggccaagg cagtggttgt cttcagctgg tcttggggct 1440

atgggatttg gtttgccggc tgctgctggt gcttctgtgg ccaacccagg tgttactgtt 1500

gttgacatcg atggagatgg tagctttctc atgaacgttc aggagctagc tatgatccga 1560

attgagaacc tcccggtgaa ggtctttgtg ctaaacaacc agcacctggg gatggtggtg 1620

cagtgggagg acaggttcta taaggccaac agagcgcaca catacttggg aaacccagag 1680

aatgaaagtg agatatatcc agatttcgtg acgatcgcca aagggttcaa cattccagcg 1740

gtccgtgtga caaagaagaa cgaagtccgc gcagcgataa agaagatgct cgagactcca 1800

gggccgtacc tcttggatat aatcgtccca caccaggagc atgtgttgcc tatgatccct 1860

agtggtgggg ctttcaagga tatgatcctg gatggtgatg gcaggactgt gtactga 1917

<210> 33
<211> 3234
<212> DNA
<213> Arabidopsis thaliana

<220>
<221> misc_feature
<223> Arabidopsis acetohydroxyacid synthase large subunit (AtAHASL)
genomic fragment, S653N allele.

<400> 33
atggcggcgg caacaacaac aacaacaaca tcttcttcga tctccttctc caccaaacca 60

tctccttcct cctccaaatc accattacca atctccagat tctccctccc attctcccta 120

aaccccaaca aatcatcctc ctcctcccgc cgccgcggta tcaaatccag ctctccctcc 180

tccatctccg ccgtgctcaa cacaaccacc aatgtcacaa ccactccctc tccaaccaaa 240

cctaccaaac ccgaaacatt catctcccga ttcgctccag atcaaccccg caaaggcgct 300
gatatcctcg tcgaagcttt agaacgtcaa ggcgtagaaa ccgtattcgc ttaccctgga 360
ggtgcatcaa tggagattca ccaagcctta acccgctctt cctcaatccg taacgtcctt 420
cctcgtcacg aacaaggagg tgtattcgca gcagaaggat acgctcgatc ctcaggtaaa 480
ccaggtatct gtatagccac ttcaggtccc ggagctacaa atctcgttag cggattagcc 540
gatgcgttgt tagatagtgt tcctcttgta gcaatcacag gacaagtccc tcgtcgtatg 600
attggtacag atgcgtttca agagactccg attgttgagg taacgcgttc gattacgaag 660
cataactatc ttgtgatgga tgttgaagat atccctagga ttattgagga agctttcttt 720
ttagctactt ctggtagacc tggacctgtt ttggttgatg ttcctaaaga tattcaacaa 780
cagcttgcga ttcctaattg ggaacaggct atgagattac ctggttatat gtctaggatg 840
cctaaacctc cggaagattc tcatttggag cagattgtta ggttgatttc tgagtctaag 900
aagcctgtgt tgtatgttgg tggtggttgt ttgaattcta gcgatgaatt gggtaggttt 960
gttgagctta cggggatccc tgttgcgagt acgttgatgg ggctgggatc ttatccttgt 1020
gatgatgagt tgtcgttaca tatgcttgga atgcatggga ctgtgtatgc aaattacgct 1080
gtggagcata gtgatttgtt gttggcgttt ggggtaaggt ttgatgatcg tgtcacgggt 1140
aagcttgagg cttttgctag tagggctaag attgttcata ttgatattga ctcggctgag 1200
attgggaaga ataagactcc tcatgtgtct gtgtgtggtg atgttaagct ggctttgcaa 1260
gggatgaata aggttcttga gaaccgagcg gaggagctta agcttgattt tggagtttgg 1320
aggaatgagt tgaacgtaca gaaacagaag tttccgttga gctttaagac gtttggggaa 1380
gctattcctc cacagtatgc gattaaggtc cttgatgagt tgactgatgg aaaagccata 1440
ataagtactg gtgtcgggca acatcaaatg tgggcggcgc agttctacaa ttacaagaaa 1500
ccaaggcagt ggctatcatc aggaggcctt ggagctatgg gatttggact tcctgctgcg 1560
attggagcgt ctgttgctaa ccctgatgcg atagttgtgg atattgacgg agatggaagc 1620
tttataatga atgtgcaaga gctagccact attcgtgtag agaatcttcc agtgaaggta 1680
cttttattaa acaaccagca tcttggcatg gttatgcaat gggaagatcg gttctacaaa 1740
gctaaccgag ctcacacatt tctcggggat ccggctcagg aggacgagat attcccgaac 1800
atgttgctgt ttgcagcagc ttgcgggatt ccagcggcga gggtgacaaa gaaagcagat 1860
ctccgagaag ctattcagac aatgctggat acaccaggac cttacctgtt ggatgtgatt 1920
tgtccgcacc aagaacatgt gttgccgatg atcccgaatg gtggcacttt caacgatgtc 1980
ataacggaag gagatggccg gattaaatac tgagagatga aaccggtgat tatcagaacc 2040
ttttatggtc tttgtatgca tatggtaaaa aaacttagtt tgcaatttcc tgtttgtttt 2100
ggtaatttga gtttctttta gttgttgatc tgcctgcttt ttggtttacg tcagactact 2160
actgctgttg ttgtttggtt tcctttcttt cattttataa ataaataatc cggttcggtt 2220

tactccttgt gactggctca gtttggttat tgcgaaatgc gaatggtaaa ttgagtaatt 2280

gaaattcgtt attagggttc taagctgttt taacagtcac tgggttaata tctctcgaat 2340

cttgcatgga aaatgctctt accattggtt tttaattgaa atgtgctcat atgggccgtg 2400

gtttccaaat taaataaaac tacgatgtca tcgagaagta aaatcaactg tgtccacatt 2460

atcagttttg tgtatacgat gaaatagggt aattcaaaat ctagcttgat atgccttttg 2520

gttcatttta accttctgta aacatttttt cagattttga acaagtaaat ccaaaaaaaa 2580

aaaaaaaaaa tctcaactca acactaaatt attttaatgt ataaaagatg cttaaaacat 2640

ttggcttaaa agaaagaagc taaaaacata gagaactctt gtaaattgaa gtatgaaaat 2700

atactgaatt gggtattata tgaatttttc tgatttagga ttcacatgat ccaaaaagga 2760

aatccagaag cactaatcag acattggaag taggaatatt tcaaaaagtt tttttttttt 2820

aagtaagtga caaaagcttt taaaaaatag aaaagaaact agtattaaag ttgtaaattt 2880

aataaacaaa agaaattttt tatatttttt catttctttt tccagcatga ggttatgatg 2940

gcaggatgtg gatttcattt ttttcctttt gatagccttt taattgatct attataattg 3000

acgaaaaaat attagttaat tatagatata ttttaggtag tattagcaat ttacacttcc 3060

aaaagactat gtaagttgta aatatgatgc gttgatctct tcatcattca atggttagtc 3120

aaaaaaataa aagcttaact agtaaactaa agtagtcaaa aattgtactt tagtttaaaa 3180

tattacatga ataatccaaa acgacattta tgtgaaacaa aaacaatatc taga 3234

<210> 34
<211> 5717
<212> DNA
<213> Arabidopsis thaliana


<220>
<221> misc_feature
<223> Arabidopsis acetohydroxyacid synthase large subunit (AtAHASL)
      genomic fragment, S653N allele.

<400> 34
tctagattat gtatttccaa ctttcattaa caatataatc gcatataaat gaaaaatcgt 60

ttccaggata atattttgat gaaatctcat attattgttc gtactcggat tgatgttgaa 120

ggcttgaagc gcttcaaatt atagaccaga ttatttaagt ttttcttttg tttactccat 180

atcaatttga tccattatac tacctaagaa aatttaggta acatagaatt atttattgtt 240

atagtaaaaa aaaggaaaac cacaaaaata atctactttt acgtatatac tattttcatg 300

acataagtaa ttaagttgta caactttttt ttaatgaaaa gagagagtaa atttatcatg 360

ttcatgtgta gttacctcgt gaataaccga cggttatata gacgcctaac atgaattgtt 420

cagttgaaga cagttcaaaa catgtgtttc actctaaaat cctcaacaaa aaaaaagtgt 480

taaaatttgt aaacctcttt caagcaaaaa aagaaaaagt gttagaatcc caagattctt 540

tcataatccg gaatcttggc tgaaaacgta taaaagagat tgacgtagta acaaggagtc 600

ttggtatgct tccatgcttt ttatcctttt ttgtcatgga accatgattt ggttaccatt 660

tattatgtaa ccgaaatttt cattgtaata atgaatattt aaatttttag caaaaaaaaa 720

caaaaaaaaa caaggagtct tgtcttcgtt ctcaaatttc agagctcttg cacttttcaa 780

gagttttact ttgatgagtg agacatttgt ctttttagtg tttattttct aaacttaaaa 840

tagtagcatc aacatcactc aattataatt cttaagatgt tgtagaaaaa tattttatag 900

atggaaagta atcgatatta agacaaataa gaaaccaaac cggactttgt gttcagaccg 960

aatcaaatct gaattggaga aattatggtg gaggcgaaag tcaacggaac taaagtataa 1020

aaccaaatgt caaaaataaa acccaatttt catccttaaa cgaacctgct gaaaccctaa 1080

tttcgattac caattccgat ctaaaaagaa gtcatggaag ccattgattc cgcaatcgat 1140

cctctcagag atttcgctaa gagcagtgtt cgtctcgtcc agcgctgtca caaacccgat 1200

cgcaagggta acgccttttc tcaaaaaaat ctcatttccg atttttgatc tgtagattag 1260

ggttttctga aattttgata tcatttgtaa ttgaattggt tatcagaatt cacgaaagta 1320

gctgtgcgta cggcgattgg atttgtggtg atgggattcg ttggattctt cgtgaagctc 1380

gttttcatcc caatcaacaa catcatcgtt ggatcttctt agtgtagtac tttctttacg 1440

aggtaattga tctcgcatta tatatctaca ttttggttat gttacttgac atatagtcat 1500

tgattcaata gttctgttaa ttcctttaaa gatcattttg actagaccac attcttggtt 1560

cattcctcaa taatttgtaa tcatattggt ggatatagaa gtagattggt tatagatcag 1620

atagtggaag actttaggat gaatttcagc tagttttttt ttttggctta ttgtctcaaa 1680

agattagtgc tttgctgtct ccattgcttc tgctatcgac acgcttctgt ctccttgtat 1740

ctttattata tctattcgtc ccatgagttt tgtttgttct gtattcgttc gctctggtgt 1800

catggatgga gtctctgttc catgtttctg taatgcatgt tgggttgttt catgcaagaa 1860

atgctgagat aaacactcat ttgtgaaagt ttctaaactc tgaatcgcgc tacaggcaat 1920

gctccgagga gtaggaggag aagaacgaac caaacgacat tatcagccct ttgaggaagc 1980

tcttagtttt gttattgttt ttgtagccaa attctccatt cttattccat tttcacttat 2040

ctcttgttcc ttatagacct tataagtttt ttattcatgt atacaaatta tattgtcatc 2100

aagaagtatc tttaaaatct aaatctcaaa tcaccaggac tatgtttttg tccaattcgt 2160

ggaaccaact tgcagcttgt atccattctc ttaaccaata aaaaaagaaa gaaagatcaa 2220

tttgataaat ttctcagcca caaattctac atttaggttt tagcatatcg aaggctcaat 2280

cacaaataca atagatagac tagagattcc agcgtcacgt gagttttatc tataaataaa 2340

ggaccaaaaa tcaaatcccg agggcatttt cgtaatccaa cataaaaccc ttaaacttca 2400

agtctcattt ttaaacaaat catgttcaca agtctcttct tcttctctgt ttctctatct 2460

cttgctcatc tttctcctga accatggcgg cggcaacaac aacaacaaca acatcttctt 2520
cgatctcctt ctccaccaaa ccatctcctt cctcctccaa atcaccatta ccaatctcca 2580
gattctccct cccattctcc ctaaacccca acaaatcatc ctcctcctcc cgccgccgcg 2640
gtatcaaatc cagctctccc tcctccatct ccgccgtgct caacacaacc accaatgtca 2700
caaccactcc ctctccaacc aaacctacca aacccgaaac attcatctcc cgattcgctc 2760
cagatcaacc ccgcaaaggc gctgatatcc tcgtcgaagc tttagaacgt caaggcgtag 2820
aaaccgtatt cgcttaccct ggaggtgcat caatggagat tcaccaagcc ttaacccgct 2880
cttcctcaat ccgtaacgtc cttcctcgtc acgaacaagg aggtgtattc gcagcagaag 2940
gatacgctcg atcctcaggt aaaccaggta tctgtatagc cacttcaggt cccggagcta 3000
caaatctcgt tagcggatta gccgatgcgt tgttagatag tgttcctctt gtagcaatca 3060
caggacaagt ccctcgtcgt atgattggta cagatgcgtt tcaagagact ccgattgttg 3120
aggtaacgcg ttcgattacg aagcataact atcttgtgat ggatgttgaa gatatcccta 3180
ggattattga ggaagctttc tttttagcta cttctggtag acctggacct gttttggttg 3240
atgttcctaa agatattcaa caacagcttg cgattcctaa ttgggaacag gctatgagat 3300
tacctggtta tatgtctagg atgcctaaac ctccggaaga ttctcatttg gagcagattg 3360
ttaggttgat ttctgagtct aagaagcctg tgttgtatgt tggtggtggt tgtttgaatt 3420
ctagcgatga attgggtagg tttgttgagc ttacggggat ccctgttgcg agtacgttga 3480
tggggctggg atcttatcct tgtgatgatg agttgtcgtt acatatgctt ggaatgcatg 3540
ggactgtgta tgcaaattac gctgtggagc atagtgattt gttgttggcg tttggggtaa 3600
ggtttgatga tcgtgtcacg ggtaagcttg aggcttttgc tagtagggct aagattgttc 3660
atattgatat tgactcggct gagattggga agaataagac tcctcatgtg tctgtgtgtg 3720
gtgatgttaa gctggctttg caagggatga ataaggttct tgagaaccga gcggaggagc 3780
ttaagcttga ttttggagtt tggaggaatg agttgaacgt acagaaacag aagtttccgt 3840
tgagctttaa gacgtttggg gaagctattc ctccacagta tgcgattaag gtccttgatg 3900
agttgactga tggaaaagcc ataataagta ctggtgtcgg gcaacatcaa atgtgggcgg 3960
cgcagttcta caattacaag aaaccaaggc agtggctatc atcaggaggc cttggagcta 4020
tgggatttgg acttcctgct gcgattggag cgtctgttgc taaccctgat gcgatagttg 4080
tggatattga cggagatgga agctttataa tgaatgtgca agagctagcc actattcgtg 4140
tagagaatct tccagtgaag gtacttttat taaacaacca gcatcttggc atggttatgc 4200
aatgggaaga tcggttctac aaagctaacc gagctcacac atttctcggg gatccggctc 4260
aggaggacga gatattcccg aacatgttgc tgtttgcagc agcttgcggg attccagcgg 4320
cgagggtgac aaagaaagca gatctccgag aagctattca gacaatgctg gatacaccag 4380
gaccttacct gttggatgtg atttgtccgc accaagaaca tgtgttgccg atgatcccga 4440

atggtggcac tttcaacgat gtcataacgg aaggagatgg ccggattaaa tactgagaga 4500

tgaaaccggt gattatcaga acctttatgg gtctttgtat gcatatggta aaaaaactta 4560

gtttgcaatt tcctgtttgt tttggtaatt tgagtttctt ttagttgttg atctgcctgc 4620

tttttggttt acgtcagact actactgctg ttgttgtttg gtttcctttc tttcatttta 4680

taaataaata atccggttcg gtttactcct tgtgactggc tcagtttggt tattgcgaaa 4740

tgcgaatggt aaattgagta attgaaattc gttattaggg ttctaagctg ttttaacagt 4800

cactgggtta atatctctcg aatcttgcat ggaaaatgct cttaccattg gtttttaatt 4860

gaaatgtgct catatgggcc gtggtttcca aattaaataa aactacgatg tcatcgagaa 4920

gtaaaatcaa ctgtgtccac attatcagtt ttgtgtatac gatgaaatag ggtaattcaa 4980

aatctagctt gatatgcctt ttggttcatt ttaaccttct gtaaacattt tttcagattt 5040

tgaacaagta aatccaaaaa aaaaaaaaaa aaatctcaac tcaacactaa attattttaa 5100

tgtataaaag atgcttaaaa catttggctt aaaagaaaga agctaaaaac atagagaact 5160

cttgtaaatt gaagtatgaa aatatactga attgggtatt atatgaattt ttctgattta 5220

ggattcacat gatccaaaaa ggaaatccag aagcactaat cagacattgg aagtaggaat 5280

atttcaaaaa gttttttttt tttaagtaag tgacaaaagc ttttaaaaaa tagaaaagaa 5340

actagtatta aagttgtaaa tttaataaac aaaagaaatt ttttatattt tttcatttct 5400

ttttccagca tgaggttatg atggcaggat gtggatttca tttttttcct tttgatagcc 5460

ttttaattga tctattataa ttgacgaaaa aatattagtt aattatagat atattttagg 5520

tagtattagc aatttacact tccaaaagac tatgtaagtt gtaaatatga tgcgttgatc 5580

tcttcatcat tcaatggtta gtcaaaaaaa taaaagctta actagtaaac taaagtagtc 5640

aaaaattgta ctttagttta aaatattaca tgaataatcc aaaacgacat ttatgtgaaa 5700

caaaaacaat atctaga 5717

## Description

### FIELD OF THE INVENTION

This invention relates generally to compositions and methods for increasing tolerance of plants to acetohydroxyacid synthase-inhibiting herbicides.

### BACKGROUND OF THE INVENTION

Acetohydroxyacid synthase (AHAS; EC 4.1.3.18, also known as acetolactate synthase or ALS), is the first enzyme that catalyzes the biochemical synthesis of the branched chain amino acids valine, leucine and isoleucine (Singh (1999) "Biosynthesis of valine, leucine and isoleucine," in Plant Amino Acids, Singh, B.K., ed., Marcel Dekker Inc. New York, New York, pp. 227-247). AHAS is the site of action of four structurally diverse herbicide families including the sulfonylureas (Tan et al. (2005) Pest Manag. Sci. 61:246-57; Mallory-Smith and Retzinger (2003) Weed Technology 17:620-626; LaRossa and Falco (1984) Trends Biotechnol. 2:158-161), the imidazolinones (Shaner et al. (1984) Plant Physiol. 76:545-546), the triazolopyrimidines (Subramanian and Gerwick (1989) "Inhibition of acetolactate synthase by triazolopyrimidines," in Biocatalysis in Agricultural Biotechnology, Whitaker, J.R. and Sonnet, P.E.. eds., ACS Symposium Series, American Chemical Society, Washington, D.C., pp. 277-288), and the pyrimidinyloxybenzoates (Subramanian et al. (1990) Plant Physiol. 94: 239-244). Imidazolinone and sulfonylurea herbicides are widely used in modem agriculture due to their effectiveness at very low application rates and relative non-toxicity in animals. By inhibiting AHAS activity, these families of herbicides prevent further growth and development of susceptible plants including many weed species. Several examples of commercially available imidazolinone herbicides are PURSUIT® (imazethapyr), SCEPTER® (imazaquin) and ARSENAL® (imazapyr). Examples of sulfonylurea herbicides are chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfiuon, fluzasulfuron, imazosulfuron, pyrazosulfuron ethyl and halosulfuron.

Due to their high effectiveness and low-toxicity, imidazolinone herbicides are favored for application by spraying over the top of a wide area of vegetation. The ability to spray a herbicide over the top of a wide range of vegetation decreases the costs associated with plant establishment and maintenance, and decreases the need for site preparation prior to use of such chemicals. Spraying over the top of a desired tolerant species also results in the ability to achieve maximum yield potential of the desired species due to the absence of competitive species. However, the ability to use such spray-over techniques is dependent upon the presence of imidazolinone-resistant species of the desired vegetation in the spray over area.

Among the major agricultural crops, some leguminous species such as soybean are naturally resistant to imidazolinone herbicides due to their ability to rapidly metabolize the herbicide compounds (Shaner and Robinson (1985) Weed Sci. 33:469-471). Other crops such as corn (Newhouse et al. (1992) Plant Physiol. 100:882-886) and rice (Barrett et al. (1989) Crop Safeners for Herbicides, Academic Press, New York, pp. 195-220) are somewhat susceptible to imidazolinone herbicides. The differential sensitivity to the imidazolinone herbicides is dependent on the chemical nature of the particular herbicide and differential metabolism of the compound from a toxic to a non-toxic form in each plant (Shaner et al. (1984) Plant Physiol. 76:545-546; Brown et al. (1987) Pestic. Biochem. Physiol. 27:24-29). Other plant physiological differences such as absorption and translocation also play an important role in sensitivity (Shaner and Robinson (1985) Weed Sci. 33:469-471).

Plants resistant to imidazolinones, sulfonylureas, triazolopyrimidines, and pyrimidinyloxybenzoates have been successfully produced using seed, microspore, pollen, and callus mutagenesis in *Zea mays, Arabidopsis thaliana, Brassica napus (i.e.,* canola) *Glycine max, Nicotiana tabacum,* sugarbeet *(Beta vulgaris)* and *Oryza sativa* (Sebastian et al. (1989) Crop Sci. 29:1403-1408; Swanson et al. 1989 Theor. Appl. Genet. 78:525-530; Newhouse et al. (1991) Theor. Appl. Genet. 83:65-70; Sathasivan et al. (1991) Plant Physiol. 97:1044-1050; Mourand et al. (1993) J. Heredity 84:91-96; Wright and Penner (1998) Theor. Appl. Genet. 96:612-620; U.S. Patent No. 5,545,822). In all cases, a single, partially dominant nuclear gene conferred resistance. Four imidazolinone resistant wheat plants were also previously isolated following seed mutagenesis of *Triticum aestivum* L. cv. Fidel (Newhouse et al. (1992) Plant Physiol. 100:882-886). Inheritance studies confirmed that a single, partially dominant gene conferred resistance. Based on allelic studies, the authors concluded that the mutations in the four identified lines were located at the same locus. One of the Fidel cultivar resistance genes was designated FS-4 (Newhouse et al. (1992) Plant Physiol. 100:882-886).

Computer-based modeling of the three dimensional conformation of the AHAS-inhibitor complex predicts several amino acids in the proposed inhibitor binding pocket as sites where induced mutations would likely confer selective resistance to imidazolinones (Ott et al. (1996) J. Mol. Biol. 263:359-368). Tobacco plants produced with some of these rationally designed mutations in the proposed binding sites of the AHAS enzyme have in fact exhibited specific resistance to a single class of herbicides (Ott et al. (1996) J. Mol. Biol. 263:359-368).

Plant resistance to imidazolinone herbicides has also been reported in a number of patents. U.S. Patent Nos. 4,761,373, 5,331,107, 5,304,732, 6,211,438, 6,211,439 and 6,222,100 generally describe the use of an altered AHAS gene to elicit herbicide resistance in plants, and specifically discloses certain imidazolinone resistant corn lines. U.S. Patent No. 5,013,659 discloses plants exhibiting herbicide resistance due to mutations in at least one amino acid in one or more conserved regions. The mutations described therein encode either cross-resistance for imidazolinones and sulfonylureas or sulfonylurea-specific resistance, but imidazolinone-specific resistance is not described. U.S. Patent No. 5,731,180 and U.S. Patent No. 5,767,361 discuss an isolated gene having a single amino acid substitution in a wild-type monocot AHAS amino acid sequence that results in imidazolinone-specific resistance. In addition, rice plants that are resistant to herbicides that interfere with AHAS have been developed by mutation breeding and tissue culture selection. *See,* U.S. Patent Nos. 5,545,822, 5,736,629, 5,773,703, 5,773,704, 5,952,553 and 6,274,796.

In plants, as in all other organisms examined, the AHAS enzyme is comprised of two subunits: a large subunit (catalytic role) and a small subunit (regulatory role) (Duggleby and Pang (2000) J. Biochem. Mol. Biol. 33:1-36). The AHAS large subunit (also referred to herein as AHASL) may be encoded by a single gene as in the case of *Arabidopsis,* and sugar beet or by multiple gene family members as in maize, canola, and cotton. Specific, single-nucleotide substitutions in the large subunit confer upon the enzyme a degree of insensitivity to one or more classes of herbicides (Chang and Duggleby (1998) Biochem J. 333:765-777).

For example, bread wheat, *Triticum aestivum* L., contains three homoeologous acetohydroxyacid synthase large subunit genes. Each of the genes exhibit significant expression based on herbicide response and biochemical data from mutants in each of the three genes (Ascenzi et al. (2003) International Society of Plant Molecular Biologists Congress, Barcelona, Spain, Ref. No. S10-17). The coding sequences of all three genes share extensive homology at the nucleotide level (WO 03/014357). Through sequencing the AHASL genes from several varieties of *Triticum aestivum,* the molecular basis of herbicide tolerance in most IMI-tolerant (imidazolinone-tolerant) lines was found to be the mutation S653(*At*)N, indicating a serine to asparagine substitution at a position equivalent to the serine at amino acid 653 in *Arabidopsis thaliana* (WO 03/014357). This mutation is due to a single nucleotide polymorphism (SNP) in the DNA sequence encoding the AHASL protein.

Multiple AHASL genes are also known to occur in dicotyledonous plants species. Recently, Kolkman et al. ((2004) Theor. Appl. Genet. 109: 1147-1159) reported the identification, cloning, and sequencing for three AHASL genes (AHASL1, AHASL2, and AHASL3) from herbicide-resistant and wild type genotypes of sunflower *(Helianthus annuus* L.). Kolkman *et al.* reported that the herbicide-resistance was due either to the Pro197Leu (using the *Arabidopsis* AHASL amino acid position nomenclature) substitution or the Ala205Val substitution in the AHASL1 protein and that each of these substitutions provided resistance to both imidazolinone and sulfonylurea herbicides.

A number of single mutations in the AHAS large subunit are known that result in tolerance or resistance to herbicides (Duggleby et al. (2000) Journal of Biochem and Mol. Bio. 33:1-36; Jander et al. (2003) Plant Physiology 131:139-146). For example, an alanine to valine substitution at position 122 *of Arabidopsis* AHASL (or an alanine to threonine substitution at corresponding position 100 of Cocklebur AHASL) confers resistance to imidazolinone and sulfonylureas. A methionine to glutamic acid or isoleucine substitution at position 124 of *Arabidopsis* AHASL confers resistance to imidazolinones and sulfonylureas. A proline to serine substitution at position 197 of *Arabidopsis* AHASL (or a proline to alanine, glutamic acid, leucine, glutamine, arginine, valine, tryptophan, or tyrosine substitution at corresponding position 192 of yeast AHASL) confers resistance to imidazolinones, sulfonylureas, and triazolopyrimidine. An arginine to alanine or glutamic acid substitution at position 199 *of Arabidopsis* AHASL confers imidazolinone resistance. An alanine to valine substitution at position 205 of *Arabidopsis* AHASL (or an alanine to cysteine, aspartic acid, glutamic acid, arginine, threonine, tryptophan or tyrosine substitution at corresponding position 200 of yeast AHASL) confers imidazolinones and sulfonylureas resistance. A substitution of almost any amino acid for the tryptophan at position 574 of *Arabidopsis* AHASL, corresponding to position 586 of yeast AHASL, confers resistance to imidazolinones, sulfonylureas, triazolopyrimidine, and pyrimidyl oxybenzoates. A serine to phenylalanine, asparagine, or threonine substitution at position 653 of *Arabidopsis* AHASL confers resistance to imidazolinones and pyrimidyl oxybenzoates.

U.S. Patent Nos. 5,853,973; 5,928,937; and 6,576,455 disclose structure-based modeling methods for making AHAS variants which include amino acid substitutions at specific positions that differ from the positions described above. In Mourad et al. (1992) Planta 188;491-497, it has shown that mutant lines resistant to sulfonylureas are cross-resistant to triazolopyrimidine, and mutant lines resistant to imidazolinones are cross-resistant to pyrimidyl oxybenzoates.

U.S. Patent No. 5,859,348 discloses a double mutant sugar beet AHAS large subunit having an alanine to threonine substitution at amino acid 113 and a proline to serine substitution at amino acid 188. Sugar beet plants containing the double mutant AHAS protein are described as being both imidazolinone and sulfonylurea resistant.

Mourad et al. (1994) Mol. Gen. Genet. 242:178-184, discloses an *Arabidopsis* AHAS double mutant designated *csr1-4.* The *csr1-4* mutant AHAS contained a C to T nucleotide substitution at position 589 (corresponding to a proline to serine substitution at amino acid 197 of *Arabidopsis* AHASL) and a G to A nucleotide substitution at position 1958 (corresponding to a serine to threonine substitution at amino acid 653 *of Arabidopsis* AHASL).

Lee et al. (1988) EMBO Journal 7:1241-1248, discloses a tobacco AHAS double mutant designated S4-Hra, which includes a Pro-Ala substitution at amino acid 196 (corresponding to the amino acid 197 *of Arabidopsis* AHASL) and a Trp-Leu substitution at amino acid 573 (corresponding to amino acid 574 *of Arabidopsis* AHASL). Transgenic lines carrying the double mutant gene show resistance to sulfonylurea herbicide.

U. S. Patent No. 7,119,256 discloses a double mutant rice AHAS large subunit having a tryptophan to leucine substitution at amino acid 548 and a serine to isoleucine substitution at amino acid 627. Transgenic rice plants expressing a polynucleotide encoding this double mutant AHAS protein were reported to have increased resistance to the pyrimidinyl carboxy herbicide, bispyribac-sodium.

Given their high effectiveness and low-toxicity, imidazolinone herbicides are favored for agricultural use. However, the ability to use imidazolinone herbicides in a particular crop production system depends upon the availability of imidazolinone-resistant varieties of the crop plant of interest. To produce such imidazolinone-resistant varieties, there remains a need for crop plants comprising mutant AHAS polypeptides which confer demonstrated improved tolerance to imidazolinones and/or other AHAS-inhibiting herbicides when compared to crop plants with existing AHAS mutants.

Although some AHAS mutants have been characterized, there remains a need for mutant AHAS polypeptides which confer, when expressed in a crop plant of interest, demonstrated improved herbicide tolerance to one or more classes of AHAS-inhibiting herbicides when compared to existing AHAS mutants in crop plants.

### SUMMARY OF THE INVENTION

This invention relates to new mutant AHAS polypeptides that demonstrate tolerance to a herbicide, in particular, an imidazolinone herbicide, or sulfonylurea herbicide, or a mixture thereof. In preferred embodiments, the herbicide tolerance conferred by the mutants of the invention is improved and/or enhanced relative to that obtained using known AHAS mutants. The mutants of the invention comprise at least two amino acid substitutions in the AHAS large subunit polypeptide.

In one embodiment, the invention provides an isolated polynucleotide encoding an AHAS large subunit double mutant polypeptide selected from the group consisting of a polypeptide having a valine, threonine, glutamine, cysteine, or methionine at a position corresponding to position 122 of SEQ ID NO:1 or position 90 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a valine, threonine, glutamine, cysteine, or methionine at a position corresponding to position 122 of SEQ ID NO:1 or position 90 of SEQ ID NO:2 and a serine, alanine, glutamic acid, leucine, glutamine, arginine, valine, tryptophan, tyrosine, or isoleucine at a position corresponding to position 197 of SEQ ID NO:1or position 165 of SEQ ID NO:2; a polypeptide having a valine, threonine, glutamine, cysteine, or methionine at a position corresponding to position 122 of SEQ ID NO:1 or position 90 of SEQ ID NO:2 and a glutamic acid, isoleucine, leucine, or asparagine at a position corresponding to position 124 of SEQ ID NO:1 or position 92 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 139 of SEQ ID NO:1 or position 107 of SEQ ID NO:2; a polypeptide having a valine, threonine, glutamine, cysteine, or methionine at a position corresponding to position 122 of SEQ ID NO:1 or position 90 of SEQ ID NO:2 and a histidine at a position corresponding to position 269 of SEQ ID NO:1 or position 237 of SEQ ID NO:2; a polypeptide having a valine, threonine, glutamine, cysteine, or methionine at a position corresponding to position 122 of SEQ ID NO:1 or position 90 of SEQ ID NO:2 and a methionine at a position corresponding to position 416 of SEQ ID NO:1 or position 384 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 426 of SEQ ID NO: 1 or position 394 of SEQ ID NO:2; a polypeptide having a valine, threonine, glutamine, cysteine, or methionine at a position corresponding to position 122 of SEQ ID NO:1 or position 90 of SEQ ID NO:2 and a valine at a position corresponding to position 430 of SEQ ID NO:1 or position 398 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 442 of SEQ ID NO:1 or position 410 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine or aspartic acid at a position corresponding to position 445 of SEQ ID NO:1 or position 413 of SEQ ID NO:2; a polypeptide having a valine, threonine, glutamine, cysteine, or methionine at a position corresponding to position 122 of SEQ ID NO:1 or position 90 of SEQ ID NO:2 and a glutamic acid at a position corresponding to position 580 of SEQ ID NO:1 or position 548 of SEQ ID NO:2; a polypeptide having a glutamic acid, isoleucine, leucine, or asparagine at a position corresponding to position 124 of SEQ ID NO:1 or position 92 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an asparagine at a position corresponding to position 375 of SEQ ID NO:1 or position 343 of SEQ ID NO:2; a polypeptide having an alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2; and a polypeptide having a valine, cysteine, aspartic acid, glutamic acid, arginine, threonine, tryptophan, or tyrosine at a position corresponding to position 205 of SEQ ID NO:1 or position 173 of SEQ ID NO:2 and a phenylalanine, asparagine, threonine, glycine, valine, or tryptophan at a position corresponding to position 653 of SEQ ID NO:1 or position 621 of SEQ ID NO:2.

In another embodiment, the invention provides an isolated polynucleotide encoding an AHAS large subunit triple mutant polypeptide selected from the group consisting of a polypeptide having a valine, threonine, glutamine, cysteine, or methionine at a position corresponding to position 122 of SEQ ID NO:1 or position 90 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a valine, threonine, glutamine, cysteine, or methionine at a position corresponding to position 122 of SEQ ID NO:1 or position 90 of SEQ ID NO:2, an arginine at a position corresponding to position 57 of SEQ ID NO:1 and a leucine at a position corresponding to position 398 of SEQ ID NO:1 or position 366 of SEQ ID NO:2; a polypeptide having a glutamic acid, isoleucine, leucine, or asparagine at a position corresponding to position 124 of SEQ ID NO:1 or position 92 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a leucine at a position corresponding to position 95 of SEQ ID NO: 1 or position 63 of SEQ ID NO:2, a glutamic acid at a position corresponding to position 416 of SEQ ID NO:1 or position 3 84 of SEQ ID NO:2 and a phenylalanine, asparagine, threonine, glycine, valine, or tryptophan at a position corresponding to position 653 of SEQ ID NO:1 or position 621 of SEQ ID NO:2; and a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and any amino acid at a position corresponding to position 574 of SEQ ID NO:1 or position 542 of SEQ ID NO:2.

The invention also relates to AHASL polypeptides comprising the double and triple mutants described above, expression vectors comprising the polynucleotides encoding the AHASL double and triple mutants described above, cells comprising the polynucleotides encoding the AHASL double and triple mutants described above, transgenic plants comprising the polynucleotides and polypeptides described above and methods of making and using transgenic plants comprising the polynucleotides encoding the AHASL double and triple mutants described above.

The invention further relates to transgenic and non-transgenic plants comprising one or more polynucleotides comprising two or more mutations. In one embodiment, the plants of the invention comprise a first polynucleotide encoding a first AHASL single mutant polypeptide and a second polynucleotide encoding a second AHASL single mutant polypeptide, or an AHASL encoding polynucleotide comprising two mutations that result in the amino acid mutations of said first and second AHASL single mutant polypeptides, wherein said first and second AHASL single mutant polypeptides are selected from the group consisting of: a first polypeptide having a valine, threonine, glutamine, cysteine, or methionine at a position corresponding to position 122 of SEQ ID NO:1 or position 90 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a glutamic acid, isoleucine, leucine, or asparagine at a position corresponding to position 124 of SEQ ID NO:1 or position 92 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 139 of SEQ ID NO:1 or position 107 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a histidine at a position corresponding to position 269 of SEQ ID NO:1 or position 237 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a methionine at a position corresponding to position 416 of SEQ ID NO:1 or position 3 84 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 426 of SEQ ID NO:1 or position 394 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a valine at a position corresponding to position 430 of SEQ ID NO:1 or position 398 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 442 of SEQ ID NO:1 or position 410 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an isoleucine or aspartic acid at a position corresponding to position 445 of SEQ ID NO:1 or position 413 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a glutamic acid at a position corresponding to position 580 of SEQ ID NO:1 or position 548 of SEQ ID NO:2; a first glutamic acid, isoleucine, leucine, or asparagine at a position corresponding to position 124 of SEQ ID NO:1 or position 92 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2; a first polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2; a first polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having an asparagine at a position corresponding to position 375 of SEQ ID NO:1 or position 343 of SEQ ID NO:2; a first polypeptide having an alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2; a first polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2; and a first polypeptide having a valine, cysteine, aspartic acid, glutamic acid, arginine, threonine, tryptophan, or tyrosine at a position corresponding to position 205 of SEQ ID NO:1 or position 173 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2.

In another embodiment, the invention provides transgenic and non-transgenic plants comprising a first polynucleotide encoding a first AHASL single mutant polypeptide, a second polynucleotide encoding a second AHASL single mutant polypeptide, and a third polynucleotide encoding a third AHASL single mutant polypeptide; or an AHASL encoding polynucleotide comprising three mutations, wherein the three nucleotide mutations result in the amino acid mutations corresponding to the mutations of said first, second and third AHASL single mutant polypeptides; or an AHASL encoding polynucleotide comprising a single mutation and an AHASL encoding polynucleotide comprising a double mutations, wherein the nucleotide mutations result in the amino acid mutations corresponding to the amino acid mutations of said first, second and third AHASL single mutant polypeptides, wherein said first, second, and third AHASL single mutant polypeptides are selected from the group consisting of: a first polypeptide having a valine, threonine, glutamine, cysteine, or methionine at a position corresponding to position 122 of SEQ ID NO:1 or position 90 of SEQ ID NO:2, a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a third polypeptide having a phenylalanine, asparagine, threonine, glycine, valine, or tryptophan at a position corresponding to position 653 of SEQ ID NO:1 or position 621 of SEQ ID NO:2; a first polypeptide having a valine, threonine, glutamine, cysteine, or methionine at a position corresponding to position 122 of SEQ ID NO:1 or position 90 of SEQ ID NO:2, a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a third polypeptide having a phenylalanine, asparagine, threonine, glycine, valine, or tryptophan at a position corresponding to position 653 of SEQ ID NO:1 or position 621 of SEQ ID NO:2; a first polypeptide having a valine, threonine, glutamine, cysteine, or methionine at a position corresponding to position 122 of SEQ ID NO:1 or position 90 of SEQ ID NO:2, a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a third polypeptide having an alanine, glutamic acid, serine, phenylalanine, threonine, aspartic acid, cysteine, or asparagine at a position corresponding to position 199 of SEQ ID NO:1 or position 167 of SEQ ID NO:2; a first polypeptide having a valine, threonine, glutamine, cysteine, or methionine at a position corresponding to position 122 of SEQ ID NO:1 or position 90 of SEQ ID NO:2, a second polypeptide having an arginine at a position corresponding to position 57 of SEQ ID NO:1 and a third polypeptide having a leucine at a position corresponding to position 398 of SEQ ID NO:1 or position 366 of SEQ ID NO:2; a first polypeptide having a glutamic acid, isoleucine, leucine, or asparagine at a position corresponding to position 124 of SEQ ID NO:1 or position 92 of SEQ ID NO:2, a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a third polypeptide having an alanine, glutamic acid, serine, phenylalanine, threonine, aspartic acid, cysteine, or asparagine at a position corresponding to position 199 of SEQ ID NO:1 or position 167 of SEQ ID NO:2; a first polypeptide having a leucine at a position corresponding to position 95 of SEQ ID NO:1 or position 63 of SEQ ID NO:2, a second polypeptide having a glutamic acid at a position corresponding to position 416 of SEQ ID NO:1 or position 3 84 of SEQ ID NO:2 and a third polypeptide having a phenylalanine, asparagine, threonine, glycine, valine, or tryptophan at a position corresponding to position 653 of SEQ ID NO:1 or position 621 of SEQ ID NO:2; and a first polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2, a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a third polypeptide having any amino acid at a position corresponding to position 574 of SEQ ID NO:1 or position 542 of SEQ ID NO:2.

The present invention provides a method for controlling weeds in the vicinity of the transgenic and non-transgenic plants of the invention. Such plants comprise increased herbicide resistance relative to a wild-type plant. The method comprises applying an effective amount of an AHAS-inhibiting herbicide to the weeds and to the plant of the invention.

### BRIEF DESCRIPTION OF THE DRAWINGS

Figure 1 sets forth the full length sequence of the *Arabidopsis* AHAS large subunit protein (amino acid sequence SEQ ID NO: 1; nucleic acid sequence SEQ ID NO: 31) with putative translation showing positions of mutations indicated in bold and underlined. DNA numbering is on the left and amino acid numbering on the right.
Figure 2 sets forth the sequence of the maize AHAS large subunit protein (amino acid sequence SEQ ID NO: 2; nucleic acid sequence SEQ ID NO: 32) with amino acids at positions of claimed mutations indicated in bold and underlined. DNA numbering is on the left and amino acid numbering on the right.

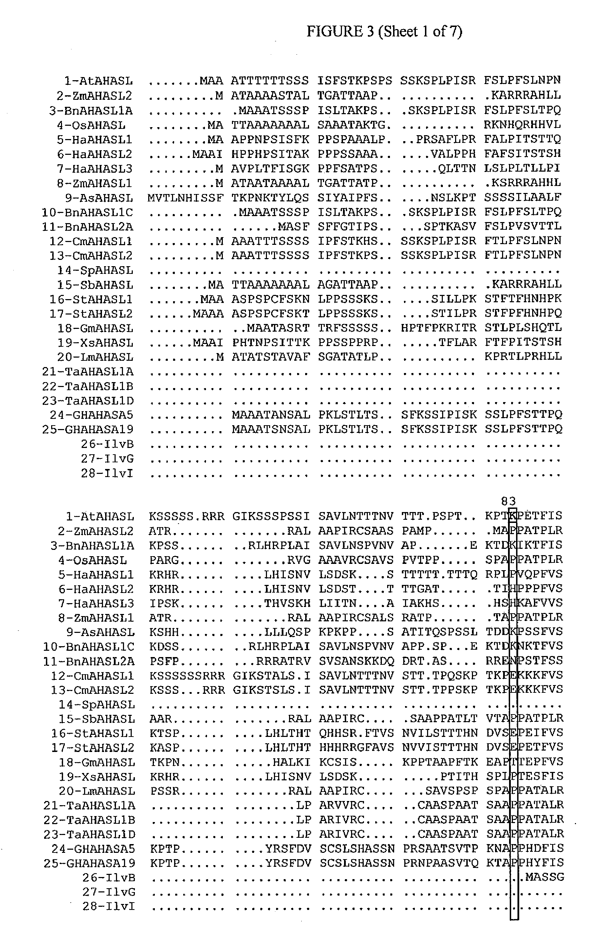

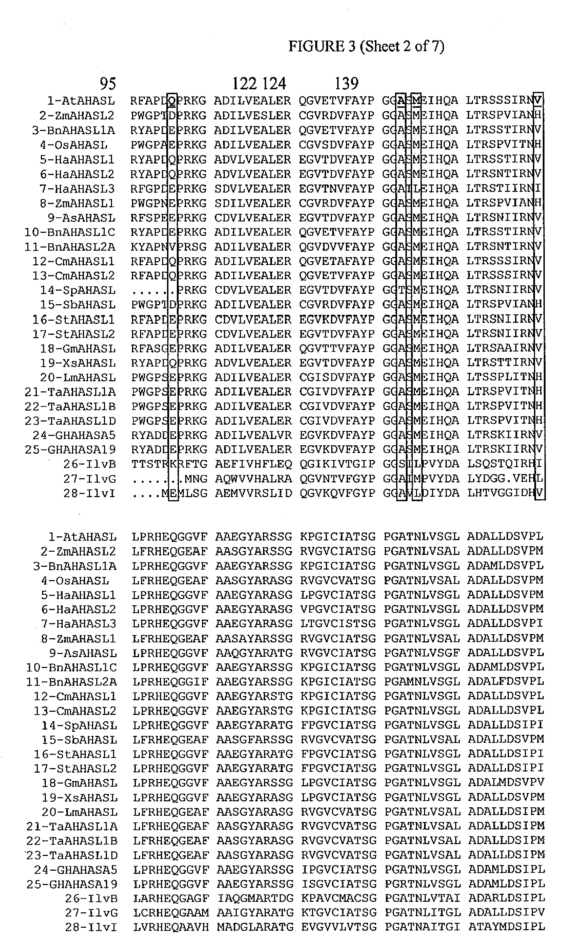

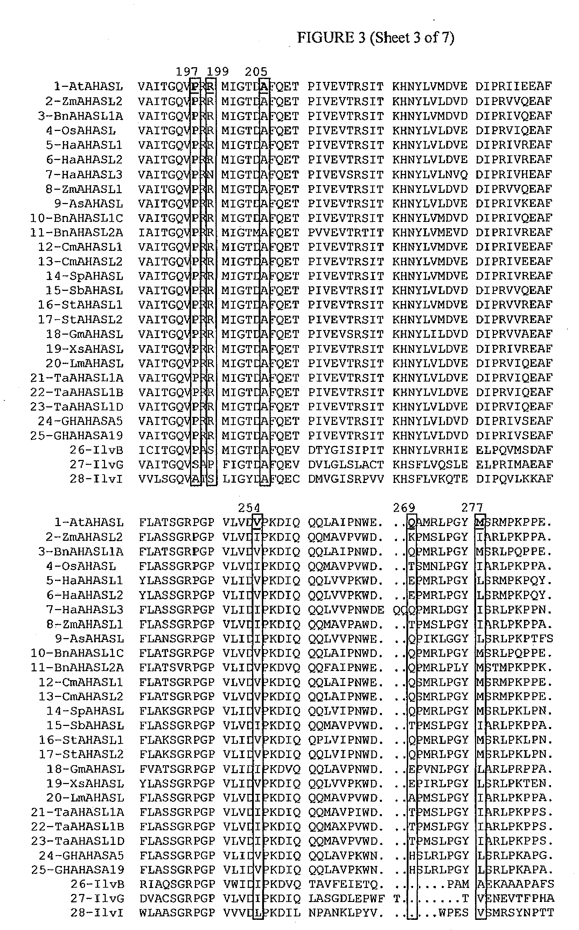

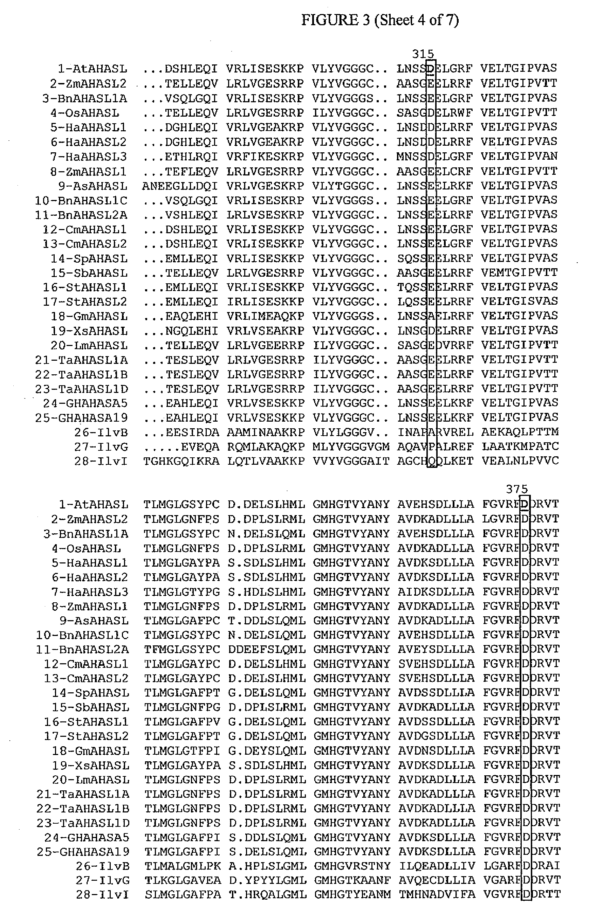

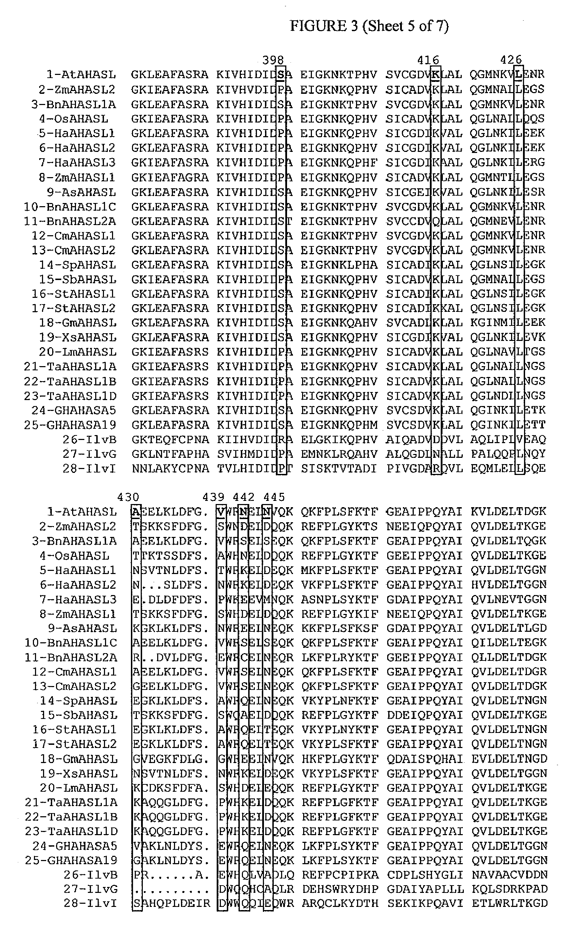

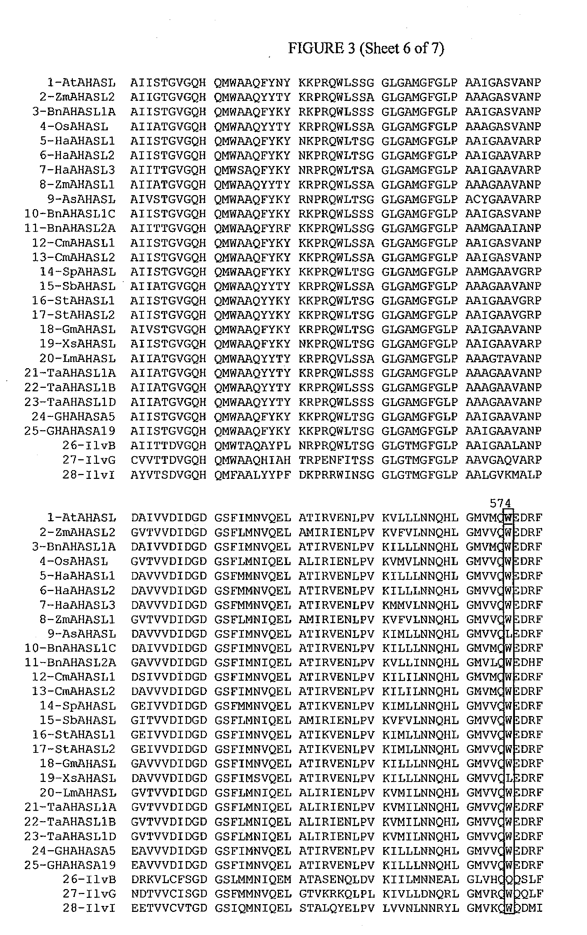

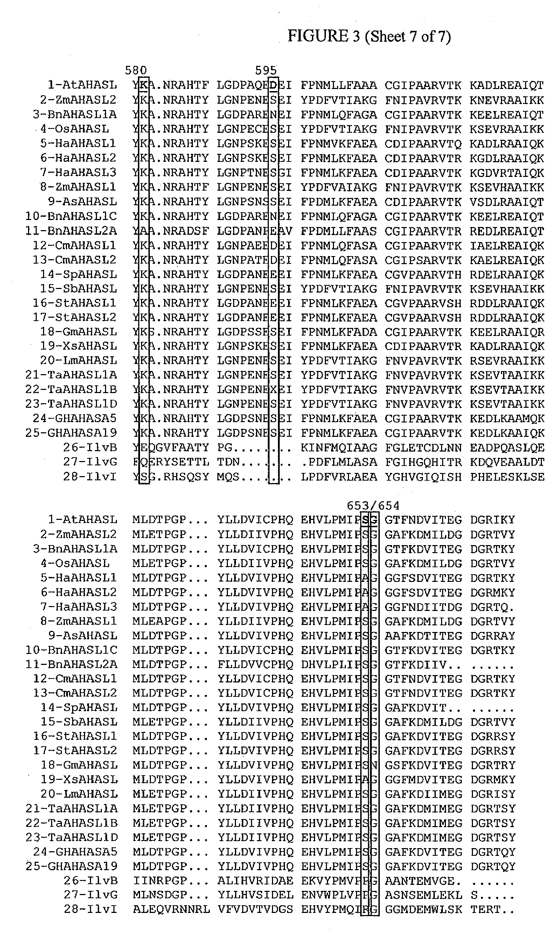

Figure 3 is an alignment of the positions of correspondence of the *Arabidopsis* AHAS large subunit protein (AtAHASL, SEQ ID NO:1) with the AHAS large subunit protein of a number of species where the double and triple mutations of the invention may be made showing the position of substitutions which correspond to the positions of substitution in SEQ ID NO: 1: *Amaranthus* sp. (AsAHASL SEQ ID NO:9), *Brassica napus* (BnAHASL1A SEQ ID NO:3, BnAHASL1C SEQ ID NO: 10, BnAHASL2A SEQ ID NO: 11), *Camelina microcarpa* (CmAHASL1 SEQ ID NO:12, CmAHASL2 SEQ ID NO:13), *Solanum tuberosum* (StAHASL1 SEQ ID NO:16, StAHASL2 SEQ ID NO: 17), *Oryza sativa* (OsAHASL SEQ ID NO:4), *Lolium multiflorum* (LmAHASL SEQ ID NO:20), *Solanum ptychanthum* (SpAHASL SEQ ID NO:14), *Sorghum bicolor* (SbAHASL SEQ ID NO:15), *Glycine max* (GmAHASL SEQ ID NO:18), *Helianthus annuus* (HaAHASL1 SEQ ID NO: 5, HaAHASL2 SEQ ID NO:6, HaAHASL3 SEQ ID NO:7), Triticum aestivum (TaAHASL1A SEQ ID NO:21, TaAHASL1B SEQ ID NO:22, TaAHASL1D SEQ ID NO:23), *Xanthium* sp. (XsAHASL SEQ ID NO:19), *Zea mays* (ZmAHASL1 SEQ ID NO: 8, ZmAHASL2 SEQ ID NO:2), *Gossypium hirsutum* (GhAHASA5 SEQ ID NO:24, GhAHASA19 SEQ ID NO:25), and *E.coli* (ilvB SEQ ID NO:26, ilvG SEQ ID NO:27, ilvI SEQ ID NO:28).
Figure 4 is a map of the AE base vector used for construction of *Arabidopsis* AHASL mutants AE2-AE8 in *E. coli,* with relative positions of mutations in *Arabidopsis* AHASL indicated.
Figure 5 is a vector map of plant transformation base vector AP used for construction of vectors AP2-AP5, which differ only by the mutations indicated in Table 1.

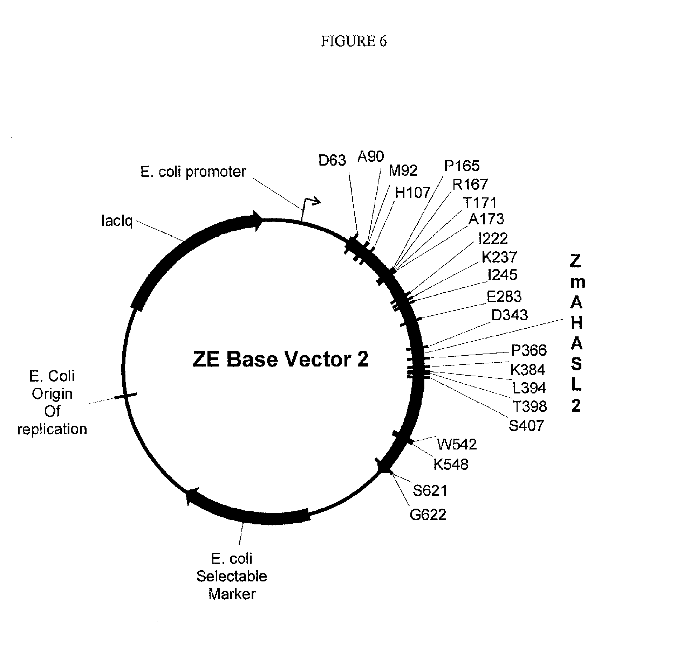

Figure 6 is a map of base vector ZE used to study maize AHASL mutants ZE2, ZE5, ZE6, and ZE7 in *E. coli,* with relative positions of mutations indicated.
Figure 7 is a map of plant transformation vector ZP used as a base vector for construction of vectors ZP2-ZP10.
Figure 8 is a table showing the concordant amino acid positions of AHASL genes derived from different species.

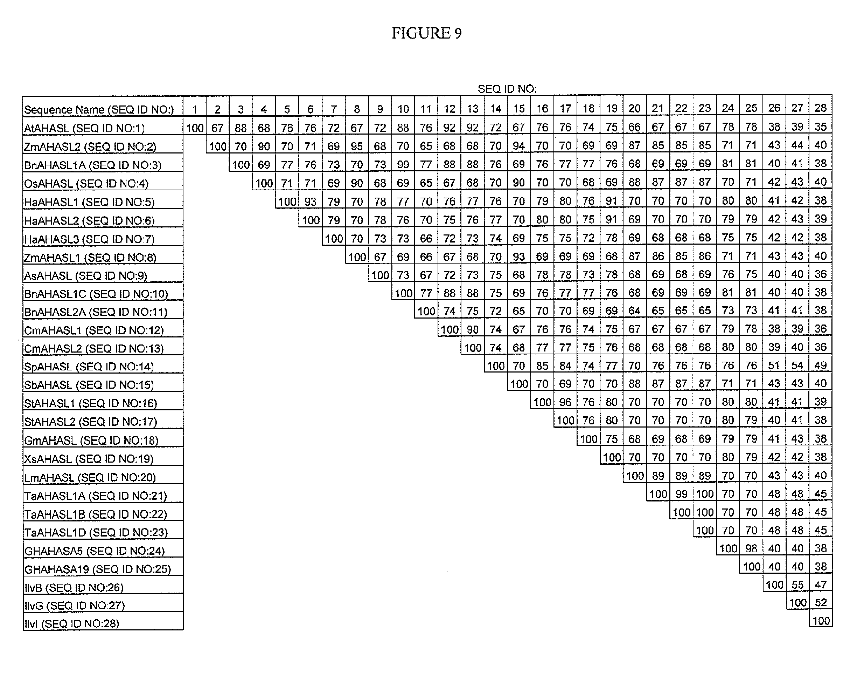

Figure 9 is a table showing the protein identity percentage of the AHASL genes derived from different species. The analysis was performed in Vector NTI software suite (gap opening penalty = 10, gap extension penalty = 0.05, gap separation penalty = 8, blosum 62MT2 matrix).
Figure 10 sets forth the results of a vertical plate growth assay of seeds from several lines of *Arabidopsis* plated on media with 37.5 micromolar of imazethapyr. The seeds used were: 1) wild type ecotype Columbia 2; 2) the csr1-2 mutant (homozygous for the AtAHASL S653N mutation in the genomic copy of the AHAS large subunit gene); 3) Columbia 2 transformed with AP1; 4) Columbia 2 transformed with AP7; and 5) Columbia 2 transformed with AP2.
Figure 11 is a vector map of plant transformation base vector AUP used for construction of vectors AUP2 and AUP, which differ only by the mutations indicated in Table 3.
Figure 12 is a vector map of plant transformation vector BAP 1, which comprises the coding sequence for an AtAHASL with the S653N mutation.

### DETAILED DESCRIPTION OF THE INVENTION

The invention provides polynucleotides encoding AHASL with at least two mutations, for example double and triple mutants, that demonstrate tolerance to herbicides, in particular, to imidazolinone herbicides and optionally, to sulfonylurea, triazolopyrimidine sulfoanilide, and/or pyrimidyl oxybenzoate herbicides. The AHASL mutants of the invention may be used to create transgenic plants that demonstrate levels of herbicide resistance sufficient to confer commercial levels of herbicide tolerance when present on only one parent of a hybrid cross or on one genome of a polyploid plant. The polynucleotides of the invention may also be used as selectable markers for transformation of linked genes encoding other traits, as set forth in U.S. Patent No. 6,025,541.

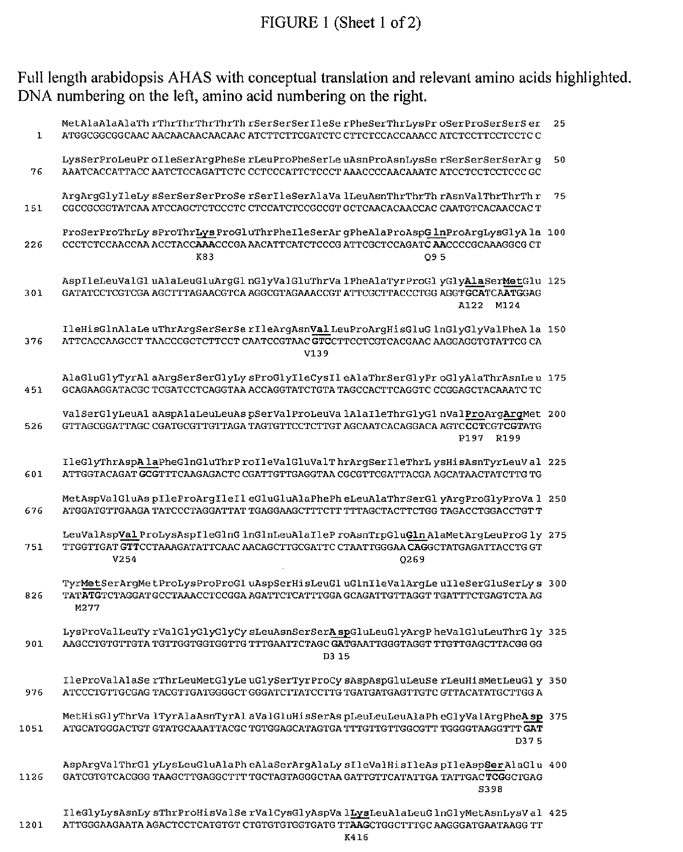

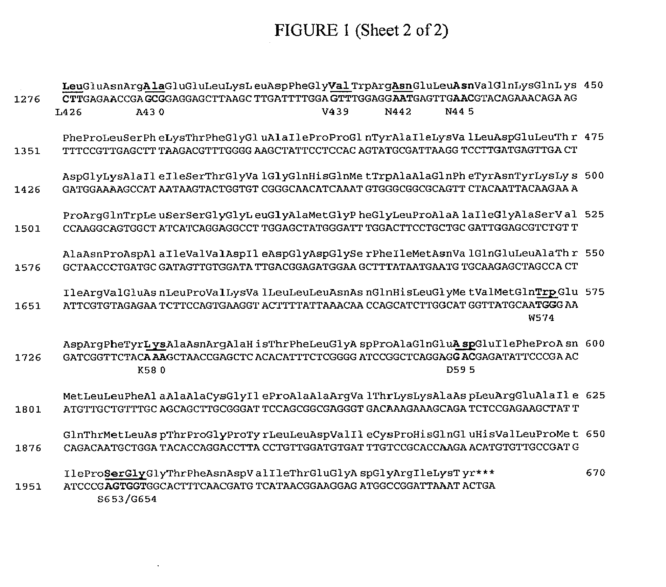

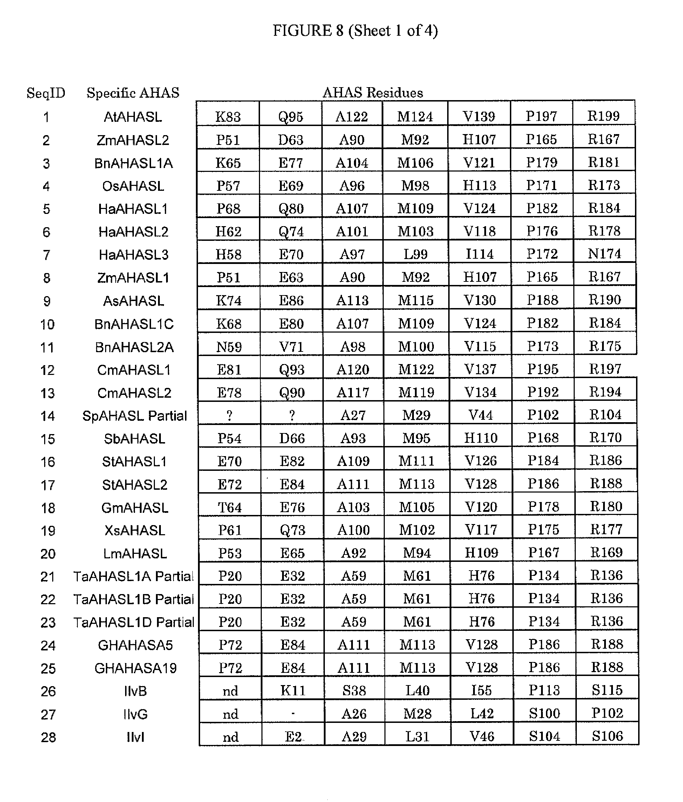

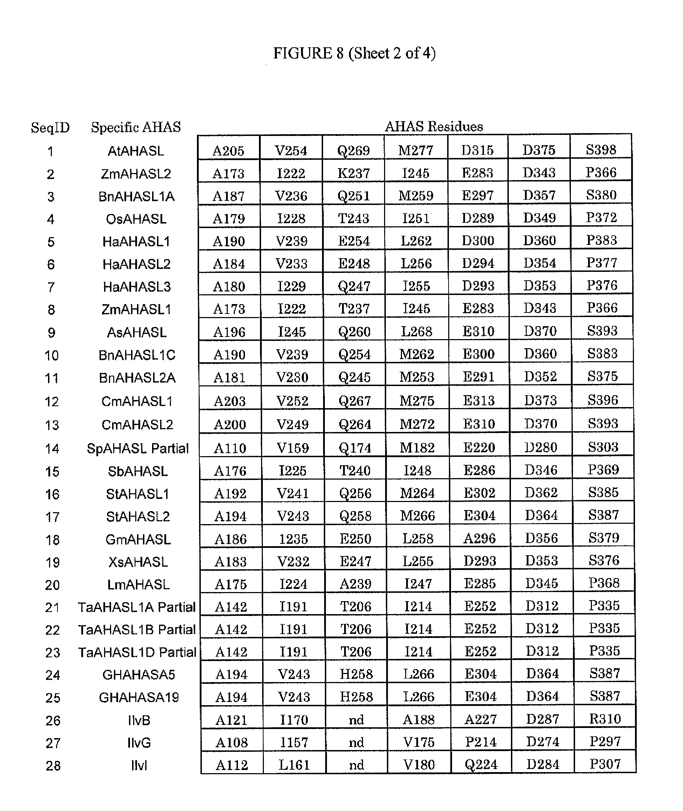

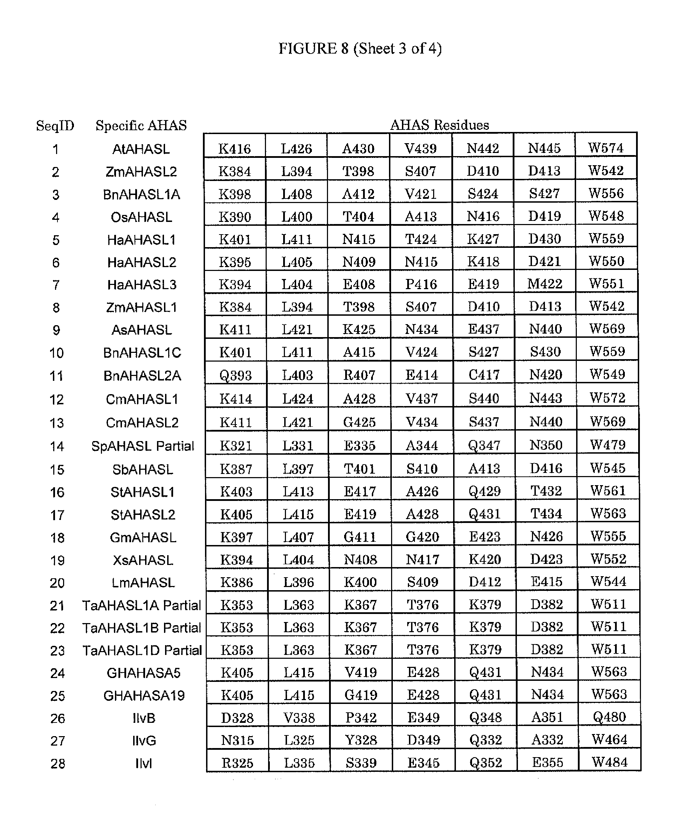

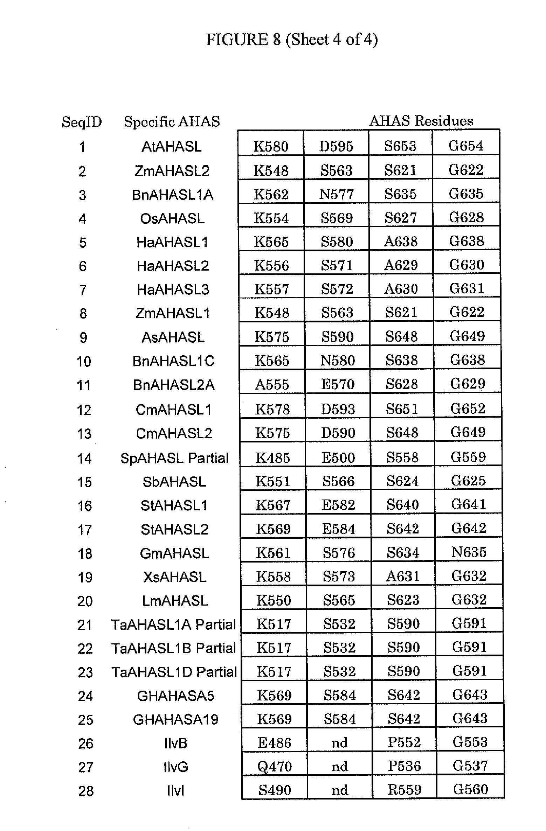

Although the AHASL proteins of various species differ in length by a few amino acids, the relative positions of residues subject to modification in accordance with the present invention are conserved (Figure 8). Accordingly, the mutations described herein are expressed in terms of positions corresponding to the amino acid residue numbers of the *Arabidopsis* AHASL polypeptide (SEQ ID NO: 1, Figure 1, Figure 8) unless noted otherwise or apparent from the context. For example, residue 122 of the *Arabidopsis* AHASL corresponds to residue 90 of maize AHASL, residue 104 of *Brassica napus* AHASL 1A residue 107 of *B. napus* AHASL 1C, residue 96 of *O*. *sativa* AHASL, residue 113 of *Amaranthus* AHASL, residue 26 of *Escherichia coli* ilvG, residue 117 of *Saccharomyces cerevisiae* AHASL, residue 113 of sugar beet, residue 111 of cotton, residue 120 of *Camelina microcarpa* AHASL1, residue 117 of *Camelina microcarpa* AHASL2, residue 109 of *Solanum tuberosum* AHASL1, residue 111 of *Solanum tuberosum* AHASL2, residue 92 of *Lolium multiflorum,* residue 27 of *Solanum ptychanthum,* residue 93 of *Sorghum bicolor,* residue 103 of *Glycine max,* residue 107 of *Helianthus annuus* AHASL1, residue 101 of *Helianthus annuus* AHASL2, residue 97 of *Helianthus annuus* AHASL3, residue 59 of *Triticum aestivum,* and residue 100 of *Xanthium* sp. These correspondences are well known to those of skill in the art. Based on such correspondence, the corresponding positions in AHAS large subunit sequences not specifically disclosed herein could be readily determined by the skilled artisan. Specific exemplary regions of correspondence relevant to the present invention are set forth in Figure 3.

In a preferred embodiment, the invention provides an isolated polynucleotide encoding an *Arabidopsis* AHASL double mutant selected from the group consisting of a polypeptide having a valine, threonine, glutamine, cysteine, or methionine at a position corresponding to position 122 of SEQ ID NO:1 or position 90 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a valine, threonine, glutamine, cysteine, or methionine at a position corresponding to position 122 of SEQ ID NO:1 or position 90 of SEQ ID NO:2 and a serine, alanine, glutamic acid, leucine, glutamine, arginine, valine, tryptophan, tyrosine, or isoleucine at a position corresponding to position 197 of SEQ ID NO:1 or position 165 of SEQ ID NO:2; a polypeptide having a valine, threonine, glutamine, cysteine, or methionine at a position corresponding to position 122 of SEQ ID NO:1 or position 90 of SEQ ID NO:2 and a glutamic acid, isoleucine, leucine, or asparagine at a position corresponding to position 124 of SEQ ID NO:1 or position 92 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 139 of SEQ ID NO:1 or position 107 of SEQ ID NO:2; a polypeptide having a valine, threonine, glutamine, cysteine, or methionine at a position corresponding to position 122 of SEQ ID NO:1 or position 90 of SEQ ID NO:2 and a histidine at a position corresponding to position 269 of SEQ ID NO:1 or position 237 of SEQ ID NO:2; a polypeptide having a valine, threonine, glutamine, cysteine, or methionine at a position corresponding to position 122 of SEQ ID NO:1 or position 90 of SEQ ID NO:2 and a methionine at a position corresponding to position 416 of SEQ ID NO:1 or position 384 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 426 of SEQ ID NO:1 or position 394 of SEQ ID NO:2; a polypeptide having a valine, threonine, glutamine, cysteine, or methionine at a position corresponding to position 122 of SEQ ID NO:1 or position 90 of SEQ ID NO:2 and a valine at a position corresponding to position 430 of SEQ ID NO:1 or position 398 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 442 of SEQ ID NO:1 or position 410 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine or aspartic acid at a position corresponding to position 445 of SEQ ID NO:1 or position 413 of SEQ ID NO:2; a polypeptide having a valine, threonine, glutamine, cysteine, or methionine at a position corresponding to position 122 of SEQ ID NO:1 or position 90 of SEQ ID NO:2 and a glutamic acid at a position corresponding to position 580 of SEQ ID NO:1 or position 548 of SEQ ID NO:2; a polypeptide having a glutamic acid, isoleucine, leucine, or asparagine at a position corresponding to position 124 of SEQ ID NO:1 or position 92 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an asparagine at a position corresponding to position 375 of SEQ ID NO:1 or position 343 of SEQ ID NO:2; a polypeptide having an alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2; and a polypeptide having a valine, cysteine, aspartic acid, glutamic acid, arginine, threonine, tryptophan, or tyrosine at a position corresponding to position 205 of SEQ ID NO:1 or position 173 of SEQ ID NO:2 and a phenylalanine, asparagine, threonine, glycine, valine, or tryptophan at a position corresponding to position 653 of SEQ ID NO:1 or position 621 of SEQ ID NO:2.

In another preferred embodiment, the invention provides an isolated polynucleotide encoding an *Arabidopsis* AHAS large subunit triple mutant polypeptide selected from the group consisting of: a polypeptide having a valine, threonine, glutamine, cysteine, or methionine at a position corresponding to position 122 of SEQ ID NO:1 or position 90 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a valine, threonine, glutamine, cysteine, or methionine at a position corresponding to position 122 of SEQ ID NO:1 or position 90 of SEQ ID NO:2, an arginine at a position corresponding to position 57 of SEQ ID NO:1 and a leucine at a position corresponding to position 398 of SEQ ID NO:1 or position 366 of SEQ ID NO:2; a polypeptide having a glutamic acid, isoleucine, leucine, or asparagine at a position corresponding to position 124 of SEQ ID NO:1 or position 92 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a leucine at a position corresponding to position 95 of SEQ ID NO:1 or position 63 of SEQ ID NO:2, a glutamic acid at a position corresponding to position 416 of SEQ ID NO:1 or position 384 of SEQ ID NO:2 and a phenylalanine, asparagine, threonine, glycine, valine, or tryptophan at a position corresponding to position 653 of SEQ ID NO:1 or position 621 of SEQ ID NO:2; and a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and any amino acid at a position corresponding to position 574 of SEQ ID NO:1 or position 542 of SEQ ID NO:2.

Other preferred embodiments include AHASL double and triple mutants from other species, wherein the double and triple mutations occur at positions corresponding to those of the specific *Arabidopsis* and maize mutants described above and in table shown in Figure 8. For example, corresponding double and triple mutants of AHASL from microorganisms such as *E. coli, S. cerevisiae, Salmonella, Synichocystis;* and from plants such as wheat, rye, oat, triticale, rice, barley, sorghum, millet, sugar beet, sugarcane, soybean, peanut, cotton, rapeseed, canola, *Brassica* species, manihot, melon, squash, pepper, sunflower, tagetes, solanaceous plants, potato, sweet potato, tobacco, eggplant, tomato, *Vicia* species, pea, alfalfa, coffee, cacao are also within the scope of the present invention. Such double and triple mutants can be made using known methods, for example, *in vitro* using site-directed mutagenesis, or *in vivo* using targeted mutagenesis or similar techniques, as described in U.S. Patent Nos. 5,565,350; 5,731,181; 5756,325; 5,760,012; 5,795,972 and 5,871,984.

The polynucleotides of the invention are provided in expression cassettes for expression in the plant of interest. The cassette will include regulatory sequences operably linked to an AHASL polynucleotide sequence of the invention. The term "regulatory element" as used herein refers to a polynucleotide that is capable of regulating the transcription of an operably linked polynucleotide. It includes, but not limited to, promoters, enhancers, introns, 5' UTRs, and 3' UTRs. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

Such an expression cassette is provided with a plurality of restriction sites for insertion of the AHASL polynucleotide sequence to be under the transcriptional regulation of the regulatory regions. The expression cassette may additionally contain selectable marker genes.

The expression cassette will include in the 5'-3' direction of transcription, a transcriptional and translational initiation region (i.e., a promoter), an AHASL polynucleotide sequence of the invention, and a transcriptional and translational termination region (i.e., termination region) functional in plants. The promoter may be native or analogous, or foreign or heterologous, to the plant host and/or to the AHASL polynucleotide sequence of the invention. Additionally, the promoter may be the natural sequence or alternatively a synthetic sequence. Where the promoter is "foreign" or "heterologous" to the plant host, it is intended that the promoter is not found in the native plant into which the promoter is introduced. Where the promoter is "foreign" or "heterologous" to the AHASL polynucleotide sequence of the invention, it is intended that the promoter is not the native or naturally occurring promoter for the operably linked AHASL polynucleotide sequence of the invention. As used herein, a chimeric gene comprises a coding sequence operably linked to a transcription initiation region that is heterologous to the coding sequence.

While it may be preferable to express the AHASL polynucleotides of the invention using heterologous promoters, the native promoter sequences may be used. Such constructs would change expression levels of the AHASL protein in the plant or plant cell. Thus, the phenotype of the plant or plant cell is altered.

The termination region may be native with the transcriptional initiation region, may be native with the operably linked AHASL sequence of interest, may be native with the plant host, or may be derived from another source (i.e., foreign or heterologous to the promoter, the AHASL polynucleotide sequence of interest, the plant host, or any combination thereof). Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed plant. That is, the genes can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498, herein incorporated by reference.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

Nucleotide sequences for enhancing gene expression can also be used in the plant expression vectors. These include the introns of the maize Adhl, intronl gene (Callis et al. Genes and Development 1:1183-1200, 1987), and leader sequences, (W-sequence) from the Tobacco Mosaic virus (TMV), Maize Chlorotic Mottle Virus and Alfalfa Mosaic Virus (Gallie et al. Nucleic Acid Res. 15:8693-8711, 1987 and Skuzeski et al. Plant Mol. Biol. 15:65-79, 1990). The first intron from the shrunken-1 locus of maize, has been shown to increase expression of genes in chimeric gene constructs. U.S. Pat. Nos. 5,424,412 and 5,593,874 disclose the use of specific introns in gene expression constructs, and Gallie et al. (Plant Physiol. 106:929-939, 1994) also have shown that introns are useful for regulating gene expression on a tissue specific basis. To further enhance or to optimize AHAS large subunit gene expression, the plant expression vectors of the invention may also contain DNA sequences containing matrix attachment regions (MARs). Plant cells transformed with such modified expression systems, then, may exhibit overexpression or constitutive expression of a nucleotide sequence of the invention.

The expression cassettes may additionally contain 5' leader sequences in the expression cassette construct. Such leader sequences can act to enhance translation. Translation leaders are known in the art and include: picornavirus leaders, for example, EMCV leader (Encephalomyocarditis 5' noncoding region) (Elroy-Stein et al. (1989) Proc. Natl. Acad. Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus) (Virology 154:9-20), and human immunoglobulin heavy-chain binding protein (BiP) (Macejak et al. (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol. 84:965-968. Other methods known to enhance translation can also be utilized, for example, introns, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

A number of promoters can be used in the practice of the invention. The promoters can be selected based on the desired outcome. The nucleic acids can be combined with constitutive, tissue-preferred, or other promoters for expression in plants.

Such constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

Tissue-preferred promoters can be utilized to target enhanced AHASL expression within a particular plant tissue. Such tissue-preferred promoters include, but are not limited to, leaf-preferred promoters, root-preferred promoters, seed-preferred promoters, and stem-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3): 1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression.

In one embodiment, the nucleic acids of interest are targeted to the chloroplast for expression. In this manner, where the nucleic acid of interest is not directly inserted into the chloroplast, the expression cassette will additionally contain a chloroplast-targeting sequence comprising a nucleotide sequence that encodes a chloroplast transit peptide to direct the gene product of interest to the chloroplasts. Such transit peptides are known in the art. With respect to chloroplast-targeting sequences, "operably linked" means that the nucleic acid sequence encoding a transit peptide (*i.e.,* the chloroplast-targeting sequence) is linked to the AHASL polynucleotide of the invention such that the two sequences are contiguous and in the same reading frame. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481. While the AHASL proteins of the invention include a native chloroplast transit peptide, any chloroplast transit peptide known in the art can be fused to the amino acid sequence of a mature AHASL protein of the invention by operably linking a choloroplast-targeting sequence to the 5'-end of a nucleotide sequence encoding a mature AHASL protein of the invention.

Chloroplast targeting sequences are known in the art and include the chloroplast small subunit of ribulose-1,5-bisphosphate carboxylase (Rubisco) (de Castro Silva Filho et al. (1996) Plant Mol. Biol. 30:769-780; Schnell et al. (1991) J. Biol. Chem. 266(5):3335-3342); 5-(enolpyruvyl)shikimate-3-phosphate synthase (EPSPS) (Archer et al. (1990) J. Bioenerg. Biomemb. 22(6):789-810); tryptophan synthase (Zhao et al. (1995) J. Biol. Chem. 270(11):6081-6087); plastocyanin (Lawrence et al. (1997) J. Biol. Chem. 272(33):20357-20363); chorismate synthase (Schmidt et al. (1993) J. Biol. Chem. 268(36):27447-27457); and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831, herein incorporated by reference.

In particular, the present invention describes using polynucleotides encoding AHASL mutant polypeptides comprising at least two mutations to engineer plants which are herbicide tolerant. This strategy has herein been demonstrated using *Arabidopsis* AHASL mutants in *Arabidopsis thaliana* and maize AHASL2 mutants in corn, but its application is not restricted to these genes or to these plants. In preferred embodiments, the herbicide is imidazolinone and/or sulfonylurea. In other preferred embodiments, the herbicide tolerance is improved and/or enhanced compared to wild-type plants and to known AHAS mutants.

The invention also provides a method of producing a transgenic crop plant containing AHASL mutant coding nucleic acid comprising at least two mutations, wherein expression of the nucleic acid(s) in the plant results in herbicide tolerance as compared to wild-type plants or to known AHAS mutant type plants comprising: (a) introducing into a plant cell an expression vector comprising nucleic acid encoding an AHASL mutant with at least two mutations, and (b) generating from the plant cell a transgenic plant which is herbicide tolerant. The plant cell includes, but is not limited to, a protoplast, gamete producing cell, and a cell that regenerates into a whole plant. As used herein, the term "transgenic" refers to any plant, plant cell, callus, plant tissue, or plant part that contains all or part of at least one recombinant polynucleotide. In many cases, all or part of the recombinant polynucleotide is stably integrated into a chromosome or stable extra-chromosomal element, so that it is passed on to successive generations.

In another embodiment, the invention relates to using the mutant AHASL polypeptides of the invention as selectable markers. The invention provides a method of identifying or selecting a transformed plant cell, plant tissue, plant or part thereof comprising a) providing a transformed plant cell, plant tissue, plant or part thereof, wherein said transformed plant cell, plant tissue, plant or part thereof comprises an isolated nucleic acid encoding an AHAS large subunit double mutant polypeptide of the invention as described above, wherein the polypeptide is used as a selection marker, and wherein said transformed plant cell, plant tissue, plant or part thereof may optionally comprise a further isolated nucleic acid of interest; b) contacting the transformed plant cell, plant tissue, plant or part thereof with at least one AHAS inhibitor or AHAS inhibiting compound; c) determining whether the plant cell, plant tissue, plant or part thereof is affected by the inhibitor or inhibiting compound; and d) identifying or selecting the transformed plant cell, plant tissue, plant or part thereof.

The invention is also embodied in purified AHASL proteins that contain the double and triple mutations described herein, which are useful in molecular modeling studies to design further improvements to herbicide tolerance. Methods of protein purification are well known, and can be readily accomplished using commercially available products or specially designed methods, as set forth for example, in Protein Biotechnology, Walsh and Headon (Wiley, 1994).

The invention further provides non-transgenic and transgenic herbicide-tolerant plants comprising one polynucleotide encoding an AHASL double mutant polypeptide, or two polynucleotides encoding AHASL single mutant polypeptides. Non-transgenic plants generated therefrom can be produced by cross-pollinating a first plant with a second plant and allowing the pollen acceptor plant (can be either the first or second plant) to produce seed from this cross pollination. Seeds and progeny plants generated thereof can have the double mutations crossed onto one single allele or two alleles. The pollen-acceptor plant can be either the first or second plant. The first plant comprises a first polynucleotide encoding a first AHASL single mutant polypeptide. The second plant comprises a second polynucleotide encoding a second AHASL single mutant polypeptide. The first and second AHASL single mutant polypeptides comprise a different single amino acid substitution relative to a wild-type AHASL polypeptide. Seeds or progeny plants arising therefrom which comprise one polynucleotide encoding the AHASL double mutant polypeptide or two polynucleotides encoding the two AHASL single mutant polypeptides can be selected. The selected progeny plants display an unexpectedly higher level of tolerance to an AHAS-inhibiting herbicide, for example an imidazolinone herbicide or sulfonylurea herbicide, than is predicted from the combination of the two AHASL single mutant polypeptides in a single plant. The progeny plants display a synergy with respect to herbicide tolerance, whereby the level of herbicide tolerance in the progeny plants comprising the first and second mutations from the parent plants is greater than the herbicide tolerance of a plant comprising two copies of the first polynucleotide or two copies of the second polynucleotide.

When the first and second plants are homozygous for the first and second polynucleotides, respectively, each of the resulting progeny plants comprises one copy of each of the first and second polynucleotides and the selection step can be omitted. When at least one of the first and second plants is heterozygous, progeny plants comprising both polynucleotides can be selected, for example, by analyzing the DNA of progeny plants to identify progeny plants comprising both the first and second polynucleotides or by testing the progeny plants for increased herbicide tolerance. The progeny plants that comprise both the first and second polynucleotides display a level of herbicide tolerance that is greater than the herbicide tolerance of a plant comprising two copies of the first polypeptide or two copies of the second polypeptide.

In one embodiment, the plants of the invention comprise a first polynucleotide encoding a first AHASL single mutant polypeptide and a second polynucleotide encoding a second AHASL single mutant polypeptide, or an AHASL encoding polynucleotide comprising two nucleotide mutations that result in the amino acid mutations corresponding to the amino acid mutations of said first and said second AHASL single mutant polypeptides, wherein said first and said second AHASL single mutant polypeptides are selected from the group consisting of: a first polypeptide having a valine, threonine, glutamine, cysteine, or methionine at a position corresponding to position 122 of SEQ ID NO:1 or position 90 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a glutamic acid, isoleucine, leucine, or asparagine at a position corresponding to position 124 of SEQ ID NO:1 or position 92 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 139 of SEQ ID NO:1 or position 107 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a histidine at a position corresponding to position 269 of SEQ ID NO:1 or position 237 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a methionine at a position corresponding to position 416 of SEQ ID NO:1 or position 3 84 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 426 of SEQ ID NO:1 or position 394 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a valine at a position corresponding to position 430 of SEQ ID NO:1 or position 398 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 442 of SEQ ID NO:1 or position 410 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having an isoleucine or aspartic acid at a position corresponding to position 445 of SEQ ID NO:1 or position 413 of SEQ ID NO:2; a first polypeptide having a valine, threonine, glutamine, cysteine, or methionine at a position corresponding to position 122 of SEQ ID NO:1 or position 90 of SEQ ID NO:2 and a second polypeptide having a glutamic acid at a position corresponding to position 580 of SEQ ID NO:1 or position 548 of SEQ ID NO:2; a first glutamic acid, isoleucine, leucine, or asparagine at a position corresponding to position 124 of SEQ ID NO:1 or position 92 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2; a first polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2; a first polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having an asparagine at a position corresponding to position 375 of SEQ ID NO:1 or position 343 of SEQ ID NO:2; a first polypeptide having an alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2; a first polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2; and a first polypeptide having a valine, cysteine, aspartic acid, glutamic acid, arginine, threonine, tryptophan, or tyrosine at a position corresponding to position 205 of SEQ ID NO:1 or position 173 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2. Non-transgenic plants comprising the double mutations of AHASL polynucleotides can be produced by methods other than the cross pollination described above, such as, for example but not limited to, targeted *in vivo* mutagenesis as described in Kochevenko et al. (Plant Phys. 132:174-184, 2003). The double mutations can be localized on a single allele, or two alleles of a plant genome.

Another embodiment of the invention relates to a transgenic plant transformed with an expression vector comprising an isolated polynucleotide, wherein the isolated polynucleotide encodes an acetohydroxyacid synthase large subunit (AHASL) double mutant polypeptide selected from the group consisting of: a polypeptide having a valine, threonine, glutamine, cysteine, or methionine at a position corresponding to position 122 of SEQ ID NO:1 or position 90 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a valine, threonine, glutamine, cysteine, or methionine at a position corresponding to position 122 of SEQ ID NO:1 or position 90 of SEQ ID NO:2 and a serine, alanine, glutamic acid, leucine, glutamine, arginine, valine, tryptophan, tyrosine, or isoleucine at a position corresponding to position 197 of SEQ ID NO:1 or position 165 of SEQ ID NO:2; a polypeptide having a valine, threonine, glutamine, cysteine, or methionine at a position corresponding to position 122 of SEQ ID NO:1 or position 90 of SEQ ID NO:2 and a glutamic acid, isoleucine, leucine, or asparagine at a position corresponding to position 124 of SEQ ID NO:1 or position 92 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 139 of SEQ ID NO:1 or position 107 of SEQ ID NO:2; a polypeptide having a valine, threonine, glutamine, cysteine, or methionine at a position corresponding to position 122 of SEQ ID NO:1 or position 90 of SEQ ID NO:2 and a histidine at a position corresponding to position 269 of SEQ ID NO:1 or position 237 of SEQ ID NO:2; a polypeptide having a valine, threonine, glutamine, cysteine, or methionine at a position corresponding to position 122 of SEQ ID NO:1 or position 90 of SEQ ID NO:2 and a methionine at a position corresponding to position 416 of SEQ ID NO:1 or position 384 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 426 of SEQ ID NO:1 or position 394 of SEQ ID NO:2; a polypeptide having a valine, threonine, glutamine, cysteine, or methionine at a position corresponding to position 122 of SEQ ID NO:1 or position 90 of SEQ ID NO:2 and a valine at a position corresponding to position 430 of SEQ ID NO:1 or position 398 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine at a position corresponding to position 442 of SEQ ID NO:1 or position 410 of SEQ ID NO:2; a polypeptide having a valine, threonine, glutamine, cysteine, or methionine at a position corresponding to position 122 of SEQ ID NO:1 or position 90 of SEQ ID NO:2 and an isoleucine or aspartic acid at a position corresponding to position 445 of SEQ ID NO:1 or position 413 of SEQ ID NO:2; a polypeptide having a valine, threonine, glutamine, cysteine, or methionine at a position corresponding to position 122 of SEQ ID NO:1 or position 90 of SEQ ID NO:2 and a glutamic acid at a position corresponding to position 580 of SEQ ID NO:1 or position 548 of SEQ ID NO:2; a polypeptide having a glutamic acid, isoleucine, leucine, or asparagine at a position corresponding to position 124 of SEQ ID NO:1 or position 92 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an asparagine at a position corresponding to position 375 of SEQ ID NO:1 or position 343 of SEQ ID NO:2; a polypeptide having an alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2; and a polypeptide having a valine, cysteine, aspartic acid, glutamic acid, arginine, threonine, tryptophan, or tyrosine at a position corresponding to position 205 of SEQ ID NO:1 or position 173 of SEQ ID NO:2 and a phenylalanine, asparagine, threonine, glycine, valine, or tryptophan at a position corresponding to position 653 of SEQ ID NO:1 or position 621 of SEQ ID NO:2.

The invention further provides non-transgenic and transgenic herbicide-tolerant plants comprising one polynucleotide encoding an AHASL triple mutant polypeptide, or one or more AHASL encoding polynucleotides comprising three mutations. For the production of a non-transgenic plant with one or more polynucleotides comprising three mutations, a progeny plant comprising one or two polynucleotides comprising said first and said second mutations described above is cross pollinated with third plant that comprises a third polynucleotide encoding a third AHASL single mutant polypeptide. The third AHASL single mutant polypeptide comprises a different single amino acid substitution relative to a wild-type AHASL polypeptide than either the first or second AHASL single mutant polypeptides. Seeds or progeny plants that comprise one or more polynucleotides comprising the three mutations are selected as described above. The selected progeny plants comprise a level of herbicide tolerance that is greater than the additive effect of combining the three AHASL single mutant polypeptides in a single plant. Non-transgenic plants comprising the triple or multiple mutations of AHASL polynucleotides can be produced by methods other than the cross pollination described above, such as, for example but not limited to, targeted *in vivo* mutagenesis as described above. The multiple mutations can be localized on a single allele, or multiple alleles of a plant genome.

In one embodiment, plants of the invention comprise a first polynucleotide encoding a first AHASL single mutant polypeptide, a second polynucleotide encoding a second AHASL single mutant polypeptide, and a third polynucleotide encoding a third AHASL single mutant polypeptide. In another embodiment, plants of the invention comprise an AHASL encoding polynucleotide comprising three mutations, wherein the three nucleotide mutations result in the amino acid mutations corresponding to the mutations of said first, said second and said third AHASL single mutant polypeptides. In yet another embodiment, plants of the invention comprise an AHASL encoding polynucleotide comprising a single mutation and a polynucleotide comprising a double mutations, wherein the nucleotide mutations result in the amino acid mutations corresponding to the mutations of aforementioned first, second and third AHASL single mutant polypeptides, wherein said first, second, and third AHASL single mutant polypeptides are selected from the group consisting of: a polypeptide having a valine, threonine, glutamine, cysteine, or methionine at a position corresponding to position 122 of SEQ ID NO:1 or position 90 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a valine, threonine, glutamine, cysteine, or methionine at a position corresponding to position 122 of SEQ ID NO:1 or position 90 of SEQ ID NO:2, an arginine at a position corresponding to position 57 of SEQ ID NO:1 and a leucine at a position corresponding to position 398 of SEQ ID NO:1 or position 366 of SEQ ID NO:2; a polypeptide having a glutamic acid, isoleucine, leucine, or asparagine at a position corresponding to position 124 of SEQ ID NO:1 or position 92 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a leucine at a position corresponding to position 95 of SEQ ID NO:1 or position 63 of SEQ ID NO:2, a glutamic acid at a position corresponding to position 416 of SEQ ID NO:1 or position 3 84 of SEQ ID NO:2 and a phenylalanine, asparagine, threonine, glycine, valine, or tryptophan at a position corresponding to position 653 of SEQ ID NO:1 or position 621 of SEQ ID NO:2; and a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and any amino acid at a position corresponding to position 574 of SEQ ID NO:1 or position 542 of SEQ ID NO:2.

Alternatively, plants comprising one or more polynucleotides encoding AHASL single mutant polypeptides are produced by transforming a plant with two or more of such polynucleotides or transforming a first plant with a first polynucleotide encoding a first AHASL single mutant polypeptide and cross pollinating the first plant with a second plant comprising a second polynucleotide encoding a second AHASL single mutant polypeptide. The second plant comprises a second polynucleotide comprising second AHASL single mutant polypeptide that is endogenous or was introduced via transformation. The first and second AHASL single mutant polypeptides comprise a different single amino acid substitution relative to a wild-type AHASL polypeptide. As necessary, seeds or progeny plants comprising both the first and second polynucleotides are selected as described above.

Yet another embodiment of the invention relates to a transgenic plant transformed with an expression vector comprising an isolated polynucleotide, wherein the isolated polynucleotide encodes an acetohydroxyacid synthase large subunit (AHASL) triple mutant polypeptide selected from the group consisting of: a polypeptide having a valine, threonine, glutamine, cysteine, or methionine at a position corresponding to position 122 of SEQ ID NO:1 or position 90 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a valine, threonine, glutamine, cysteine, or methionine at a position corresponding to position 122 of SEQ ID NO:1 or position 90 of SEQ ID NO:2, an arginine at a position corresponding to position 57 of SEQ ID NO:1 and a leucine at a position corresponding to position 398 of SEQ ID NO:1 or position 366 of SEQ ID NO:2; a polypeptide having a glutamic acid, isoleucine, leucine, or asparagine at a position corresponding to position 124 of SEQ ID NO:1 or position 92 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2; a polypeptide having a leucine at a position corresponding to position 95 of SEQ ID NO:1 or position 63 of SEQ ID NO:2, a glutamic acid at a position corresponding to position 416 of SEQ ID NO:1 or position 3 84 of SEQ ID NO:2 and a phenylalanine, asparagine, threonine, glycine, valine, or tryptophan at a position corresponding to position 653 of SEQ ID NO:1 or position 621 of SEQ ID NO:2; and a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and any amino acid at a position corresponding to position 574 of SEQ ID NO:1 or position 542 of SEQ ID NO:2.

The present invention provides herbicide-tolerant or herbicide-resistant plants comprising a herbicide-tolerant or herbicide-resistant AHASL protein including, but not limited to, AHASL single mutant polypeptides and AHASL double and triple mutant polypeptides that are encoded by the polynucleotides of the present invention. By a "herbicide-tolerant" or "herbicide-resistant" plant, it is intended that a plant that is tolerant or resistant to at least one herbicide at a level that would normally kill, or inhibit the growth of, a normal or wild-type plant. By "herbicide-tolerant AHASL protein" or "herbicide-resistant AHASL protein", it is intended that such an AHASL protein displays higher AHAS activity, relative to the AHAS activity of a wild-type AHASL protein, when in the presence of at least one herbicide that is known to interfere with AHAS activity and at a concentration or level of the herbicide that is known to inhibit the AHAS activity of the wild-type AHASL protein. Furthermore, the AHAS activity of such a herbicide-tolerant or herbicide-resistant AHASL protein may be referred to herein as "herbicide-tolerant" or "herbicide-resistant" AHAS activity.

For the present invention, the terms "herbicide-tolerant" and "herbicide-resistant" are used interchangeable and are intended to have an equivalent meaning and an equivalent scope. Similarly, the terms "herbicide-tolerance" and "herbicide-resistance" are used interchangeable and are intended to have an equivalent meaning and an equivalent scope. Likewise, the terms "imidazolinone-resistant" and "imidazolinone-resistance" are used interchangeable and are intended to be of an equivalent meaning and an equivalent scope as the terms "imidazolinone-tolerant" and "imidazolinone-tolerance", respectively.

The invention encompasses herbicide-resistant AHASL polynucleotides and herbicide-resistant AHASL proteins. By "herbicide-resistant AHASL polynucleotide" is intended a polynucleotide that encodes a protein comprising herbicide-resistant AHAS activity. By "herbicide-resistant AHASL protein" is intended a protein or polypeptide that comprises herbicide-resistant AHAS activity.

Further, it is recognized that a herbicide-tolerant or herbicide-resistant AHASL protein can be introduced into a plant by transforming a plant or ancestor thereof with a nucleotide sequence encoding a herbicide-tolerant or herbicide-resistant AHASL protein. Such herbicide-tolerant or herbicide-resistant AHASL proteins are encoded by the herbicide-tolerant or herbicide-resistant AHASL polynucleotides. Alternatively, a herbicide-tolerant or herbicide-resistant AHASL protein such as, for example, an AHASL single mutation polypeptide as disclosed herein, may occur in a plant as a result of a naturally occurring or induced mutation in an endogenous AHASL gene in the genome of a plant or progenitor thereof.

The present invention provides plants, plant tissues, plant cells, and host cells with increased resistance or tolerance to at least one herbicide, particularly an imidazolinone or sulfonylurea herbicide. The preferred amount or concentration of the herbicide is an "effective amount" or "effective concentration." By "effective amount" and "effective concentration" is intended an amount and concentration, respectively, that is sufficient to kill or inhibit the growth of a similar, wild-type, plant, plant tissue, plant cell, or host cell, but that said amount does not kill or inhibit as severely the growth of the herbicide-resistant plants, plant tissues, plant cells, and host cells of the present invention. Typically, the effective amount of a herbicide is an amount that is routinely used in agricultural production systems to kill weeds of interest. Such an amount is known to those of ordinary skill in the art.

By "similar, wild-type, plant, plant tissue, plant cell or host cell" is intended a plant, plant tissue, plant cell, or host cell, respectively, that lacks the herbicide-resistance characteristics and/or particular polynucleotide of the invention that are disclosed herein. The use of the term "wild-type" is not, therefore, intended to imply that a plant, plant tissue, plant cell, or other host cell lacks recombinant DNA in its genome, and/or does not possess herbicide-resistant characteristics that are different from those disclosed herein.

As used herein unless clearly indicated otherwise, the term "plant" intended to mean a plant at any developmental stage, as well as any part or parts of a plant that may be attached to or separate from a whole intact plant. Such parts of a plant include, but are not limited to, organs, tissues, and cells of a plant. Examples of particular plant parts include a stem, a leaf, a root, an inflorescence, a flower, a floret, a fruit, a pedicle, a peduncle, a stamen, an anther, a stigma, a style, an ovary, a petal, a sepal, a carpel, a root tip, a root cap, a root hair, a leaf hair, a seed hair, a pollen grain, a microspore, a cotyledon, a hypocotyl, an epicotyl, xylem, phloem, parenchyma, endosperm, a companion cell, a guard cell, and any other known organs, tissues, and cells of a plant. Furthermore, it is recognized that a seed is a plant.

The plants of the present invention include both non-transgenic plants and transgenic plants. By "non-transgenic plant" is intended to mean a plant lacking recombinant DNA in its genome. By "transgenic plant" is intended to mean a plant comprising recombinant DNA in its genome. Such a transgenic plant can be produced by introducing recombinant DNA into the genome of the plant. When such recombinant DNA is incorporated into the genome of the transgenic plant, progeny of the plant can also comprise the recombinant DNA. A progeny plant that comprises at least a portion of the recombinant DNA of at least one progenitor transgenic plant is also a transgenic plant.

In certain embodiments, the present invention involves herbidicide-resistant plants that are produced by mutation breeding. Such plants comprise a polynucleotide encoding an AHAS large subunit single mutant polypeptide and are tolerant to one or more AHAS-inhibiting herbicides. Such methods can involve, for example, exposing the plants or seeds to a mutagen, particularly a chemical mutagen such as, for example, ethyl methanesulfonate (EMS) and selecting for plants that have enhanced tolerance to at least one AHAS-inhibiting herbicide, particularly an imidazolinone herbicide or sulfonylurea herbicide. However, the present invention is not limited to herbicide-tolerant plants that are produced by a mutagenesis method involving the chemical mutagen EMS. Any mutagenesis method known in the art may be used to produce the herbicide-resistant plants of the present invention. Such mutagenesis methods can involve, for example, the use of any one or more of the following mutagens: radiation, such as X-rays, Gamma rays (e.g., cobalt 60 or cesium 137), neutrons, (e.g., product of nuclear fission by uranium 235 in an atomic reactor), Beta radiation (e.g., emitted from radioisotopes such as phosphorus 32 or carbon 14), and ultraviolet radiation (preferably from 2500 to 2900 nm), and chemical mutagens such as base analogues (e.g., 5-bromo-uracil), related compounds (e.g., 8-ethoxy caffeine), antibiotics (e.g., streptonigrin), alkylating agents (e.g., sulfur mustards, nitrogen mustards, epoxides, ethylenamines, sulfates, sulfonates, sulfones, lactones), azide, hydroxylamine, nitrous acid, or acridines. Herbicide-resistant plants can also be produced by using tissue culture methods to select for plant cells comprising herbicide-resistance mutations and then regenerating herbicide-resistant plants therefrom. See, for example, U.S. Patent Nos. 5,773,702 and 5,859,348, both of which are herein incorporated in their entirety by reference. Further details of mutation breeding can be found in "Principals of Cultivar Development" Fehr, 1993 Macmillan Publishing Company the disclosure of which is incorporated herein by reference.

The present invention provides methods for enhancing the tolerance or resistance of a plant, plant tissue, plant cell, or other host cell to at least one herbicide that interferes with the activity of the AHAS enzyme. Preferably, such a herbicide is an imidazolinone herbicide, a sulfonylurea herbicide, a triazolopyrimidine herbicide, a pyrimidinyloxybenzoate herbicide, a sulfonylamino-carbonyltriazolinone herbicide, or mixture thereof. More preferably, such a herbicide is an imidazolinone herbicide, a sulfonylurea herbicide, or mixture thereof. For the present invention, the imidazolinone herbicides include, but are not limited to, PURSUIT® (imazethapyr), CADRE® (imazapic), RAPTOR® (imazamox), SCEPTER® (imazaquin), ASSERT® (imazethabenz), ARSENAL® (imazapyr), a derivative of any of the aforementioned herbicides, and a mixture of two or more of the aforementioned herbicides, for example, imazapyr/imazamox (ODYSSEY®). More specifically, the imidazolinone herbicide can be selected from, but is not limited to, 2- (4-isopropyl-4-methyl-5-oxo-2-imidiazolin-2-yl) -nicotinic acid, [2- (4-isopropyl)-4-] [methyl-5-oxo-2-imidazolin-2-yl)-3-quinolinecarboxylic] acid, [5-ethyl-2- (4-isopropyl-] 4-methyl-5-oxo-2-imidazolin-2-yl) -nicotinic acid, 2- (4-isopropyl-4-methyl-5-oxo-2- imidazolin-2-yl)-5- (methoxymethyl)-nicotinic acid, [2- (4-isopropyl-4-methyl-5-oxo-2-] imidazolin-2-yl)-5-methylnicotinic acid, and a mixture of methyl [6- (4-isopropyl-4-] methyl-5-oxo-2-imidazolin-2-yl) -m-toluate and methyl [2- (4-isopropyl-4-methyl-5-] oxo-2-imidazolin-2-yl) -p-toluate. The use of 5-ethyl-2- (4-isopropyl-4-methyl-5-oxo- 2-imidazolin-2-yl) -nicotinic acid and [2- (4-isopropyl-4-methyl-5-oxo-2-imidazolin-2-] yl)-5- (methoxymethyl)-nicotinic acid is preferred. The use of [2- (4-isopropyl-4-] methyl-5-oxo-2-imidazolin-2-yl)-5- (methoxymethyl)-nicotinic acid is particularly preferred.

For the present invention, the sulfonylurea herbicides include, but are not limited to, chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfiuon, fluzasulfuron, imazosulfuron, pyrazosulfuron ethyl, halosulfuron, azimsulfuron, cyclosulfuron, ethoxysulfuron, flazasulfuron, flupyrsulfuron methyl, foramsulfuron, iodosulfuron, oxasulfuron, mesosulfuron, prosulfuron, sulfosulfuron, trifloxysulfuron, tritosulfuron, a derivative of any of the aforementioned herbicides, and a mixture of two or more of the aforementioned herbicides. The triazolopyrimidine herbicides of the invention include, but are not limited to, cloransulam, diclosulam, florasulam, flumetsulam, metosulam, and penoxsulam. The pyrimidinyloxybenzoate (or pyrimidinyl carboxy) herbicides of the invention include, but are not limited to, bispyribac, pyrithiobac, pyriminobac, pyribenzoxim and pyriftalid. The sulfonylamino-carbonyltriazolinone herbicides include, but are not limited to, flucarbazone and propoxycarbazone.

It is recognized that pyrimidinyloxybenzoate herbicides are closely related to the pyrimidinylthiobenzoate herbicides and are generalized under the heading of the latter name by the Weed Science Society of America. Accordingly, the herbicides of the present invention further include pyrimidinylthiobenzoate herbicides, including, but not limited to, the pyrimidinyloxybenzoate herbicides described above.

The present invention provides methods for enhancing AHAS activity in a plant comprising transforming a plant with a polynucleotide construct comprising a promoter operably linked to an AHASL nucleotide sequence of the invention. The methods involve introducing a polynucleotide construct of the invention into at least one plant cell and regenerating a transformed plant therefrom. The methods involve the use of a promoter that is capable of driving gene expression in a plant cell. Preferably, such a promoter is a constitutive promoter or a tissue-preferred promoter. The methods find use in enhancing or increasing the resistance of a plant to at least one herbicide that interferes with the catalytic activity of the AHAS enzyme, particularly an imidazolinone herbicide.

The present invention provides expression cassettes for expressing the polynucleotides of the invention in plants, plant tissues, plant cells, and other host cells. The expression cassettes comprise a promoter expressible in the plant, plant tissue, plant cell, or other host cells of interest operably linked to a polynucleotide of the invention that comprises a nucleotide sequence encoding either a full-length (*i.e.* including the chloroplast transit peptide) or mature AHASL protein (i.e. without the chloroplast transit peptide). If expression is desired in the plastids or chloroplasts of plants or plant cells, the expression cassette may also comprise an operably linked chloroplast-targeting sequence that encodes a chloroplast transit peptide.

The expression cassettes of the invention find use in a method for enhancing the herbicide tolerance of a plant or a host cell. The method involves transforming the plant or host cell with an expression cassette of the invention, wherein the expression cassette comprises a promoter that is expressible in the plant or host cell of interest and the promoter is operably linked to a polynucleotide of the invention that comprises a nucleotide sequence encoding an imidazolinone-resistant AHASL protein of the invention.

The use of the term "polynucleotide constructs" herein is not intended to limit the present invention to polynucleotide constructs comprising DNA. Those of ordinary skill in the art will recognize that polynucleotide constructs, particularly polynucleotides and oligonucleotides, comprised of ribonucleotides and combinations of ribonucleotides and deoxyribonucleotides may also be employed in the methods disclosed herein. Thus, the polynucleotide constructs of the present invention encompass all polynucleotide constructs that can be employed in the methods of the present invention for transforming plants including, but not limited to, those comprised of deoxyribonucleotides, ribonucleotides, and combinations thereof. Such deoxyribonucleotides and ribonucleotides include both naturally occurring molecules and synthetic analogues. The polynucleotide constructs of the invention also encompass all forms of polynucleotide constructs including, but not limited to, single-stranded forms, double-stranded forms, hairpins, stem-and-loop structures, and the like. Furthermore, it is understood by those of ordinary skill in the art that each nucleotide sequences disclosed herein also encompasses the complement of that exemplified nucleotide sequence.

Further, it is recognized that, for expression of a polynucleotide of the invention in a host cell of interest, the polynucleotide is typically operably linked to a promoter that is capable of driving gene expression in the host cell of interest. The methods of the invention for expressing the polynucleotides in host cells do not depend on particular promoter. The methods encompass the use of any promoter that is known in the art and that is capable of driving gene expression in the host cell of interest.

The present invention encompasses AHASL polynucleotide molecules and fragments and variants thereof. Polynucleotide molecules that are fragments of these nucleotide sequences are also encompassed by the present invention. By "fragment" is intended a portion of the nucleotide sequence encoding an AHASL protein of the invention. Preferably, a fragment of an AHASL nucleotide sequence of the invention encodes a biologically active portion of an AHASL protein. A biologically active portion of an AHASL protein can be prepared by isolating a portion of one of the AHASL nucleotide sequences of the invention, expressing the encoded portion of the AHASL protein (e.g., by recombinant expression *in vitro*), and assessing the activity of the encoded portion of the AHASL protein. Polynucleotide molecules that are fragments of an AHASL nucleotide sequence and encode biologically active portions of AHASL proteins comprise at least about 500, 750, 1000, 1250, 1500, 1600, 1700, 1800, 1900, or 2000 nucleotides, or up to the number of nucleotides present in a full-length nucleotide sequence disclosed herein (for example, 2013 nucleotides for SEQ ID NO:30) depending upon the intended use.

A fragment of an AHASL nucleotide sequence that encodes a biologically active portion of an AHASL protein of the invention will encode at least about 200, 300, 400, 500, 550, 650, or 650 contiguous amino acids, or up to the total number of amino acids present in a full-length AHASL protein of the invention (for example, 670 amino acids for SEQ ID NO: 1).

\ Polynucleotide molecules comprising nucleotide sequences that are variants of the nucleotide sequences disclosed herein are also encompassed by the present invention. "Variants" of the AHASL nucleotide sequences of the invention include those sequences that encode the mutant AHASL polypeptides disclosed herein but that differ conservatively because of the degeneracy of the genetic code. These naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the AHASL protein disclosed in the present invention as discussed below. Generally, polynucleotide sequence variants of the invention will have at least about 80%, 85%, 90%, 91%, 92%, 93%, 94%, 95%, 96%, 97%, 98%, or 99% identity to a particular nucleotide sequence disclosed herein. A variant AHASL polynucleotide sequence will encode an AHASL mutant polypeptide, respectively, that has an amino acid sequence having at least about 80%, 85%, 90%, 91%, 92%, 93%, 94%, 95%, 96%, 97%, 98%, or 99% identity to the amino acid sequence of an AHASL polypeptide disclosed herein.

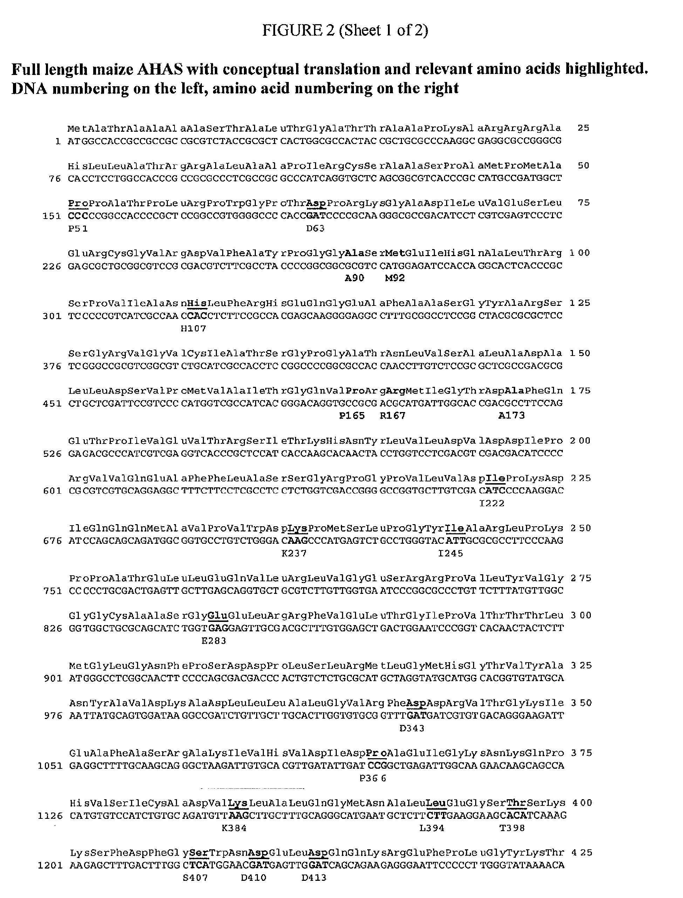

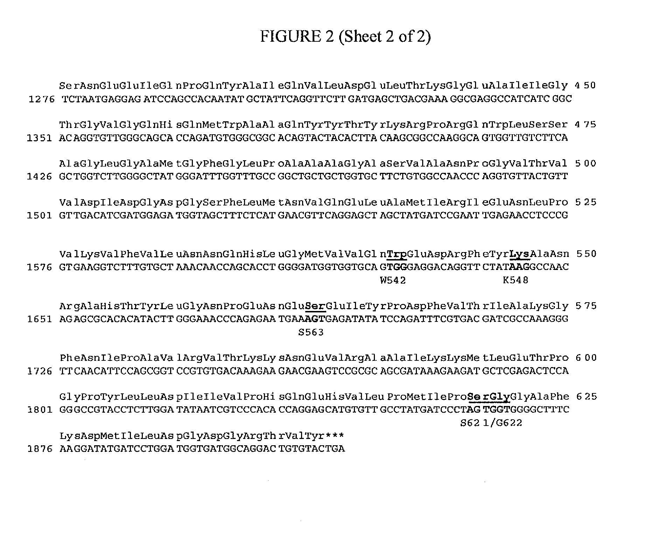

In addition, the skilled artisan will further appreciate that changes can be introduced by mutation into the polynucleotides sequences of the invention thereby leading to changes in the amino acid sequence of the encoded AHASL double and triple mutant polypeptides without altering the biological activity of the double and triple mutant polypeptides. Thus, an isolated polynucleotide molecule encoding an AHASL double and triple mutant polypeptide having a sequence that differs from the double and triple mutant sequences set forth in Figures 1 and 2 can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention.

For example, preferably, conservative amino acid substitutions may be made at one or more predicted, preferably nonessential amino acid residues. A "nonessential" amino acid residue is a residue that can be altered from the wild-type sequence of an AHASL protein (*e.g*., the sequence of SEQ ID NO: 1) without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e*.*g*., tyrosine, phenylalanine, tryptophan, histidine). Such substitutions would not be made for conserved amino acid residues, or for amino acid residues residing within a conserved motif.

The proteins of the invention may be altered in various ways including amino acid substitutions, deletions, truncations, and insertions. Methods for such manipulations are generally known in the art. For example, amino acid sequence variants of the AHASL proteins can be prepared by mutations in the DNA. Methods for mutagenesis and nucleotide sequence alterations are well known in the art. See, for example, Kunkel (1985) Proc. Natl. Acad. Sci. USA 82:488-492; Kunkel et al. (1987) Methods in Enzymol. 154:367-382; U.S. Patent No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.), herein incorporated by reference. Conservative substitutions, such as exchanging one amino acid with another having similar properties, may be preferable.

It is recognized that the polynucleotide molecules and polypeptides of the invention encompass polynucleotide molecules and polypeptides comprising a nucleotide or an amino acid sequence that is sufficiently identical to the double or triple nucleotide sequences set forth in Figures 1 and 2, or to the amino acid sequences set forth in Figures 1 and 2. The term "sufficiently identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity. For example, amino acid or nucleotide sequences that contain a common structural domain having at least about 80% identity, preferably 85% identity, more preferably 90%, 95%, or 98% identity are defined herein as sufficiently identical.

To determine the percent identity of two amino acid sequences or of two nucleic acids, the sequences are aligned for optimal comparison purposes. The percent identity between the two sequences is a function of the number of identical positions shared by the sequences (*i.e*., percent identity = number of identical positions/total number of positions (*e*.*g*., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A preferred, nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the NBLAST and XBLAST programs of Altschul et al. (1990) J. Mol. Biol. 215:403. BLAST nucleotide searches can be performed with the NBLAST program, score = 100, wordlength = 12, to obtain nucleotide sequences homologous to the polynucleotide molecules of the invention. BLAST protein searches can be performed with the XBLAST program, score = 50, wordlength = 3, to obtain amino acid sequences homologous to protein molecules of the invention. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. *See,* Altschul *et al.* (1997) *supra.* When utilizing BLAST, Gapped BLAST, and PSI-Blast programs, the default parameters of the respective programs (e.g., XBLAST and NBLAST) can be used. *See* http://www.ncbi.nlm.nih.gov. Another preferred, non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package. When utilizing the ALIGN program for comparing amino acid sequences, a PAM120 weight residue table, a gap length penalty of 12, and a gap penalty of 4 can be used. Alignment may also be performed manually by inspection.

Unless otherwise stated, sequence identity/similarity values provided herein refer to the value obtained using the full-length sequences of the invention and using multiple alignment by mean of the algorithm Clustal W (Nucleic Acid Research, 22(22):4673-4680, 1994) using the program AlignX included in the software package Vector NTI Suite Version 9 (Invitrogen, 1600 Faraday Ave., Carlsbad, CA 92008) using the default parameters; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by AlignX in the software package Vector NTI Suite Version 9.

The deletions, insertions, and substitutions of the protein sequences encompassed herein are not expected to produce radical changes in the characteristics of the protein. However, when it is difficult to predict the exact effect of the substitution, deletion, or insertion in advance of doing so, one skilled in the art will appreciate that the effect will be evaluated by routine screening assays. That is, the activity can be evaluated by AHAS activity assays. *See,* for example, Singh et al. (1988) Anal. Biochem. 171:173-179, herein incorporated by reference.

As disclosed herein, the polynucleotides of the invention find use in enhancing the herbicide tolerance of plants that comprise in their genomes a gene encoding a herbicide-tolerant AHASL protein. Such a gene may be an endogenous gene or a transgene. Additionally, in certain embodiments, the polynucleotides of the present invention can be stacked with any combination of polynucleotide sequences of interest in order to create plants with a desired phenotype. For example, the polynucleotides of the present invention may be stacked with any other polynucleotides encoding polypeptides having pesticidal and/or insecticidal activity, such as, for example, the *Bacillus thuringiensis* toxin proteins (described in U.S. Patent Nos. 5,366,892; 5,747,450; 5,737,514; 5,723,756; 5,593,881; and Geiser et al. (1986) Gene 48:109). The combinations generated can also include multiple copies of any one of the polynucleotides of interest.

While the polynucleotides of the invention find use as selectable marker genes for plant transformation, the expression cassettes of the invention can include another selectable marker gene for the selection of transformed cells. Selectable marker genes, including those of the present invention, are utilized for the selection of transformed cells or tissues. Marker genes include, but are not limited to, genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). See generally, Yarranton (1992) Curr. Opin. Biotech. 3:506-511; Christopherson et al. (1992) Proc. Natl. Acad. Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschle et al. (1989) Proc. Natl. Acad. Aci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 (Springer-Verlag, Berlin); Gill et al. (1988) Nature 334:721-724. Such disclosures are herein incorporated by reference.

The above list of selectable marker genes is not meant to be limiting. Any selectable marker gene can be used in the present invention.

The isolated polynucleotide molecules comprising nucleotide sequence that encode the AHASL proteins of the invention can be used in vectors to transform plants so that the plants created have enhanced resistant to herbicides, particularly an imidazolinone herbicide or sulfonylurea herbicide. The isolated AHASL polynucleotide molecules of the invention can be used in vectors alone or in combination with a nucleotide sequence encoding the small subunit of the AHAS (AHASS) enzyme in conferring herbicide resistance in plants. *See,* U.S. Patent No. 6,348,643; which is herein incorporated by reference.

The invention also relates to a plant expression vector comprising a promoter that drives expression in a plant operably linked to an isolated polynucleotide molecule of the invention. The isolated polynucleotide molecule comprises a nucleotide sequence encoding an AHASL protein of the invention, or a functional fragment and variant thereof. The plant expression vector of the invention does not depend on a particular promoter, only that such a promoter is capable of driving gene expression in a plant cell. Preferred promoters include constitutive promoters and tissue-preferred promoters.

The transformation vectors of the invention can be used to produce plants transformed with a gene of interest. The transformation vector will comprise a selectable marker gene of the invention and a gene of interest to be introduced and typically expressed in the transformed plant. Such a selectable marker gene comprises a polynucleotide of the invention that encodes an AHASL double or triple mutant polypeptide, wherein the polynucleotide is operably linked to a promoter that drives expression in a host cell. For use in plants and plant cells, the transformation vector comprises a selectable marker gene comprising a polynucleotide of the invention that encodes an AHASL double or triple mutant polypeptide operably linked to a promoter that drives expression in a plant cell.

The genes of interest of the invention vary depending on the desired outcome. For example, various changes in phenotype can be of interest including modifying the fatty acid composition in a plant, altering the amino acid content of a plant, altering a plant's insect and/or pathogen defense mechanisms, and the like. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in a change in phenotype of the transformed plant.

In one embodiment of the invention, the genes of interest include insect resistance genes such as, for example, *Bacillus thuringiensis* toxin protein genes (U.S. Patent Nos. 5,366,892; 5,747,450; 5,736,514; 5,723,756; 5,593,881; and Geiser et al. (1986) Gene 48:109).

The AHASL proteins or polypeptides of the invention can be purified from, for example, sunflower plants and can be used in compositions. Also, an isolated polynucleotide molecule encoding an AHASL protein of the invention can be used to express an AHASL protein of the invention in a microbe such as *E. coli* or a yeast. The expressed AHASL protein can be purified from extracts of *E. coli* or yeast by any method known to those of ordinary skill in the art.

The polynucleotides of the invention find use in methods for enhancing the resistance of herbicide-tolerant plants. In one embodiment of the invention, the herbicide-tolerant plants that comprise a polynucleotide of the invention that encodes an AHASL double or triple mutant polypeptide. The invention further provides herbicide-tolerant plants that comprise two or more polynucleotides encoding AHASL single mutant polypeptides. Polynucleotides encoding herbicide-tolerant AHASL proteins and herbicide-tolerant plants comprising an endogenous gene that encodes a herbicide-tolerant AHASL protein include the polynucleotides and plants of the present invention and those that are known in the art. *See,* for example, U.S. Patent Nos. 5,013,659, 5,731,180, 5,767,361, 5,545,822, 5,736,629, 5,773,703, 5,773,704, 5,952,553 and 6,274,796; all of which are herein incorporated by reference. Such methods for enhancing the resistance of herbicide-tolerant plants comprise transforming a herbicide-tolerant plant with at least one polynucleotide construct comprising a promoter that drives expression in a plant cell that is operably linked to a polynucleotide of the invention.

Numerous plant transformation vectors and methods for transforming plants are available. *See,* for example, An, G. et al. (1986) Plant Physiol., 81:301-305; Fry, J., et al. (1987) Plant Cell Rep. 6:321-325; Block, M. (1988) Theor. Appl Genet.76:767-774; Hinchee, et al. (1990) Stadler. Genet. Symp.203212.203-212; Cousins, et al. (1991) Aust. J. Plant Physiol. 18:481-494; Chee, P. P. and Slightom, J. L. (1992) Gene. 118:255-260; Christou, et al. (1992) Trends. Biotechnol. 10:239-246; D'Halluin, et al. (1992) Bio/Technol. 10:309-314; Dhir, et al. (1992) Plant Physiol. 99:81-88; Casas et al. (1993) Proc. Nat. Acad Sci. USA 90:11212-11216; Christou, P. (1993) In Vitro Cell. Dev. Biol.-Plant; 29P:119-124; Davies, et al. (1993) Plant Cell Rep. 12:180-183; Dong, J. A. and Mchughen, A. (1993) Plant Sci. 91:139-148; Franklin, C. I. and Trieu, T. N. (1993) Plant. Physiol. 102:167; Golovkin, et al. (1993) Plant Sci. 90:41-52; Guo Chin Sci. Bull. 38:2072-2078; Asano, et al. (1994) Plant Cell Rep. 13; Ayeres N. M. and Park, W. D. (1994) Crit. Rev. Plant. Sci. 13:219-239; Barcelo, et al. (1994) Plant. J. 5:583-592; Becker, et al. (1994) Plant. J. 5:299-307; Borkowska et al. (1994) Acta. Physiol Plant. 16:225-230; Christou, P. (1994) Agro. Food. Ind. Hi Tech. 5: 17-27; Eapen et al. (1994) Plant Cell Rep. 13:582-586; Hartman, et al. (1994) Bio-Technology 12: 919923; Ritala, et al. (1994) Plant. Mol. Biol. 24:317-325; and Wan, Y. C. and Lemaux, P. G. (1994) Plant Physiol. 104:3748*.*

The methods of the invention involve introducing a polynucleotide construct into a plant. By "introducing" is intended presenting to the plant the polynucleotide construct in such a manner that the construct gains access to the interior of a cell of the plant. The methods of the invention do not depend on a particular method for introducing a polynucleotide construct to a plant, only that the polynucleotide construct gains access to the interior of at least one cell of the plant. Methods for introducing polynucleotide constructs into plants are known in the art including, but not limited to, stable transformation methods, transient transformation methods, and virus-mediated methods.

By "stable transformation" is intended that the polynucleotide construct introduced into a plant integrates into the genome of the plant and is capable of being inherited by progeny thereof. By "transient transformation" is intended that a polynucleotide construct introduced into a plant does not integrate into the genome of the plant.

For the transformation of plants and plant cells, the nucleotide sequences of the invention are inserted using standard techniques into any vector known in the art that is suitable for expression of the nucleotide sequences in a plant or plant cell. The selection of the vector depends on the preferred transformation technique and the target plant species to be transformed. In an embodiment of the invention, an AHASL nucleotide sequence is operably linked to a plant promoter that is known for high-level expression in a plant cell, and this construct is then introduced into a plant that is susceptible to an imidazolinone or sulfonylurea herbicide and a transformed plant is regenerated. The transformed plant is tolerant to exposure to a level of an imidazolinone or sulfonylurea herbicide that would kill or significantly injure an untransformed plant. This method can be applied to any plant species; however, it is most beneficial when applied to crop plants.

Methodologies for constructing plant expression cassettes and introducing foreign nucleic acids into plants are generally known in the art and have been previously described. For example, foreign DNA can be introduced into plants, using tumor-inducing (Ti) plasmid vectors. Agrobacterium based transformation techniques are well known in the art. The Agrobacterium strain (e.g., *Agrobacterium tumefaciens* or *Agrobacterium rhizogenes)* comprises a plasmid (Ti or Ri plasmid) and a T-DNA element which is transferred to the plant following infection with Agrobacterium. The T-DNA (transferred DNA) is integrated into the genome of the plant cell. The T-DNA may be localized on the Ri- or Ti-plasmid or is separately comprised in a so-called binary vector. Methods for the Agrobacterium-mediated transformation are described, for example, in Horsch RB et al. (1985) Science 225:1229f. The Agrobacterium-mediated transformation can be used in both dicotyledonous plants and monocotyledonous plants. The transformation of plants by *Agrobacteria* is described in White FF, Vectors for Gene Transfer in Higher Plants; Vol. 1, Engineering and Utilization, edited by S.D. Kung and R. Wu, Academic Press, 1993, pp. 15 - 38; Jenes B et al. (1993) Techniques for Gene Transfer, in: Transgenic Plants, Vol. 1, Engineering and Utilization, edited by S.D. Kung and R. Wu, Academic Press, pp. 128-143; Potrykus (1991) Annu Rev Plant Physiol Plant Molec Biol 42:205- 225. Other methods utilized for foreign DNA delivery involve the use of PEG mediated protoplast transformation, electroporation, microinjection whiskers, and biolistics or microprojectile bombardment for direct DNA uptake. Such methods are known in the art. (U.S. Pat. No. 5,405,765 to Vasil et al.*;* Bilang et al. (1991) Gene 100: 247-250; Scheid et al. (1991) Mol. Gen. Genet., 228: 104-112; Guerche et al. (1987) Plant Science 52: 111-116; Neuhause et al. (1987) Theor. Appl Genet. 75: 30-36; Klein et al. (1987) Nature 327: 70-73; Howell et al. (1980) Science 208:1265; Horsch et al. (1985) Science 227: 1229-1231; DeBlock et al. (1989) Plant Physiology 91: 694-701; Methods for Plant Molecular Biology (Weissbach and Weissbach, eds.) Academic Press, Inc. (1988) and Methods in Plant Molecular Biology (Schuler and Zielinski, eds.) Academic Press, Inc. (1989). The method of transformation depends upon the plant cell to be transformed, stability of vectors used, expression level of gene products and other parameters.

Other suitable methods of introducing nucleotide sequences into plant cells and subsequent insertion into the plant genome include microinjection as Crossway et al. (1986) Biotechniques 4:320-334, electroporation as described by Riggs et al. (1986) Proc. Natl. Acad. Sci. USA 83:5602-5606, *Agrobacterium-*mediated transformation as described by Townsend *et al.* U.S. Patent No. 5,563,055, Zhao et al. U.S. Patent No. 5,981,840, direct gene transfer as described by Paszkowski et al. (1984) EMBO J. 3:2717-2722, and ballistic particle acceleration as described in, for example, Sanford et al. U.S. Patent No. 4,945,050; Tomes et al. U.S. Patent No. 5,879,918; Tomes et al. U.S. Patent No. 5,886,244; Bidney et al. U.S. Patent No. 5,932,782; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); McCabe et al. (1988) Biotechnology 6:923-926); and Lec1 transformation (WO 00/28058). *Also see,* Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); Tomes, U.S. Patent No. 5,240,855; Buising et al. U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin) (maize); Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; Bowen et al. U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osj oda et al. (1996) Nature Biotechnology 14:745-750 (maize via *Agrobacterium tumefaciens*); all of which are herein incorporated by reference.

The polynucleotides of the invention may be introduced into plants by contacting plants with a virus or viral nucleic acids. Generally, such methods involve incorporating a polynucleotide construct of the invention within a viral DNA or RNA molecule. It is recognized that the AHASL protein of the invention may be initially synthesized as part of a viral polyprotein, which later may be processed by proteolysis *in vivo* or *in vitro* to produce the desired recombinant protein. Further, it is recognized that promoters of the invention also encompass promoters utilized for transcription by viral RNA polymerases. Methods for introducing polynucleotide constructs into plants and expressing a protein encoded therein, involving viral DNA or RNA molecules, are known in the art. See, for example, U.S. Patent Nos. 5,889,191, 5,889,190, 5,866,785, 5,589,367 and 5,316,931; herein incorporated by reference.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a polynucleotide construct of the invention, for example, an expression cassette of the invention, stably incorporated into their genome.

The present invention may be used for transformation of any plant species, including, but not limited to, monocots and dicots. Examples of plant species of interest include, but are not limited to, corn or maize (*Zea mays*), *Brassica sp.* (*e.g., B. napus, B. rapa, B. juncea),* particularly those *Brassica* species useful as sources of seed oil, alfalfa (*Medicago sativa*), rice (*Oryza sativa*), rye (*Secale cereale*)*,* sorghum (*Sorghum bicolor, Sorghum vulgare*)*,* millet (e.g., pearl millet (*Pennisetum glaucum*), proso millet (*Panicum miliaceum*), foxtail millet (*Setaria italica*), finger millet (*Eleusine coracana*)), sunflower (*Helianthus annuus*), safflower (*Carthamus tinctorius*), wheat (*Triticum aestivum, T. Turgidum ssp. durum*), soybean (*Glycine max*), tobacco (*Nicotiana tabacum),* potato (*Solanum tuberosum*), peanuts (*Arachis hypogaea*), cotton (*Gossypium barbadense, Gossypium hirsutum*), sweet potato (*Ipomoea batatus*)*,* cassava (*Manihot esculenta*), coffee (*Coffea spp*.), coconut (*Cocos nucifera*), pineapple (*Ananas comosus*)*,* citrus trees (*Citrus spp.*), cocoa (*Theobroma cacao*), tea (*Camellia sinensis*)*,* banana (*Musa* spp.), avocado (*Persia americana*), fig *(Ficus casica*), guava (*Psidium guajava*), mango (*Mangifera indica*), olive (*Olea europaea*), papaya (*Carica papaya*)*,* cashew (*Anacardium occidentale*)*,* macadamia (*Macadamia integrifolia*), almond (*Prunus amygdalus*), sugar beets (*Beta vulgaris*)*,* sugarcane (*Saccharum* spp.), oats, barley, vegetables, ornamentals, and conifers. Preferably, plants of the present invention are crop plants (for example, sunflower, *Brassica sp.,* cotton, sugar beet, soybean, peanut, alfalfa, safflower, tobacco, corn, rice, wheat, rye, barley triticale, sorghum, millet, etc.).

The plants of the invention are herbicide-resistant plants and thus, find use in methods for controlling weeds that involve the application of a herbicide. Thus, the present invention further provides a method for controlling weeds in the vicinity of a herbicide-resistant plant of the invention. The method comprises applying an effective amount of a herbicide to the weeds and to the herbicide-resistant plant, wherein the plant has increased resistance to at least one AHAS-inhibiting herbicide, particularly an imidazolinone or sulfonylurea herbicide, when compared to a wild-type plant. In such a method for controlling weeds, the herbicide-resistant plants of the invention are preferably crop plants, including, but not limited to, sunflower, alfalfa, *Brassica sp.,* soybean, cotton, safflower, peanut, tobacco, tomato, potato, wheat, rice, maize, sorghum, barley, rye, millet, and sorghum.

By providing plants having increased resistance to herbicides, particularly imidazolinone and sulfonylurea herbicides, a wide variety of formulations can be employed for protecting plants from weeds, so as to enhance plant growth and reduce competition for nutrients. A herbicide can be used by itself for pre-emergence, post-emergence, pre-planting and at planting control of weeds in areas surrounding the plants described herein or an imidazolinone herbicide formulation can be used that contains other additives. The herbicide can also be used as a seed treatment. Additives found in an imidazolinone or sulfonylurea herbicide formulation include other herbicides, detergents, adjuvants, spreading agents, sticking agents, stabilizing agents, or the like. The herbicide formulation can be a wet or dry preparation and can include, but is not limited to, flowable powders, emulsifiable concentrates and liquid concentrates. The herbicide and herbicide formulations can be applied in accordance with conventional methods, for example, by spraying, irrigation, dusting, or the like.

The present invention provides non-transgenic and transgenic plants and seeds with increased tolerance to at least one herbicide, particularly an AHAS-inhibiting herbicide, more particularly imidazolinone and sulfonylurea herbicides, most particularly imidazolinone herbicides. In preferred embodiment of the invention, the plants and seeds of the invention display a higher level of herbicide tolerance that similar plants that comprise only one AHASL single mutant polypeptide. Such plants and seeds of the invention find use in improved methods for controlling weeds that allow for the application of a herbicide to the weeds and to the herbicide-resistant plant at an effective amount that comprises a higher herbicidal concentration or rate than can be used with similar plants that comprise only one AHASL single mutant polypeptide. Accordingly, such improved methods provide superior weed control when compared to existing methods involving plants comprising only one AHASL single mutant polypeptide and the application of a lower herbicidal concentration or rate.

The present invention provides herbicide-resistant plants comprising polynucleotides encoding AHASL double or triple mutant polypeptides and herbicide-resistant plants comprising two or more polynucleotides encoding AHASL single mutant polypeptides. These herbicide-resistant plants of the present invention find use in methods for producing herbicide-resistant plants through conventional plant breeding involving sexual reproduction. The methods comprise crossing a first plant that is a herbicide-resistant plant of the invention to a second plant that is not resistant to the herbicide. The second plant can be any plant that is capable of producing viable progeny plants (i.e., seeds) when crossed with the first plant. Typically, but not necessarily, the first and second plants are of the same species. The methods can optionally involve selecting for progeny plants that comprise the polynucleotide encoding the AHASL mutant polypeptide or the two or more polynucleotides encoding AHASL single mutant polypeptides of the first plant. The methods of the invention can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant.

The herbicide-resistant plants of the invention that comprise polynucleotides encoding AHASL double or triple mutant polypeptides and herbicide-resistant plants comprising two or more polynucleotides encoding AHASL single mutant polypeptides also find use in methods for increasing the herbicide-resistance of a plant through conventional plant breeding involving sexual reproduction. The methods comprise crossing a first plant that is a herbicide-resistant plant of the invention to a second plant that may or may not be resistant to the same herbicide or herbicides as the first plant or may be resistant to different herbicide or herbicides than the first plant. The second plant can be any plant that is capable of producing viable progeny plants (i.e., seeds) when crossed with the first plant. Typically, but not necessarily, the first and second plants are of the same species. The methods can optionally involve selecting for progeny plants that comprise the polynucleotide encoding the AHASL mutant polypeptide or the two or more polynucleotides encoding AHASL single mutant polypeptides of the first plant and the herbicide resistance characteristics of the second plant. The progeny plants produced by this method of the present invention have increased resistance to a herbicide when compared to either the first or second plant or both. When the first and second plants are resistant to different herbicides, the progeny plants will have the combined herbicide tolerance characteristics of the first and second plants. The methods of the invention can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant.

The present invention also provides plants, plant organs, plant tissues, plant cells, seeds, and non-human host cells that are transformed with the at least one polynucleotide molecule, expression cassette, or transformation vector of the invention. Such transformed plants, plant organs, plant tissues, plant cells, seeds, and non-human host cells have enhanced tolerance or resistance to at least one herbicide, at levels of the herbicide that kill or inhibit the growth of an untransformed plant, plant tissue, plant cell, or non-human host cell, respectively. Preferably, the transformed plants, plant tissues, plant cells, and seeds of the invention are *Arabidopsis thaliana* and crop plants.

The present invention provides methods that involve the use of at least one AHAS-inhibiting herbicide selected from the group consisting of imidazolinone herbicides, sulfonylurea herbicides, triazolopyrimidine herbicides, pyrimidinyloxybenzoate herbicides, sulfonylamino-carbonyltriazolinone herbicides, and mixtures thereof. In these methods, the AHAS-inhibiting herbicide can be applied by any method known in the art including, but not limited to, seed treatment, soil treatment, and foliar treatment.

Prior to application, the AHAS-inhibiting herbicide can be converted into the customary formulations, for example solutions, emulsions, suspensions, dusts, powders, pastes and granules. The use form depends on the particular intended purpose; in each case, it should ensure a fine and even distribution of the compound according to the invention.

The formulations are prepared in a known manner (see e.g. for review US 3,060,084, EP-A 707 445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al. Weed Control Handbook, 8th Ed., Blackwell Scientific Publications, Oxford, 1989 and Mollet, H., Grubemann, A., Formulation technology, Wiley VCH Verlag GmbH, Weinheim (Germany), 2001, 2. D. A. Knowles, Chemistry and Technology of Agrochemical Formulations, Kluwer Academic Publishers, Dordrecht, 1998 (ISBN 0-7514-0443-8), for example by extending the active compound with auxiliaries suitable for the formulation of agrochemicals, such as solvents and/or carriers, if desired emulsifiers, surfactants and dispersants, preservatives, antifoaming agents, anti-freezing agents, for seed treatment formulation also optionally colorants and/or binders and/or gelling agents.

Examples of suitable solvents are water, aromatic solvents (for example Solvesso products, xylene), paraffins (for example mineral oil fractions), alcohols (for example methanol, butanol, pentanol, benzyl alcohol), ketones (for example cyclohexanone, gamma-butyrolactone), pyrrolidones (NMP, NOP), acetates (glycol diacetate), glycols, fatty acid dimethylamides, fatty acids and fatty acid esters. In principle, solvent mixtures may also be used.

Examples of suitable carriers are ground natural minerals (for example kaolins, clays, talc, chalk) and ground synthetic minerals (for example highly disperse silica, silicates).

Suitable emulsifiers are nonionic and anionic emulsifiers (for example polyoxyethylene fatty alcohol ethers, alkylsulfonates and arylsulfonates).

Examples of dispersants are lignin-sulfite waste liquors and methylcellulose.

Suitable surfactants used are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol ethylene oxide condensates, ethoxylated castor oil, polyoxyethylene alkyl ethers, ethoxylated polyoxypropylene, lauryl alcohol polyglycol ether acetal, sorbitol esters, lignosulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, highly polar solvents, for example dimethyl sulfoxide, N-methylpyrrolidone or water.

Also anti-freezing agents such as glycerin, ethylene glycol, propylene glycol and bactericides such as can be added to the formulation.

Suitable antifoaming agents are for example antifoaming agents based on silicon or magnesium stearate.

Suitable preservatives are, for example, dichlorophenol and benzylalcoholhemiformaldehyde.

Seed Treatment formulations may additionally comprise binders and optionally colorants.

Binders can be added to improve the adhesion of the active materials on the seeds after treatment. Suitable binders are block copolymers EO/PO surfactants but also polyvinylalcoholsl, polyvinylpyrrolidones, polyacrylates, polymethacrylates, polybutenes, polyisobutylenes, polystyrene, polyethyleneamines, polyethyleneamides, polyethyleneimines (Lupasol®, Polymin®), polyethers, polyurethans, polyvinylacetate, tylose and copolymers derived from these polymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

An example of a suitable gelling agent is carrageen (Satiagel®).

Powders, materials for spreading, and dustable products can be prepared by mixing or concomitantly grinding the active substances with a solid carrier.

Granules, for example coated granules, impregnated granules and homogeneous granules, can be prepared by binding the active compounds to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the AHAS-inhibiting herbicide. In this case, the AHAS-inhibiting herbicides are employed in a purity of from 90% to 100% by weight, preferably 95% to 100% by weight (according to NMR spectrum). For seed treatment purposes, respective formulations can be diluted 2-10 fold leading to concentrations in the ready to use preparations of 0.01 to 60% by weight active compound by weight, preferably 0.1 to 40% by weight.

The AHAS-inhibiting herbicide can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the AHAS-inhibiting herbicide according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. However, it is also possible to prepare concentrates composed of active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, preferably from 0.01 to 1% per weight.

The AHAS-inhibiting herbicide may also be used successfully in the ultra-low-volume process (ULV), it being possible to apply formulations comprising over 95% by weight of active compound, or even to apply the active compound without additives.

The following are examples of formulations:
1. Products for dilution with water for foliar applications. For seed treatment purposes, such products may be applied to the seed diluted or undiluted.
   A) Water-soluble concentrates (SL, LS)
      Ten parts by weight of the AHAS-inhibiting herbicide are dissolved in 90 parts by weight of water or a water-soluble solvent. As an alternative, wetters or other auxiliaries are added. The AHAS-inhibiting herbicide dissolves upon dilution with water, whereby a formulation with 10 % (w/w) of AHAS-inhibiting herbicide is obtained.
   B) Dispersible concentrates (DC)
      Twenty parts by weight of the AHAS-inhibiting herbicide are dissolved in 70 parts by weight of cyclohexanone with addition of 10 parts by weight of a dispersant, for example polyvinylpyrrolidone. Dilution with water gives a dispersion, whereby a formulation with 20% (w/w) of AHAS-inhibiting herbicide is obtained.
   C) Emulsifiable concentrates (EC)
      Fifteen parts by weight of the AHAS-inhibiting herbicide are dissolved in 7 parts by weight of xylene with addition of calcium dodecylbenzenesulfonate and castor oil ethoxylate (in each case 5 parts by weight). Dilution with water gives an emulsion, whereby a formulation with 15% (w/w) of AHAS-inhibiting herbicide is obtained.
   D) Emulsions (EW, EO, ES)
      Twenty-five parts by weight of the AHAS-inhibiting herbicide are dissolved in 35 parts by weight of xylene with addition of calcium dodecylbenzenesulfonate and castor oil ethoxylate (in each case 5 parts by weight). This mixture is introduced into 30 parts by weight of water by means of an emulsifier machine (e.g. Ultraturrax) and made into a homogeneous emulsion. Dilution with water gives an emulsion, whereby a formulation with 25% (w/w) of AHAS-inhibiting herbicide is obtained.
   E) Suspensions (SC, OD, FS)
      In an agitated ball mill, 20 parts by weight of the AHAS-inhibiting herbicide are comminuted with addition of 10 parts by weight of dispersants, wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained.
   F) Water-dispersible granules and water-soluble granules (WG, SG)
      Fifty parts by weight of the AHAS-inhibiting herbicide are ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the AHAS-inhibiting herbicide, whereby a formulation with 50% (w/w) of AHAS-inhibiting herbicide is obtained.
   G) Water-dispersible powders and water-soluble powders (WP, SP, SS, WS)
      Seventy-five parts by weight of the AHAS-inhibiting herbicide are ground in a rotor-stator mill with addition of 25 parts by weight of dispersants, wetters and silica gel. Dilution with water gives a stable dispersion or solution of the AHAS-inhibiting herbicide, whereby a formulation with 75% (w/w) of AHAS-inhibiting herbicide is obtained.
   H) Gel-Formulation (GF)
      In an agitated ball mill, 20 parts by weight of the AHAS-inhibiting herbicide are comminuted with addition of 10 parts by weight of dispersants, 1 part by weight of a gelling agent wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained. This gel formulation is suitable for us as a seed treatment.
2. Products to be applied undiluted for foliar applications. For seed treatment purposes, such products may be applied to the seed diluted.
   A) Dustable powders (DP, DS)
      Five parts by weight of the AHAS-inhibiting herbicide are ground finely and mixed intimately with 95 parts by weight of finely divided kaolin. This gives a dustable product having 5% (w/w) of AHAS-inhibiting herbicide.
   B) Granules (GR, FG, GG, MG)
      One-half part by weight of the AHAS-inhibiting herbicide is ground finely and associated with 95.5 parts by weight of carriers, whereby a formulation with 0.5% (w/w) of AHAS-inhibiting herbicide is obtained. Current methods are extrusion, spray-drying or the fluidized bed. This gives granules to be applied undiluted for foliar use.

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, or either directly on the seeds.

In a preferred embodiment a FS formulation is used for seed treatment. Typcially, an FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.
For seed treatment, seeds of the herbicide resistant plants according of the present invention are treated with herbicides, preferably herbicides selected from the group consisting of AHAS-inhibiting herbicides such as amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid, pyrithiobac, and mixtures thereof, or with a formulation comprising a AHAS-inhibiting herbicide.

The term seed treatment comprises all suitable seed treatment techniques known in the art, such as seed dressing, seed coating, seed dusting, seed soaking, and seed pelleting.

In accordance with one variant of the present invention, a further subject of the invention is a method of treating soil by the application, in particular into the seed drill: either of a granular formulation containing the AHAS-inhibiting herbicide as a composition/formulation (e.g.a granular formulation, with optionally one or more solid or liquid, agriculturally acceptable carriers and/or optionally with one or more agriculturally acceptable surfactants. This method is advantageously employed, for example, in seedbeds of cereals, maize, cotton, and sunflower.

The present invention also comprises seeds coated with or containing with a seed treatment formulation comprising at least one AHAS-inhibiting herbicide selected from the group consisting of amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid and pyrithiobac.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

The seed treatment application with the AHAS-inhibiting herbicide or with a formulation comprising the AHAS-inhibiting herbicide is carried out by spraying or dusting the seeds before sowing of the plants and before emergence of the plants.

In the treatment of seeds, the corresponding formulations are applied by treating the seeds with an effective amount of the AHAS-inhibiting herbicide or a formulation comprising the AHAS-inhibiting herbicide. Herein, the application rates are generally from 0.1 g to 10 kg of the a.i. (or of the mixture of a.i. or of the formulation) per 100 kg of seed, preferably from 1 g to 5 kg per 100 kg of seed, in particular from 1 g to 2.5 kg per 100 kg of seed. For specific crops such as lettuce the rate can be higher.

The present invention provides a method for combating undesired vegetation or controlling weeds comprising contacting the seeds of the resistant plants according to the present invention before sowing and/or after pregermination with an AHAS-inhibiting herbicide. The method can further comprise sowing the seeds, for example, in soil in a field or in a potting medium in greenhouse. The method finds particular use in combating undesired vegetation or controlling weeds in the immediate vicinity of the seed.

The control of undesired vegetation is understood as meaning the killing of weeds and/or otherwise retarding or inhibiting the normal growth of the weeds. Weeds, in the broadest sense, are understood as meaning all those plants which grow in locations where they are undesired.

The weeds of the present invention include, for example, dicotyledonous and monocotyledonous weeds. Dicotyledonous weeds include, but are not limited to, weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, and Taraxacum. Monocotyledonous weeds include, but are not limited to, weeds of of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, and Apera.

In addition, the weeds of the present invention can include, for example, crop plants that are growing in an undesired location. For example, a volunteer maize plant that is in a field that predominantly comprises soybean plants can be considered a weed, if the maize plant is undesired in the field of soybean plants.

The articles "a" and "an" are used herein to refer to one or more than one (i.e., to at least one) of the grammatical object of the article. By way of example, "an element" means one or more elements.

As used herein, the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation. Any variations in the exemplified methods that occur to the skilled artisan are intended to fall within the scope of the present invention.

### Example 1

### Vectors containing Arabidopsis AHASL mutant genes

The entire XbaI fragment of *Arabidopsis thaliana* genomic DNA that contains the entire AHAS large subunit gene with some additional DNA, inclusive of the XbaI sites at the 5' and 3' ends is set forth in SEQ ID NO: 34 (AtAHASL). Bases 2484 to 4496 of SEQ ID NO: 34 encompass the coding sequence of the *Arabidopsis thaliana* AHAS large subunit gene serine 653 to threonine mutant allele, inclusive of the stop codon shown in SEQ ID NO: 30. A smaller genomic fragment of the *Arabidopsis thaliana* AHAS large subunit gene serine 653 to threonine mutant allele shown in SEQ ID NO: 33, encompassed in bases 2484 to 5717 of SEQ ID NO: 34, includes the coding sequence and the 3' end, up to and including the 3' end XbaI site, with the first two bases of the NcoI site found at the start codon of AtAHASL left off for clarity.

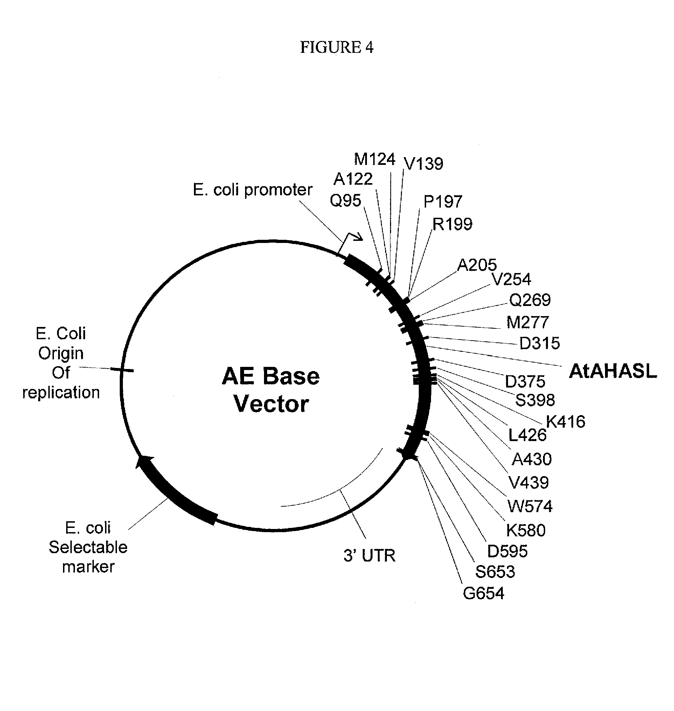

The DNA fragment of SEQ ID NO: 33 encoding the full-length *Arabidopsis* AHASL single mutation S653N and 3' untranslated region was cloned into pKK233-2 to yield the vector designated AE1 for expression and testing in *E. coli.* (pKK233-2, bacterial expression vector, Pharmacia, GenBank Accession No. X70478). Vectors AE2 through AE9 were generated from AE1 by mutagenesis and standard cloning procedures. Figure 4 shows the map of the AE1 base vector, with positions of mutations indicated.

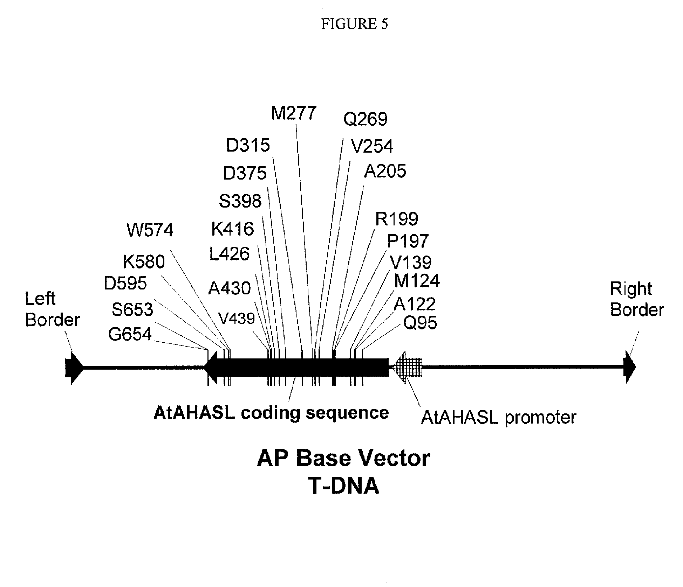

Vector AP1 (Figure 5) is a plant transformation vector that includes a genomic fragment of *Arabidopsis thaliana* DNA that includes the AtAHASL gene with the single S653N mutation (SEQ ID NO:34). The DNA fragment as shown in SEQ ID NO: 34 was cloned into AP1 in the reverse-complement orientation. Vectors AP2-AP7 were generated from AP1 and the AE plasmids using standard cloning procedures and differ only by mutations as indicated in Table 1. For convenience in cloning, different fragments were used to generate AP6 and AP7, compared to AP2-AP5. Thus, AP6 and AP7 are 47 base pairs shorter than AP1-AP5 . This difference is in the plasmid backbone and not the *Arabidopsis thaliana* genomic fragment.

Vectors AE10 through AE24 were made as follows. The wild type *Arabidopsis thaliana* AHAS large subunit gene was amplified under mutagenic conditions using the Genemorph II random mutagenesis kit (Stratagene, La Jolla, California), resulting in randomly mutagenized amplified DNA fragments of this gene. This mutant DNA was then cloned back into AE7, replacing the wild type A. *thaliana* large subunit gene (between the unique SacII and AgeI sites on AE7) with the mutagenized forms. This DNA was transformed into *E. coli* strain TOP10 and selected on LB agar medium in such a fashion as to have a large number of unique transformants, each with independent, mutagenized AHAS genes. These colonies were scraped together and plasmid DNA was prepared from this entire primary library. This DNA was transformed into AHAS minus *E. coli* and again selected on LB agar media with carbenicillin. Plasmid positive colonies from this step were replica plated using velveteen onto minimal agar medium without branched chain amino acids and containing 30-micromolar imazethapyr. Those colonies that grew on this selective media possessed a functional *A. thaliana* AHAS mutant gene that was also imidazolinone tolerant.

The DNA sequence of the *A. thaliana* AHAS large subunit gene was determined for each of the growth positive colonies. No effort was made to determine the sequence of the *A. thaliana* AHAS large subunit genes that did not confer growth on the selective media. Because the AHAS function and imidazolinone tolerance screen was on a secondary library, replicates of the same mutations were found, as determined by DNA sequence analysis. Only one clone of each was advanced for testing on increasing imidazolinone concentrations and inclusion in Table 1.

**Table 1**

| *E. coli* Plasmid^{*} | *Arabidopsis* Transformation Vector* | Mutations^{*} | *E. coli* Imazethapyr Tolerance Score^{**} | Transgenic Plant Tolerance: X-fold improvement over AP 1 ^{@}(approximate) |
|---|---|---|---|---|
| AE1 | AP1 | S653N | + | NA |
| AE2 | AP2 | A122T & S653N | ++ | 16 |
| AE3 | AP3 | P197S & S653N | + | 2 |
| AE4 | AP8 | A122T, R199A, & S653N | NA | 16 |
| AE5 | AP4 | R199A, & S653N | +++ | 1.5 |
| AE6 | AP5 | A122T, P197S, & S653N | NA | 8 |
| AE7 | | Wild type | - (IN) | NA |
| AE8 | AP6 | A122T and R199A | +++ | 2 |
| AE9 | AP7 | A122T and P197S | NA | 8 |
| AE10 | | A122T, S57R and S398L | +++ | NA |
| AE11 | | A122T and V139I | ++ | NA |
| AE12 | | A122T and Q269H | + | NA |
| AE13 | | A122T and K416M | ++ | NA |
| AE14 | | A122T and L426I | +++ | NA |
| AE15 | | A122T and A430V | +++ | NA |
| AE16 | | A122T and N442I | ++ | NA |
| AE17 | | A122T and N445I | ++ | NA |
| AE18 | | A122T and N445D | +++ | NA |
| AE19 | | A122T and K580E | +++ | NA |
| AE20 | | A122T, V439G, D595G, and S653N | + | NA |
| AE21 | | P197S and D375N | +++ | NA |
| AE22 | | D375N | untested^{†} | NA |
| AE23 | | D375N, K83R, V254I, M277I, and D315Y | + | NA |
| AE24 | | Q95L, K416E, and S653N | + | NA |

| | | | | |
|---|---|---|---|---|
| ^{*} List of vectors for expression of AtAHASL2 in *E. coli* (AE plasmids) and for plant transformation plasmids (AP plasmids). Mutations in each vector are indicated relative to SEQ ID NO: 1. ^{**} A simple single, double or triple plus system, +, ++, or +++ for respectively increasing colony size, was used to visually score the tolerance of the *Arabidopsis* AHAS function in AHAS minus *E. coli* containing the AE plasmids in the presence of the AHAS inhibitor imazethapyr. A "-", indicates there was no growth, meaning the mutation combination caused an inactive protein or there was no tolerance for imazethapyr at the selected rate. IN means inactive protein, while NT means not imidazolinone tolerant. NA means no data available (not tested). ^{†} Not tested compared to S653N, fact of tolerance determined by screening protocol. ^{@} For transgenic plants comprising the AP1 vector, 18.75 µM imazethapyr was the highest concentration which allowed good growth of the plants in the microtiter format plates. This concentration was used as the basis for determining X-fold improvement over AP1. | | | | |

### Example 2

### Vectors containing Zea mays AHASL mutant genes

The *Zea mays* AHASL2 gene (SEQ ID NO: 29) was cloned into the bacterial expression vector pTrcHis A (Invitrogen Corporation, Carlsbad, CA), fused to the vector tag and translational start site, beginning with base 160 of SEQ ID NO: 29. Mutagenesis and subcloning procedures were utilized to create vectors ZE2, ZE5, ZE6, and ZE7 using ZE1 as a base vector. Subcloning procedures were used to make ZE3 from ZE1, which is the maize AHASL2 gene fused to the vector tag and translational start site of pTrcHis A, beginning with base 121 of SEQ ID NO: 29. Since no functional difference was noted in *E. coli* between ZE1 or ZE3, standard mutagenesis and subcloning procedures were utilized to create vectors ZE4 and ZE8 through ZE22 using ZE3 as a base vector.

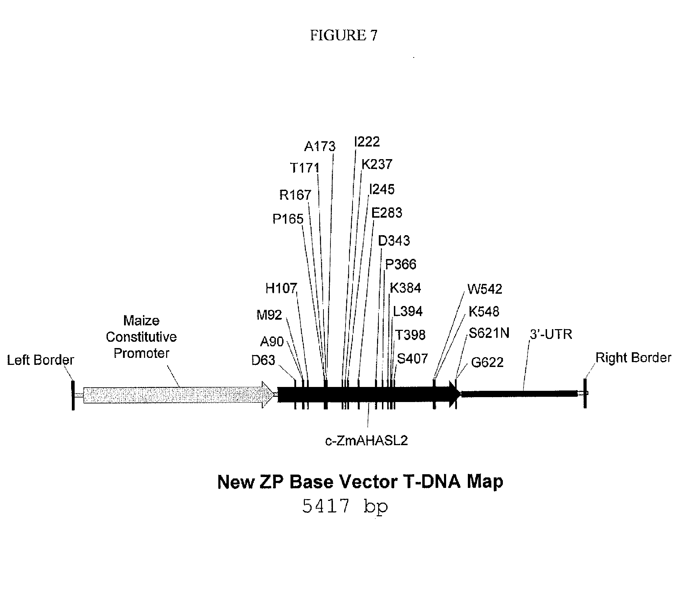

A plant transformation vector with an expression cassette comprising the maize ubiquitin promoter in combination with a polynucleotide encoding the maize AHASL2 S653N mutant was prepared using standard methods and designated ZP1 (Figure 7). To produce plant transformation vectors for expression of the other AHASL mutants, standard cloning techniques were used to replace polynucleotide segments of ZP1 with polynucleotide fragments of the ZE vectors encoding the mutations.

Vectors ZE23 through ZE38 were made as follows. Vector ZE3 was subjected to saturating site directed mutagenesis using the QuikChange® Multi Site Directed Mutagenesis Kit (Stratagene, La Jolla, California) following the "General Guidelines for Creating Engineered Mutant Clone™ Collections" appendix protocol. Mutagenic oligonucleotides that would generate all possible codons at the critical sites of the maize AHAS large subunit were used in various combinations to create a collection of mutants with substitutions at residues A90, M92, P165, R167, S621, and G622. The mutant collection plasmids were transformed into AHAS deficient *E. coli* and plated on LB agar medium supplemented with 100 ug per liter of carbenicillin. Colonies from this were picked into M9 salts at 1x concentration (for an isotonic buffer) in microtiter plates and then replica plated on minimal agar medium without branched chain amino acids and containing 150 micromolar imazethapyr. Those colonies that grew on this selective media possessed a functional maize AHAS mutant gene that was also imidazolinone tolerant.

The DNA sequence of the maize AHAS large subunit gene was determined for each of the growth positive colonies. No effort was made to determine the sequence of the maize AHAS large subunit genes that did not confer growth on the selective media.

**Table 2**

| *E. coli* Plasmid^{*} | Maize Transformation Vector^{*} | Mutations^{*} | *E. coli* Imidazolinone Tolerance Score^{**} | Maize Whole Plant Tolerance Score^{@} |
|---|---|---|---|---|
| ZE1 | - | S621N | + | NA |
| ZE2 | - | wild type | - (NT) | NA |
| ZE3 | - | wild type | - (NT) | NA |
| ZE4 | ZP1 | S621N | + | + |
| ZE5 | - | W542L, S621N | - (IN) | NA |
| ZE6 | ZP4 | P165S, S621N | + | + |
| ZE7 | - | W542L | - (NT) | NA |
| ZE8 | - | M92E, S621N | - (IN) | NA |
| ZE9 | ZP5 | R167S, S621N | +++ | +++ |
| ZE10 | ZP2 | A90T, S621N | +++ | +++ |
| ZE11 | ZP3 | A90T, R167S, S621N | +++ | +++ |
| ZE12 | ZP9 | M92I, S621N | +++ | +++ |
| ZE13 | - | R167A, S621N | NA | NA |
| ZE14 | - | A173V, S621N | ++ | NA |
| ZE15 | ZP8 | A90T, M92I | +++ | +++ |
| ZE16 | ZP10 | A90T, M92E | NA | NA |
| ZE17 | ZP6 | A90T, R167A | +++ | +++ |
| ZE18 | | P165S | - (NT) | NA |
| ZE19 | - | P165S, R167A | - (NT) | NA |
| ZE20 | - | T171I, S621N | + | NA |
| ZE21 | ZP7 | A90T | ++ | +++ |
| ZE22 | - | A90T, P165S | +++ | NA |
| ZE23 | ZP11 | A90Q | +++ | +++ |
| ZE24 | ZP12 | A90Q, M92L | ++ | +++ |
| ZE25 | - | A90Q, M92I | +++ | NA |
| ZE26 | - | A90C | ++ | NA |
| ZE27 | - | A90M, M92I | + | NA |
| ZE28 | - | P165E, R167F | - (NT) | NA |
| ZE29 | - | P165V, R167A | - (NT) | NA |
| ZE30 | - | P165E, R167T | + | + |
| ZE31 | - | P165I, R167D | - (IN) | NA |
| ZE32 | - | P165I, R167E | + | NA |
| ZE33 | - | M92I, P165E, R167A | - (NT) | NA |
| ZE34 | - | A90M, P165R, R167C | - (IN) | NA |
| ZE35 | - | M92N, S621G | +++ | NA |
| ZE36 | - | P165S, R167N, S621V, G622D | +++ | NA |
| ZE37 | - | S621W | +++ | NA |
| ZE38 | - | P165S, R167C, W542M | +++ | NA |

| | | | | |
|---|---|---|---|---|
| ^{*} List of vectors for expression of ZmAHASL2 in E. coli (ZE plasmids) and for plant transformation plasmids (ZP plasmids). Mutations in each vector are indicated relative to SEQ ID NO: 2. ^{**} A simple single, double or triple plus system, +, ++, or +++ for respectively increasing colony size, was used to visually score the tolerance of the maize AHAS function in AHAS minus E. coli containing the ZE plasmids in the presence of the AHAS inhibitor imazethapyr. A "-", indicates there was no growth, meaning the mutation combination caused an inactive protein or there was no tolerance for imazethapyr at the selected rate. IN means inactive protein, while NT means not imidazolinone tolerant. NA means no data available (not tested). ^{@} The maize whole plant tolerance scores are based on combined results from tests conducted in the greenhouse and at multiple field sites over several growing seasons. The scoring system for the maize whole plant tolerance was the same as described above for the *E. coli* imidazolinone tolerance. Note that all ZP constructs with +++ scores are tolerant to more than three thousand grams imazamox per hectare, which represents the highest tested spray rate. | | | | |

### Example 3

### E. coli Complementation Assay

*E. coli* strain JMC1 (genotype [*ilvB1201 ilvHI2202 rbs-221 ara thi delta(prolac) recA56 srlC300::Tn10*], *DE(hsdR)::Cat*) is a knockout for all copies of *ilvG* of the native *E. coli* AHASL gene. This strain can only grow on minimal growth medium lacking leucine, isoleucine, and valine if AHASL is complemented by an exogenous AHASL gene (see Singh, et al. (1992) Plant Physiol. 99, 812-816; Smith, et al. (1989) Proc. Natl. Acad. Sci. USA 86, 4179-4183). This *E. coli* complementation assay was used to screen for AHASL enzyme activity and herbicide tolerance encoded by the AE and ZE vectors in the absence and presence of the imidazolinone herbicide Pursuit® (imazethapyr, BASF Corporation, Florham Park, NJ).

### Example 4

### Biochemical Characterization

Based on growth during complementation testing or simple activity tests, certain of the ZE series of vectors were used for AHAS biochemical assay inhibition testing in crude E. coli lysates. A 2-4 ml culture of LB containing 50 µg/ml carbenicillin (LB-carb) was inoculated with a single colony of JMC1 transformed with the ZE vector to be tested and incubated overnight at 37°C with shaking. The following morning, 0.5-1 ml of overnight culture was used to inoculate 20 ml of LB-carb, which was incubated at 37°C with shaking until the culture optical density (OD) at 600 nm was approximately 0.6 to 0.8 OD units. Isopropyl-1-thio-beta D-galactopyranoside (IPTG) was added to a concentration of 0.1 mM and the cultures incubated with shaking for 1-1.5 hours. The culture was centrifuged to pellet the cells and the supernatant discarded. The cell pellet was lysed with AHAS assay buffer (as in Singh et al. (1988) Anal. Biochem. 171:173-179) supplemented with 10 mg/ml lysozyme and subjected to brief sonication. The insoluble fraction was pelleted by centrifugation and the supernatant used in an assay for AHAS activity. At each concentration of imazethapyr inhibitor used, the activity was compared to an uninhibited control of the same ZE mutant. This results in a "percent of control" measurement.

### Example 5

### Plant Transformation

The AP vectors were transformed into *A. thaliana* ecotype Col-2. The T1 seeds were selected for transformation on plates with 100 nM Pursuit^{®} as the selective agent. T2 seeds from approximately twenty independent transformation events (lines) were plated on MS agar with increasing Pursuit^{®} concentrations, to score increases in tolerance compared to AP1. The vectors were scored by comparison of the highest concentrations of Pursuit^{®} having uninhibited growth of seedlings by visual examination. The results of the *Arabidopsis* transformation experiments are shown in Table 1.

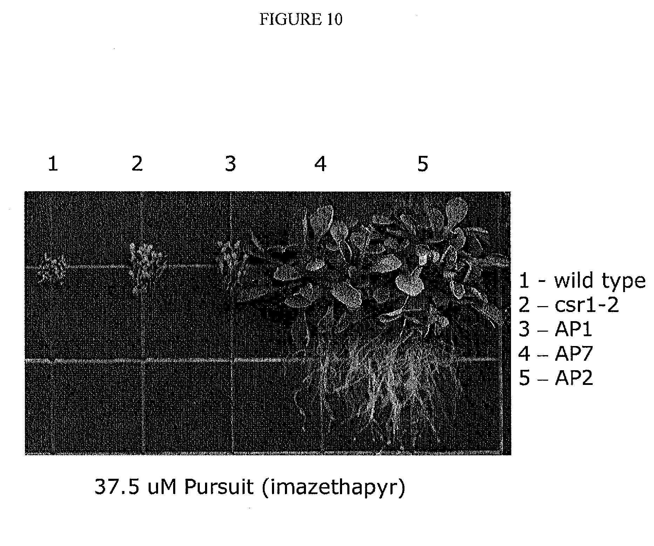

Seeds from several lines of *Arabidopsis* were tested by a vertical plate growth assay. A plate with standard Murashige and Skoog semisolid media containing 37.5 micromolar Pursuit (imazethapyr) was spotted with several seeds in 0.1% agarose. The plate was held vertically, so that the roots would grow along the agar surface. The seeds used were: 1) wild type ecotype Columbia 2; 2) the csr1-2 mutant (homozygous for the AtAHASL S653N mutation in the genomic copy of the AHAS large subunit gene); 3) Columbia 2 transformed with AP1; 4) Columbia 2 transformed with AP7; 5) Columbia 2 transformed with AP2. Note that numbers 2 and 3 are roughly equivalent in terms of probable tolerance, as the AP1 plants are transformed with a clone of the genomic XbaI fragment of csr1-2 (SEQ ID NO: 34). At this concentration of imazethapyr, the wild type seedlings failed to germinate, the single mutant plants (csr1-2 and AP1 transformants) barely germinated. AP7 and AP2 produced good tolerant growth, although the AP7 plants appear to have slightly less root growth. Note that all lines germinated and grew well on media without imazethapyr. The results of the vertical plate growth assay are depicted in Figure 10.

ZP constructs were introduced into maize immature embryos via *Agrobacterium-*mediated transformation. Transformed cells were selected on selection media supplemented with 0.75 µM Pursuit^{®} for 3-4 weeks. Transgenic plantlets were regenerated on plant regeneration media supplemented with 0.75 µM Pursuit^{®}. Transgenic plantlets were rooted in the presence of 0.5 µM Pursuit^{®}. Transgenic plants were subjected to TaqMan analysis for the presence of the transgene before being transplanted to potting mixture and grown to maturity in greenhouse. The results of the maize transformation experiments are shown in Table 2. Maize plants transformed with the ZP constructs were sprayed with varying rates of imazamox, in several field locations and in the greenhouse. The relative ratings of the ZP constructs' whole plant test data are summarized in Table 2.

### Example 6

### Expression of AtAHASL Mutant Genes in Soybean

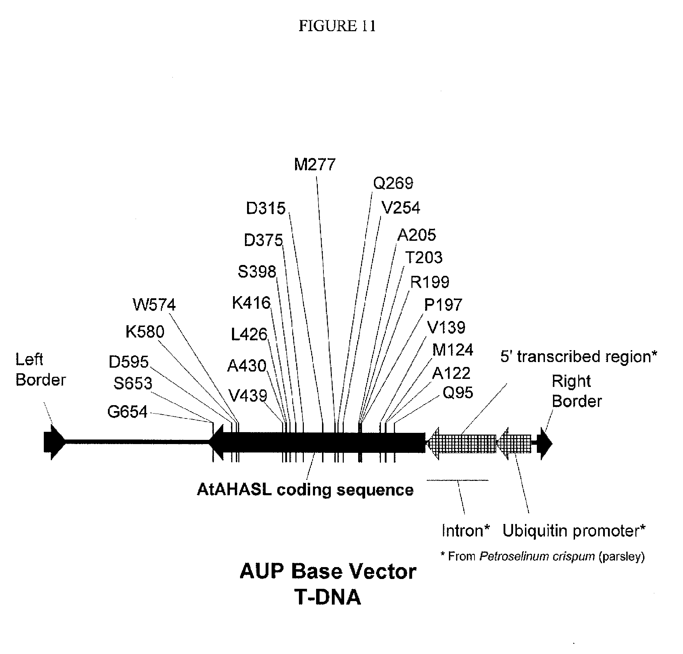

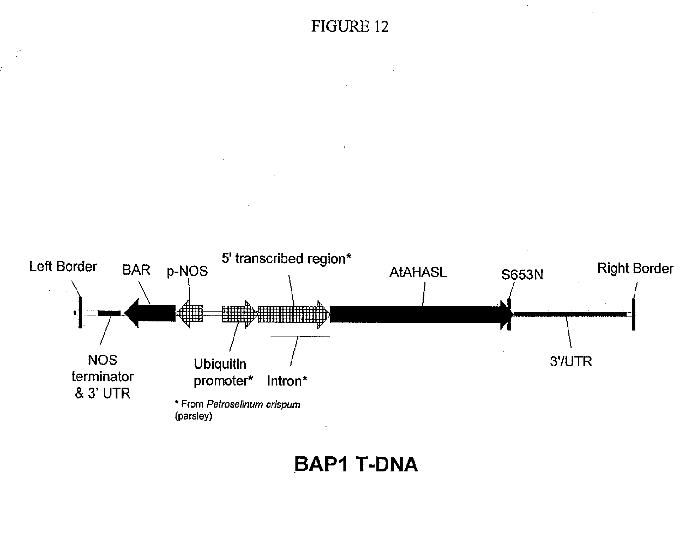

Vectors were prepared for expressing the AtAHASL genes in transformed soybean plants. Vectors AUP2 and AUP3 were made by cloning a polymerase chain reaction product of the parsley ubiquitin promoter, amplified to incorporate sites for the Asp718 and NcoI restriction enzymes, digested and ligated into the same sites of AP2 and AP3 by standard cloning techniques (see, Figures 11 and 12). AUP2 encodes an AtAHASL protein with the A122T and S653N mutations, and AUP3 encodes an AtAHASL protein with the A122T and S653N mutations. Vector BAP1 was made by cloning the entire promoter, coding sequence and 3'-untranslated region sequence of AP1 into a standard dicot transformation backbone containing the BAR selectable marker expression cassette, by standard blunt-ended cloning techniques.

Constructs AP2, AUP2, and AUP3 were introduced into soybean's axillary meristem cells at the primary node of seedling explants via *Agrobacterium-*mediated transformation. After inoculation and co-cultivation with *Agrobacteria,* the explants were transferred to shoot induction media without selection for one week. The explants were subsequently transferred to a shoot induction medium with 5 µM imazapyr (Arsenal) for 3 weeks to select for transformed cells. Explants with healthy callus/shoot pads at the primary node were then transferred to shoot elongation medium containing 3 µM imazapyr until a shoot elongated or the explant died. Transgenic plantlets were rooted, transplanted to potting mixture, subjected to TaqMan analysis for the presence of the trangene, and then grown to maturity in greenhouse. Construct BAP1 was used to produce transformed soybean plants in a like manner, except that the selection agent was BASTA.

The transformed soybean plants were tested for herbicide tolerance in both greenhouse and field studies. For the field study, imazapyr was applied at a rate of 300 g ai/ha at V3 stage. For the greenhouse study, imazapyr was applied at about the V2 stage. The results of these studies are summarized in Table 3.

**Table 3**

| Transformation Vector, Native *Arabidopsis* promoter | Transformation Vector, Parsley Ubiquitin promoter | Mutations | Maximum Greenhouse Tolerance (grams Imazapyr per hectare) | Field Tolerance (grams Imazapyr per hectare) |
|---|---|---|---|---|
| | BAP1^{*} | S653N | 500 | NA |
| AP2 | AUP2 | A122T & S653N | AP2 - 1000, AUP2 - 1500 | AP2 − NA AUP2 - 300 |
| - | AUP3 | P197S & S653N | NA | 300^{**} |

| | | | | |
|---|---|---|---|---|
| ^{*} BAP1 (figure 12) was transformed using the BAR gene for selection, as imazapyr selection in soybeans with the S653N mutation alone has not been optimized. ** Some injury compared to AUP2 | | | | |

### Example 7

### Transformant Selection

The polynucleotides generated by the invention may be used as selectable markers for plant transformation. The polynucleotides generated by the invention may be used as selectable markers to identify and/or select transformed plants which may comprise additional genes of interest. Plants or plant cells transformed with vectors containing the multiple mutant forms of the AHAS large subunit genes can be selected from non-transformed plants or plant cells by plating on minimal media, such as MS media, which incorporate AHAS inhibitors or AHAS inhibiting herbicides, such as imidazolinones. The transformed plants or tissues will be able to continue growing in the presence of these inhibitors, while the untransformed plants or tissues will die. In the case of transformed tissues, since the non-transformed tissues may receive branched chain amino acids from the transformed tissues, the actively growing tissues are removed from the slower growing or dying tissues and replated on selective media.

Whole plants may also be selected by planting the seeds and waiting for germination and seedling growth, followed by spraying the seedlings with AHAS inhibitors or AHAS inhibiting herbicides, such as imidazolinones. The transformed plants will survive while the untransformed plants will be killed.

### Example 8

### Field Trials with Transformed Maize

Field trials were conducted to assess whether or not maize plants transformed with one of the vectors comprising AHASL double and triple mutants displayed any gross physiological or reproductive affects with and without an imazamox application.

### Materials and Methods

### Source of Test Material

The genetically modified organism was produced by transforming corn inbred J553. F1 hybrid seed from 8 vector constructs were produced using TR5753 as an inbred male tester. The vector constructs are described in Table 4. Seed for the trial were produced in an isolated crossing block on the island of Kauai, Hawaii. USA during the 2006-2007 contraseason. Subsamples of each F1 hybrid produced were analyzed for the presence of the correct vector construct and absence of adventitious presence of other AHASL contructs.

Nontransformed commercial hybrids were purchased from Midwest USA corn seed companies and analyzed to confirm the absence of any adventitious presence of other AHASL contructs.

**Table 4**

| Construct | pZm UBI + I::c-ZmAHAS L2::t-ZmAHAS L2 Mutations (at Designation) |
|---|---|
| 1 | P197S |
| 2 | A122T, R199A |
| 3 | A122T |
| 4 | A122T, M124I |
| 5 | M124I, S653N |
| 6 | A122T, S653N |
| 7 | A122T, R199S, S653N |
| 8 | S653N |

### Trial Methodology

Trial design was a Split Plot in a Randomized Complete Block Design, with the main plot being an herbicide treatment, and the sub plot being an F1 hybrid entry. The herbicide treatments included 1) untreated and 2) imazamox applied at 150 gai/ha. The F1 hybrid entries included 29 events from 8 vector constructs and 4 non-transformed commercial hybrids (3395IR from Pioneer Hi-Bred International, Inc, Johnston, IA, USA; and 8342GLS/IT, 8546IT, and 8590IT from Garst Seed Co., Inc., Slater, IA. USA). Plot size was 2 rows; row width 2.5 feet; row length 20 feet. Each treatment combination had 4 replications. The trial was planted at three locations. These locations were: Ames, IA, USA; Estherville, IA, USA; and York, NE, USA. All trials were planted during May 2007.

### Location conditions

The Ames, IA location was in a corn-after-corn rotation which may have had some impact on uniformity of emergence and early growth as significant amounts of corn residue were present at planting. No major influences on the crop due to weather, disease or insects were noted. The Estherville, IA site received heavy rain driven by 70 mph wind gusts and sustained winds of around 40 mph on July 16. Root lodging was observed in essentially every plot. No major influences on the crop due to disease or insects were noted. The York, NE site received above-normal rainfall in May, July and August and no major insect or disease issues were noted.

### Results and Discussion

Data analyses combined across three locations resulted in one vector construct with a significant yield decrease (p ≤0.05) when comparing yield with or without the imazamox herbicide application (Table 5). One vector construct had a significant yield increase when treated with the imazamox application. Other agronomic characteristics were also collected from the three trial locations, and no significant differences were detected within a construct when treated or untreated with imazamox for the traits plant height, ear height, stalk lodging and root lodging (data not shown).

The objective of the trial was to identify if an herbicide application of imazamox applied to vector constructs that have been optimized to provide improved herbicide tolerance to imazamox would result in gross, or obvious, physiological or reproductive affects, primarily yield. Only one vector construct (Construct 1, single mutant, P197L) had a significant (p ≤ 0.05) negative response for grain yield when treated with imazamox. The remaining seven vector constructs exhibited no adverse physiological or reproductive affects in the presence or absence of the herbicide imazamox. The results of these field trials demonstrate the excellent agronomic potential of maize plants transformed with a vector comprising either an AHASL double or triple mutant.

**Table 5**

| | | Yield (bu/A) | | | | | |
|---|---|---|---|---|---|---|---|
| Description | | Herbicide (H) | | Non-herbicide (NH) | | (H/NH)% | p-value |
| Construct | # Event | Mean | α = 0.05 | Mean | α = 0.05 | | |
| 1 | 4 | 160.68 | B | 175.58 | BC | 91.51 | 0.003 |
| 2 | 4 | 176.72 | AB | 177.05 | ABC | 99.81 | 0.94 |
| 3 | 4 | 180.59 | A | 179.02 | AB | 100.87 | 0.74 |
| 4 | 4 | 173.57 | AB | 163.79 | C | 105.97 | 0.11 |
| 5 | 4 | 173.87 | AB | 180.84 | AB | 96.14 | 0.10 |
| 6 | 4 | 188.02 | A | 178.65 | AB | 105.24 | 0.04 |
| 7 | 1 | 187.45 | AB | 189.03 | AB | 99.16 | 0.88 |
| 8 | 4 | 183.14 | A | 177.54 | ABC | 103.15 | 0.27 |
| 3395IR | | 168.03 | AB | 176.89 | ABC | 94.99 | 0.09 |
| 8590IT | | 181.92 | AB | 197.57 | A | 92.08 | 0.04 |
| G8546IT | | 174.94 | AB | 186.74 | AB | 93.68 | 0.40 |
| G8342GLS/IT | | 180.14 | AB | 177.53 | ABC | 101.47 | 0.84 |

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

Preferred embodiments (E1-E48) of the invention are:
E 1. An isolated polynucleotide encoding an acetohydroxyacid synthase large subunit (AHASL) double mutant polypeptide selected from the group consisting of:
   a) a polypeptide having a valine, threonine, glutamine, cysteine, or methionine at a position corresponding to position 122 of SEQ ID NO:1 or position 90 of SEQ ID NO:2 and a phenylalanine, asparagine, threonine, glycine, valine, or tryptophan at a position corresponding to position 653 of SEQ ID NO: 1 or position 621 of SEQ ID NO:2;
   b) a polypeptide having a valine, threonine, glutamine, cysteine, or methionine at a position corresponding to position 122 of SEQ ID NO:1 or position 90 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2;
   c) a polypeptide having a valine, threonine, glutamine, cysteine, or methionine at a position corresponding to position 122 of SEQ ID NO:1 or position 90 of SEQ ID NO:2 and a serine, alanine, glutamic acid, leucine, glutamine, arginine, valine, tryptophan, tyrosine, or isoleucine at a position corresponding to position 197 of SEQ ID NO:1 or position 165 of SEQ ID NO:2;
   d) a polypeptide having a valine, threonine, glutamine, cysteine, or methionine at a position corresponding to position 122 of SEQ ID NO:1 or position 90 of SEQ ID NO:2 and a glutamic acid, isoleucine, leucine, or asparagine at a position corresponding to position 124 of SEQ ID NO:1 or position 92 of SEQ ID NO:2;
   e) a polypeptide having a valine, threonine, glutamine, cysteine, or methionine at a position corresponding to position 122 of SEQ ID NO:1 or position 90 of SEQ ID NO:2 and an isoleucine at a position corresponding to position 139 of SEQ ID NO:1 or position 107 of SEQ ID NO:2;
   f) a polypeptide having a valine, threonine, glutamine, cysteine, or methionine at a position corresponding to position 122 of SEQ ID NO:1 or position 90 of SEQ ID NO:2 and a histidine at a position corresponding to position 269 of SEQ ID NO:1 or position 237 of SEQ ID NO:2;
   g) a polypeptide having a valine, threonine, glutamine, cysteine, or methionine at a position corresponding to position 122 of SEQ ID NO:1 or position 90 of SEQ ID NO:2 and a methionine at a position corresponding to position 416 of SEQ ID NO:1 or position 384 of SEQ ID NO:2;
   h) a polypeptide having a valine, threonine, glutamine, cysteine, or methionine at a position corresponding to position 122 of SEQ ID NO:1 or position 90 of SEQ ID NO:2 and an isoleucine at a position corresponding to position 426 of SEQ ID NO:1 or position 394 of SEQ ID NO:2;
   i) a polypeptide having a valine, threonine, glutamine, cysteine, or methionine at a position corresponding to position 122 of SEQ ID NO:1 or position 90 of SEQ ID NO:2 and a valine at a position corresponding to position 430 of SEQ ID NO:1 or position 398 of SEQ ID NO:2;
   j) a polypeptide having a valine, threonine, glutamine, cysteine, or methionine at a position corresponding to position 122 of SEQ ID NO:1 or position 90 of SEQ ID NO:2 and an isoleucine at a position corresponding to position 442 of SEQ ID NO:1 or position 410 of SEQ ID NO:2;
   k) a polypeptide having a valine, threonine, glutamine, cysteine, or methionine at a position corresponding to position 122 of SEQ ID NO: or position 90 of SEQ ID NO:2 and an isoleucine or aspartic acid at a position corresponding to position 445 of SEQ ID NO:1 or position 413 of SEQ ID NO:2;
   l) a polypeptide having a valine, threonine, glutamine, cysteine, or methionine at a position corresponding to position 122 of SEQ ID NO:1 or position 90 of SEQ ID NO:2 and a glutamic acid at a position corresponding to position 580 of SEQ ID NO:1 or position 548 of SEQ ID NO:2;
   m) a polypeptide having a glutamic acid, isoleucine, leucine, or asparagine at a position corresponding to position 124 of SEQ ID NO:1 or position 92 of SEQ ID NO:2 and a phenylalanine, asparagine, threonine, glycine, valine, or tryptophan at a position corresponding to position 653 of SEQ ID NO:1 or position 621 of SEQ ID NO:2;
   n) a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2;
   o) a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an asparagine at a position corresponding to position 375 of SEQ ID NO:1 or position 343 of SEQ ID NO:2;
   p) a polypeptide having an alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2;
   q) a polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2; and
   r) a polypeptide having a valine, cysteine, aspartic acid, glutamic acid, arginine, threonine, tryptophan, or tyrosine at a position corresponding to position 205 of SEQ ID NO:1 or position 173 of SEQ ID NO:2 and a phenylalanine, asparagine, threonine, glycine, valine, or tryptophan at a position corresponding to position 653 of SEQ ID NO:1 or position 621 of SEQ ID NO:2.
E 2. The isolated polynucleotide of E 1, wherein the encoded AHASL double mutant is derived from a plant.
E 3. The isolated polynucleotide of _{E} 1, wherein the plant is selected from the groups consisting of *Arabidopsis thaliana,* maize, wheat, rye, oat, triticale, rice, barley, sorghum, millet, sugar beet, sugarcane, soybean, peanut, cotton, rapeseed, canola, *Brassica* species, manihot, melon, squash, pepper, sunflower, tagetes, solanaceous plants, potato, sweet potato, tobacco, eggplant, tomato, *Vicia* species, pea, alfalfa, coffee, cacao, tea, *Salix* species, oil palm, coconut, perennial grass, and forage crops.
E 4. The isolated polynucleotide of E 1, wherein the polynucleotide is selected from the group consisting of:
   i) a polypeptide having a valine, threonine, glutamine, cysteine, or methionine at a position corresponding to position 122 of SEQ ID NO:1 or position 90 of SEQ ID NO:2 and a phenylalanine, asparagine, threonine, glycine, valine, or tryptophan at a position corresponding to position 653 of SEQ ID NO:1 or position 621 of SEQ ID NO:2; and
   ii) a polypeptide having a valine, threonine, glutamine, cysteine, or methionine at a position corresponding to position 122 of SEQ ID NO:1 or position 90 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2.
_{E} 5. An expression vector comprising the isolated polynucleotide of _{E} 1.
E 6. The expression vector of E 5, wherein the vector further comprises one or more regulatory sequences.
E 7. A transgenic plant comprising the expression vector of E 5.
^{E} 8. The transgenic plant of E 7, wherein the plant is a monocot.
E 9. The transgenic plant of E 7, wherein the plant is a dicot.
E 10. The transgenic plant of E 7, wherein the plant is selected from the group consisting of *Arabidopsis,* maize, wheat, rye, oat, triticale, rice, barley, sorghum, millet, sugarcane, soybean, sugar beet, peanut, cotton, rapeseed, canola, *Brassica* species, manihot, pepper, sunflower, tagetes, solanaceous plants, potato, tobacco, eggplant, tomato, *Vicia* species, pea, alfalfa, coffee, cacao, tea, *Salix* species, oil palm, coconut, perennial grass and a forage plant.
E 11. A plant seed produced by the transgenic plant of ^{E} 7, wherein the seed comprises the isolated polynucleotide.
E 12. The transgenic plant of E 7, wherein the expression of the polynucleotide in the plant results in tolerance to a herbicide selected from the group consisting of imidazolinones, sulfonylureas, triazolopyrimidines, and pyrimidinyloxybenzoates.
E 13. A purified AHASL protein encoded by the isolated polynucleotide of E 1.
E 14. A plant comprising a first polynucleotide encoding a first AHASL single mutant polypeptide and a second polynucleotide encoding a second AHASL single mutant polypeptide, or an AHASL encoding polynucleotide comprising two nucleotide mutations that result in amino acid mutations corresponding to the amino acid mutations of said first and said second AHASL single mutant polypeptides, wherein said first and said second polypeptides are selected from the group consisting of:
   a) a first polypeptide having a valine, threonine, glutamine, cysteine, or methionine at a position corresponding to position 122 of SEQ ID NO:1 or position 90 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2;
   b) a first polypeptide having a valine, threonine, glutamine, cysteine, or methionine at a position corresponding to position 122 of SEQ ID NO:1 or position 90 of SEQ ID NO:2 and a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2;
   c) a first polypeptide having a valine, threonine, glutamine, cysteine, or methionine at a position corresponding to position 122 of SEQ ID NO:1 or position 90 of SEQ ID NO:2 and a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2;
   d) a first polypeptide having a valine, threonine, glutamine, cysteine, or methionine at a position corresponding to position 122 of SEQ ID NO:1 or position 90 of SEQ ID NO:2 and a second polypeptide having a glutamic acid, isoleucine, leucine, or asparagine at a position corresponding to position 124 of SEQ ID NO:1 or position 92 of SEQ ID NO:2;
   e) a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 139 of SEQ ID NO:1 or position 107 of SEQ ID NO:2;
   f) a first polypeptide having a valine, threonine, glutamine, cysteine, or methionine at a position corresponding to position 122 of SEQ ID NO:1 or position 90 of SEQ ID NO:2 and a second polypeptide having a histidine at a position corresponding to position 269 of SEQ ID NO:1 or position 237 of SEQ ID NO:2;
   g) a first polypeptide having a valine, threonine, glutamine, cysteine, or methionine at a position corresponding to position 122 of SEQ ID NO:1 or position 90 of SEQ ID NO:2 and a second polypeptide having a methionine at a position corresponding to position 416 of SEQ ID NO:1 or position 3 84 of SEQ ID NO:2;
   h) a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 426 of SEQ ID NO:1 or position 394 of SEQ ID NO:2;
   i) a first polypeptide having a valine, threonine, glutamine, cysteine, or methionine at a position corresponding to position 122 of SEQ ID NO:1 or position 90 of SEQ ID NO:2 and a second polypeptide having a valine at a position corresponding to position 430 of SEQ ID NO:1 or position 398 of SEQ ID NO:2;
   j) a first polypeptide having a valine, threonine, glutamine, cysteine, or methionine at a position corresponding to position 122 of SEQ ID NO:1 or position 90 of SEQ ID NO:2 and a second polypeptide having an isoleucine at a position corresponding to position 442 of SEQ ID NO:1 or position 410 of SEQ ID NO:2;
   k) a first polypeptide having a valine, threonine, glutamine, cysteine, or methionine at a position corresponding to position 122 of SEQ ID NO:1 or position 90 of SEQ ID NO:2 and a second polypeptide having an isoleucine or aspartic acid at a position corresponding to position 445 of SEQ ID NO:1 or position 413 of SEQ ID NO:2;
   l) a first polypeptide having a valine, threonine, glutamine, cysteine, or methionine at a position corresponding to position 122 of SEQ ID NO:1 or position 90 of SEQ ID NO:2 and a second polypeptide having a glutamic acid at a position corresponding to position 580 of SEQ ID NO:1 or position 548 of SEQ ID NO:2;
   m) a first glutamic acid, isoleucine, leucine, or asparagine at a position corresponding to position 124 of SEQ ID NO:1 or position 92 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2;
   n) a first polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having a phenylalanine, asparagine, threonine, glycine, valine, or tryptophan at a position corresponding to position 653 of SEQ ID NO: or position 621 of SEQ ID NO:2;
   o) a first polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having an asparagine at a position corresponding to position 375 of SEQ ID NO:1 or position 343 of SEQ ID NO:2;
   p) a first polypeptide having an alanine, glutamic acid, leucine, glutamine, arginine, valine, tryptophan, tyrosine, or isoleucine at a position corresponding to position 197 of SEQ ID NO:1 or position 165 of SEQ ID NO:2 and a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2;
   q) a first polypeptide having an alanine, glutamic acid, serine, phenylalanine, threonine, aspartic acid, cysteine, or asparagine at a position corresponding to position 199 of SEQ ID NO:1 or position 167 of SEQ ID NO: 2 and a second polypeptide having a phenylalanine, asparagine, threonine, glycine, valine, or tryptophan at a position corresponding to position 653 of SEQ ID NO:1 or position 621 of SEQ ID NO:2; and
   r) a first polypeptide having a valine, cysteine, aspartic acid, glutamic acid, arginine, threonine, tryptophan, or tyrosine at a position corresponding to position 205 of SEQ ID NO:1 or position 173 of SEQ ID NO:2 and a second polypeptide having a phenylalanine, asparagine, threonine, glycine, valine, or tryptophan at a position corresponding to position 653 of SEQ ID NO:1 or position 621 of SEQ ID NO:2.
E 15. The plant of E 14, wherein the plant is a monocot.
E 16. The plant of E 14, wherein the plant is a dicot.
^{E} 17. The plant of E 14, wherein the plant is selected from the group consisting of *Arabidopsis,* maize, wheat, rye, oat, triticale, rice, barley, sorghum, millet, sugarcane, soybean, sugar beet, peanut, cotton, rapeseed, canola, *Brassicca* species, manihot, pepper, sunflower, tagetes, solanaceous plants, potato, tobacco, eggplant, tomato, *Vicia* species, pea, alfalfa, coffee, cacao, tea, *Salix* species, oil palm, coconut, perennial grass and a forage plant.
_{E} 18. A plant seed produced by the plant of _{E} 14, wherein the seed comprises the AHASL encoding polynucleotide.
E 19. The plant of E 14, wherein the plant is selected from the groups consisting of *Arabidopsis thaliana,* maize, wheat, rye, oat, triticale, rice, barley, sorghum, millet, sugar beet, sugarcane, soybean, peanut, cotton, rapeseed, canola, *Brassica* species, manihot, melon, squash, pepper, sunflower, tagetes, solanaceous plants, potato, sweet potato, tobacco, eggplant, tomato, Vicia species, pea, alfalfa, coffee, cacao, tea, Salix species, oil palm, coconut, perennial grass, and forage crops.
E 20. An isolated polynucleotide encoding an AHASL triple mutant polypeptide selected from the group consisting of:
   a) a polypeptide having a valine, threonine, glutamine, cysteine, or methionine at a position corresponding to position 122 of SEQ ID NO:1 or position 90 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and a phenylalanine, asparagine, threonine, glycine, valine, or tryptophan at a position corresponding to position 653 of SEQ ID NO:1 or position 621 of SEQ ID NO:2;
   b) a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a phenylalanine, asparagine, threonine, glycine, valine, or tryptophan at a position corresponding to position 653 of SEQ ID NO:1 or position 621 of SEQ ID NO:2;
   c) a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2;
   d) a polypeptide having a valine, threonine, glutamine, cysteine, or methionine at a position corresponding to position 122 of SEQ ID NO:1 or position 90 of SEQ ID NO:2, an arginine at a position corresponding to position 57 of SEQ ID NO:1 and a leucine at a position corresponding to position 398 of SEQ ID NO:1 or position 366 of SEQ ID NO:2;
   e) a polypeptide having a glutamic acid, isoleucine, leucine, or asparagine at a position corresponding to position 124 of SEQ ID NO:1 or position 92 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and an alanine, glutamic acid, serine, phenylalanine, threonine, aspartic acid, cysteine, or asparagine at a position corresponding to position 199 of SEQ ID NO:1 or position 167 of SEQ ID NO:2;
   f) a polypeptide having a leucine at a position corresponding to position 95 of SEQ ID NO:1 or position 63 of SEQ ID NO:2, a glutamic acid at a position corresponding to position 416 of SEQ ID NO:1 or position 384 of SEQ ID NO:2 and a phenylalanine, asparagine, threonine, glycine, valine, or tryptophan at a position corresponding to position 653 of SEQ ID NO:1 or position 621 of SEQ ID NO:2; and
   g) a polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and any amino acid at a position corresponding to position 574 of SEQ ID NO:1 or position 542 of SEQ ID NO:2.
E 21. The isolated polynucleotide of _{E} 20, wherein the encoded AHASL triple mutant is derived from a plant.
E 22. The isolated polynucleotide of E 21, wherein the plant is selected from the group consisting of *Arabiclopsis thaliana,* maize, wheat, rye, oat, triticale, rice, barley, sorghum, millet, sugar beet, sugarcane, soybean, peanut, cotton, rapeseed, canola, *Brassicca* species, manihot, melon, squash, pepper, sunflower, tagetes, solanaceous plants, potato, sweet potato, tobacco, eggplant, tomato, Vicia species, pea, alfalfa, coffee, cacao, tea, Salix species, oil palm, coconut, perennial grass, and forage crops.
E 23. An expression vector comprising the isolated polynucleotide of E 20.
^{E} 24. The expression vector of E 23, wherein the vector further comprises one or more regulatory sequences.
E 25. A transgenic plant comprising the expression vector of E 23.
_{E} 26. The transgenic plant of E 25, wherein the plant is a monocot.
E 27. The transgenic plant of _{E} 25, wherein the plant is a dicot.
E 28. The transgenic plant of E 25, wherein the plant is selected from the group consisting of *Arabiclopsis thaliana,* maize, wheat, rye, oat, triticale, rice, barley, sorghum, millet, sugar beet, sugarcane, soybean, peanut, cotton, rapeseed, canola, *Brassica* species, manihot, melon, squash, pepper, sunflower, tagetes, solanaceous plants, potato, sweet potato, tobacco, eggplant, tomato, Vicia species, pea, alfalfa, coffee, cacao, tea, Salix species, oil palm, coconut, perennial grass, and forage crops.
_{E} 29. A plant seed produced by the plant of _{E} 25, wherein the seed comprises the AHASL encoding polynucleotide.
_{E} 30. A purified AHASL protein encoded by the isolated polynucleotide of _{E 20} .
E 31. A plant comprising a first polynucleotide encoding a first AHASL single mutant polypeptide, a second polynucleotide encoding a second AHASL single mutant polypeptide, and a third polynucleotide encoding a third AHASL single mutant polypeptide; or an AHASL encoding polynucleotide comprising three mutations, wherein the three nucleotide mutations result in the amino acid mutations corresponding to the mutations of said first, second and third AHASL single mutant polypeptides; or an AHASL encoding polynucleotide comprising a single mutation and an AHASL encoding polynucleotide comprising a double mutations, wherein the nucleotide mutations result in the amino acid mutations corresponding to the amino acid mutations of said first, second and third AHASL single mutant polypeptides, wherein said first, second, and third AHASL single mutant polypeptides are selected from the group consisting of:
   a) a first polypeptide having a valine, threonine, glutamine, cysteine, or methionine at a position corresponding to position 122 of SEQ ID NO:1 or position 90 of SEQ ID NO:2, a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a third polypeptide having a phenylalanine, asparagine, threonine, glycine, valine, or tryptophan at a position corresponding to position 653 of SEQ ID NO:1 or position 621 of SEQ ID NO:2;
   b) a first polypeptide having a valine, threonine, glutamine, cysteine, or methionine at a position corresponding to position 122 of SEQ ID NO:1 or position 90 of SEQ ID NO:2, a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a third polypeptide having a phenylalanine, asparagine, threonine, glycine, valine, or tryptophan at a position corresponding to position 653 of SEQ ID NO:1 or position 621 of SEQ ID NO:2;
   c) a first polypeptide having a valine, threonine, glutamine, cysteine, or methionine at a position corresponding to position 122 of SEQ ID NO:1 or position 90 of SEQ ID NO:2, a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a third polypeptide having an alanine, glutamic acid, serine, phenylalanine, threonine, aspartic acid, cysteine, or asparagine at a position corresponding to position 199 of SEQ ID NO:1 or position 167 of SEQ ID NO:2;
   d) a first polypeptide having a valine, threonine, glutamine, cysteine, or methionine at a position corresponding to position 122 of SEQ ID NO:1 or position 90 of SEQ ID NO:2, a second polypeptide having an arginine at a position corresponding to position 57 of SEQ ID NO:1 and a third polypeptide having a leucine at a position corresponding to position 398 of SEQ ID NO:1 or position 366 of SEQ ID NO:2;
   e) a first polypeptide having a glutamic acid, isoleucine, leucine, or asparagine at a position corresponding to position 124 of SEQ ID NO:1 or position 92 of SEQ ID NO:2, a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a third polypeptide having an alanine, glutamic acid, serine, phenylalanine, threonine, aspartic acid, cysteine, or asparagine at a position corresponding to position 199 of SEQ ID NO:1 or position 167 of SEQ ID NO:2;
   f) a first polypeptide having a leucine at a position corresponding to position 95 of SEQ ID NO:1 or position 63 of SEQ ID NO:2, a second polypeptide having a glutamic acid at a position corresponding to position 416 of SEQ ID NO:1 or position 384 of SEQ ID NO:2 and a third polypeptide having a phenylalanine, asparagine, threonine, glycine, valine, or tryptophan at a position corresponding to position 653 of SEQ ID NO:1 or position 621 of SEQ ID NO:2; and
   g) a first polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2, a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a third polypeptide having any amino acid at a position corresponding to position 574 of SEQ ID NO:1 or position 542 of SEQ ID NO:2.
E 32. The plant of E 31, wherein the plant is a monocot.
E 33. The plant of E 31, wherein the plant is a dicot.
E 34. The plant of E 31, wherein the plant is selected from the group consisting of *Arabidopsis,* maize, wheat, rye, oat, triticale, rice, barley, sorghum, millet, sugarcane, soybean, sugar beet, peanut, cotton, rapeseed, canola, *Brassica* species, manihot, pepper, sunflower, tagetes, solanaceous plants, potato, tobacco, eggplant, tomato, *Vicia* species, pea, alfalfa, coffee, cacao, tea, *Salix* species, oil palm, coconut, perennial grass and a forage plant.
_{E} 35. A plant seed produced by the plant of E 31, wherein the seed comprises the AHASL encoding polynucleotide.
_{E} 36. A method of controlling weeds in the vicinity of crop plants comprising:
   i) planting in a field seeds of any one of _{E} 11, 18, 29, and 35; or seeds produced by the plant of any one of _{E} 7-10, 14-17, 19, 25-28, and 31-34; and
   ii) applying an effective amount of an imidazolinone herbicide, a sulfonylurea herbicide, or a mixture thereof to the weeds and to the crop plants in the field to control the weeds.
_{E} 37. A method of producing a transgenic plant comprising the steps of:
   i) transforming a plant cell with the expression vector of any one of _{E} 5, 6, 23, and 24; and
   ii) regenerating from the plant cell a transgenic plant that expresses the AHASL mutant polypeptide.
E 38. A method of identifying or selecting a transformed plant cell, plant tissue, plant or part thereof comprising:
   i) providing a transformed plant cell, plant tissue, plant or part thereof, wherein said transformed plant cell, plant tissue, plant or part thereof comprises the polynucleotide of any one of _{E} 1-4 and 20-22, wherein the polynucleotide encodes an AHASL mutant polypeptide that is used as a selection marker, and wherein said transformed plant cell, plant tissue, plant or part thereof may comprise a further isolated polynucleotide;
   ii) contacting the transformed plant cell, plant tissue, plant or part thereof with at least one AHAS inhibitor or AHAS inhibiting compound;
   iii) determining whether the plant cell, plant tissue, plant or part thereof is affected by the inhibitor or inhibiting compound; and
   iv) identifying or selecting the transformed plant cell, plant tissue, plant or part thereof.
_{E} 39. A method for producing a herbicide-resistant plant comprising crossing a first plant that is resistant to a herbicide to a second plant that is not resistant to the herbicide, wherein the first plant is the plant of any one of E 7-10, 14-17, 19, 25-28, and 31-34.
E 40. The method of E 39 further comprising selecting for a progeny plant that is resistant to the herbicide.
_{E}41. A herbicide-resistant plant produced by the method of E 39 or 40.
E 42. A seed of the plant of E 41, wherein said seed comprises the herbicide resistant characteristics of the first plant.
E43. A method for increasing the herbicide-resistance of a plant comprising crossing a first plant to a second plant, wherein the first plant is the plant of any one of _{E} 7-10, 14-17, 19, 25- 28, 31-34, and 41.
_{E}44. The method of _{E} 43 further comprising selecting for a progeny plant that comprises increased herbicide resistance when compared to the herbicide resistance of said second plant.
E45. A plant produced by the method of E 43 or 44.
_{E}46. A seed of the plant of E 44, wherein said seed comprises the increased herbicide resistance.
E47. A seed of the plant of any one of E 7-10, 14-17, 19, 25-28, 31-34, 41, and 45, wherein said seed is treated with an AHAS-inhibiting herbicide.
_{E}48. A method for combating undesired vegetation comprising contacting a seed of the plant of any one of _{E} 7-10, 14-17, 19, 25-28, 31-34, 41, and 45 before sowing and/or after pregermination with an AHAS-inhibiting herbicide.

## Claims

1. A polynucleotide encoding an acetohydroxyacid synthase large subunit (AHASL) triple-substituted polypeptide having a valine, threonine, glutamine, cysteine, or methionine substitution at a position corresponding to position 122 of SEQ ID NO: or position 90 of SEQ ID NO:2, any amino acid substitution a position corresponding to position 574 of SEQ ID NO: 1 or position 542 of SEQ ID NO:2, and a phenylalanine, asparagine, threonine, glycine, valine, or tryptophan substitution at a position corresponding to position 653 of SEQ ID NO: or position 621 of SEQ ID NO:2.

2. The polynucleotide of claim 1, wherein said polynucleotide encodes an AHASL triple-substituted polypeptide having a threonine substitution at the position corresponding to position 122 of SEQ ID NO: or position 90 of SEQ ID NO:2, a leucine substitution at the position corresponding to position 574 of SEQ ID NO: or position 542 of SEQ ID NO:2, and an asparagine substitution at the position corresponding to position 653 of SEQ ID NO: 1 or position 621 of SEQ ID NO:2.

3. The polynucleotide of claim 1 or 2, wherein the polynucleotide is derived from a plant.

4. The polynucleotide of claim 3, wherein the plant is selected from the group consisting of *Arabidopsis thaliana,* maize, wheat, rye, oat, triticale, rice, barley, sorghum, millet, sugar beet, sugarcane, soybean, peanut, cotton, rapeseed, canola, *Brassica* species, manihot, melon, squash, pepper, sunflower, tagetes, solanaceous plants, potato, sweet potato, tobacco, eggplant, tomato, *Vicia* species, pea, alfalfa, coffee, cacao, tea, *Salix* species, oil palm, coconut, perennial grass, and forage crops.

5. The polynucleotide of any one of claims 1-4, wherein said polynucleotide is an isolated polynucleotide or a mutagenized AHASL gene.

6. An expression vector comprising the polynucleotide of any one of claims 1 to 5 and capable of expressing said polynucleotide in a host cell.

7. A transgenic plant comprising the expression vector of claim 6.

8. A plant comprising a first polynucleotide encoding a first AHASL single mutant polypeptide, a second polynucleotide encoding a second AHASL single mutant polypeptide, and a third polynucleotide encoding a third AHASL single mutant polypeptide; or an AHASL encoding polynucleotide comprising three mutations, wherein the three nucleotide mutations result in the amino acid mutations corresponding to the mutations of said first, second and third AHASL single mutant polypeptides; or an AHASL encoding polynucleotide comprising a single mutation and an AHASL encoding polynucleotide comprising a double mutation, wherein the nucleotide mutations result in the amino acid mutations corresponding to the amino acid mutations of said first, second and third AHASL single mutant polypeptides, wherein said first polypeptide has a valine, threonine, glutamine, cysteine, or methionine substitution at a position corresponding to position 122 of SEQ ID NO: or position 90 of SEQ ID NO:2, said second polypeptide has any amino acid substitution a position corresponding to position 574 of SEQ ID NO:1 or position 542 of SEQ ID NO:2, and said third polypeptide has a phenylalanine, asparagine, threonine, glycine, valine, isoleucine, or tryptophan substitution at a position corresponding to position 653 of SEQ ID NO:1 or position 621 of SEQ ID NO:2.

9. The plant of claim 8, wherein said first polypeptide has a threonine substitution at a position corresponding to position 122 of SEQ ID NO:1 or position 90 of SEQ ID NO:2, said second polypeptide has a leucine substitution a position corresponding to position 574 of SEQ ID NO:1 or position 542 of SEQ ID NO:2, and said third polypeptide has an asparagine substitution at a position corresponding to position 653 of SEQ ID NO:1 or position 621 of SEQ ID NO:2.

10. The plant of any one of claims 8-9, wherein said polynucleotide is an isolated polynucleotide or a mutagenized AHASL gene.

11. The plant of any one of claims 7-10, wherein the plant is a monocot or a dicot.

12. The plant of claim 11, wherein the plant is selected from the group consisting of *Arabidopsis thaliana,* maize, wheat, rye, oat, triticale, rice, barley, sorghum, millet, sugar beet, sugarcane, soybean, peanut, cotton, rapeseed, canola, *Brassica* species, manihot, melon, squash, pepper, sunflower, tagetes, solanaceous plants, potato, sweet potato, tobacco, eggplant, tomato, *Vicia* species, pea, alfalfa, coffee, cacao, tea, *Salix* species, oil palm, coconut, perennial grass, and forage crops.

13. The plant of any one of claims 7-12, wherein the expression of the polynucleotide in the plant results in tolerance to an herbicide selected from the group consisting of imidazolinones, sulfonylureas, triazolopyrimidines, and pyrimidinyloxybenzoates.

14. A seed of the plant of any one of claims 7-13, wherein the seed comprises the polynucleotide(s).

15. A method for controlling weeds in a field, said method comprising both:
i) growing in a field a plant comprising the polynucleotide of any one of claims 1-5, or the plant of any one of claims 7-13; and
ii) contacting said plant and weeds in the field with an effective amount of an AHAS-inhibiting herbicide to which the plant is tolerant and which would inhibit the growth of a corresponding wild-type plant, thereby controlling the weeds.

16. A method of producing a transgenic plant comprising the steps of:
i) transforming a plant cell with the expression vector of claim 6; and
ii) regenerating from the plant cell a transgenic plant that expresses the AHASL mutant polypeptide.

17. A method of identifying or selecting a plant cell, plant tissue, or plant or part thereof comprising:
i) providing a plant cell, plant tissue, or plant or part thereof, wherein said plant cell, plant tissue, or plant or part thereof comprises the polynucleotide of any one of claims 1-5;
ii) contacting the plant cell, plant tissue, or plant or part thereof with at least one AHAS-inhibiting compound;
iii) determining whether the plant cell, plant tissue, or plant or part thereof is affected by the inhibiting compound; and
iv) identifying or selecting the plant cell, plant tissue, or plant or part thereof.

18. An herbicide-resistant plant obtainable by a method comprising crossing a first plant that is tolerant to an herbicide to a second plant that is not tolerant to the herbicide, wherein the first plant is the plant of any one of claims 7-13.

19. The plant of claim 18 wherein said method by which said plant is obtainable further comprises selecting for a progeny plant that is tolerant to the herbicide.

20. A seed of the plant of any one of claims 18-19, wherein said seed comprises the herbicide resistance characteristics of the first plant.

21. The seed of claim 14 or 20, wherein said seed is treated with a seed treatment formulation.

22. A method for combating undesired vegetation comprising contacting a seed of a plant comprising the polynucleotide of any one of claims 1-5, or the a seed of the plant of any one of claims 7-12 and 18-19, or the seed of any one of claims 14 and 20 before sowing and/or after pregermination with an AHAS-inhibiting herbicide.

23. A method for producing a herbicide-tolerant plant comprising:
crossing a first plant comprising the polynucleotide of claim 5 or the plant of any one of claims 10-13 with a second plant; and
selecting for a progeny plant that is tolerant to the AHAS-inhibiting herbicide.

24. The method of any one of claims 15, 17, 22, and 23, wherein the AHAS-inhibiting compound, the AHAS-inhibiting herbicide, and the seed treatment formulation are selected from imidazolinones, sulfonylureas, triazolopyrimidines, and pyrimidinyloxybenzoates.

25. The method of claim 24, wherein the AHAS-inhibiting compound, the AHAS-inhibiting herbicide, and the seed treatment formulation comprise an imidazolinone.

26. A purified AHASL protein encoded by the polynucleotide of any one of claims 1-5.

27. Use of a plant comprising the polynucleotide of any one of claims 1-5, the plant of any one of claims 7-13, a seed of the plant of any one of claims 7-13 and 18-19, or the seed of any one of claims 14 and 20-21 in a method for producing an agricultural product, optionally wherein said product is seed oil.

28. Use of a plant comprising the polynucleotide of any one of claims 1-5, the plant of any one of claims 7-13, a seed of the plant of any one of claims 7-13 and 18-19, or the seed of any one of claims 14 and 20-21 in a method for controlling weeds.
